(19) Europäisches Patentamt
European Patent Office
Office européen des brevets

(11) Veröffentlichungsnummer: **0 002 666**
**B1**

# (12) EUROPÄISCHE PATENTSCHRIFT

(45) Veröffentlichungstag der Patentschrift: **17.02.82**

(51) Int. Cl.³: **C 07 D 261/20**, C 07 D 413/04, C 07 D 413/12, A 61 K 31/42

(21) Anmeldenummer: **78101409.7**

(22) Anmeldetag: **20.11.78**

## (54) 1,2-Benzisoxazolyloxy (bzw. thio) essigsäuren und verwandte Verbindungen, Verfahren zu ihrer Herstellung sowie Arzneimittel die solche Verbindungen als Wirkstoff enthalten.

(30) Priorität: **21.11.77 US 853313**
**06.10.78 US 949128**

(43) Veröffentlichungstag der Anmeldung:
**11.07.79 Patentblatt 79/14**

(45) Bekanntmachung des Hinweises auf die Patenterteilung:
**17.02.82 Patentblatt 82/7**

(84) Benannte Vertragsstaaten:
**BE CH DE FR GB LU NL SE**

(56) Entgegenhaltungen:
**CHEMICAL ABSTRACTS, Vol. 83, Nr. 131575c, 1975, Columbus, Ohio, USA, NAGANO MITSUO et al. ›Isoxazole derivatives‹, Seite 508, linke Spalte**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Shutske, Gregory Michael, Runyon Avenue 22, Somerset, N.J. (US)**
Erfinder: **Setescak, Linda Louise, Capricorn, Drive 176, South Somerville, N.J. (US)**
Erfinder: **Allen, Richard Charles, Hillcrest Road, Box 514, Flemington, N.J. (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 666 B1

## 1,2-Benzisoxazolyloxy(bzw. thio)essigsäuren und verwandte Verbindungen, Verfahren zu ihrer Herstellung sowie Arzneimittel, die solche Verbindungen als Wirkstoffe enthalten

Die Erfindung betrifft neue 1,2-Benzisoxazolyloxy(bzw. thio)essigsäuren und verwandte Verbindungen mit diuretischer, uricosurischer und blutdrucksenkender Wirkung, Verfahren zu ihrer Herstellung und Arzneimittel, die eine solche Verbindung als Wirkstoff enthalten.

Die Verbindungen gemäß der Erfindung haben die allgemeine Formel

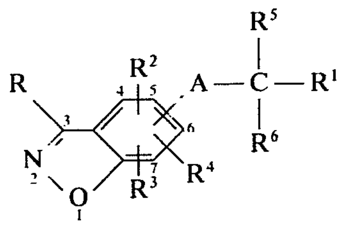

worin R Wasserstoff, Naphthyl, einen Rest der Formel

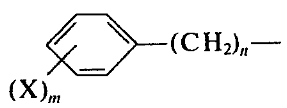

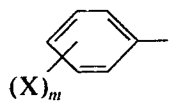

oder

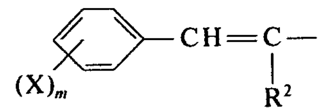 ,

unsubstituiertes oder mit Halogen oder $C_{1-4}$-Alkyl substituiertes Thienyl, Furyl, Pyridyl oder Pyridyl-N-oxyd bedeutet, $R^1$ für eine freie oder veresterte Carboxylgruppe mit 1 bis 8 Kohlenstoffatomen, einen Rest der Formel

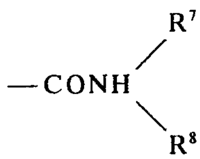

$-CH_2OH$

$-CH_6OR^9)_2$

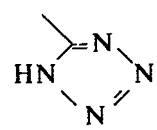

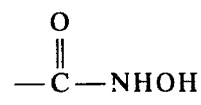

steht, $R^2$, $R^3$ und $R^4$, die gleich oder verschieden sein können, Wasserstoff, Halogen oder $C_{1-4}$-Alkyl bedeuten, X Wasserstoff, Halogen, $C_{1-4}$-Alkyl, $C_{1-4}$-Alkoxy, Hydroxy, Trifluormethyl oder Nitro bedeutet, $R^5$, $R^6$, $R^7$, $R^8$ und $R^9$, die gleich oder verschieden sein können, Wasserstoff oder $C_{1-4}$-Alkyl bedeuten, A für O oder S steht und m und n, die gleich oder verschieden sein können, 1, 2 oder 3 bedeuten. Die Erfindung umfaßt weiterhin physiologisch verträgliche Salze der obigen Verbindungen, die zur Salzbildung befähigt sind.

Einige Verbindungen gemäß der Erfindung haben eine stärkere pharmakologische Wirkung als andere. Einige Verbindungen der letzteren Gruppe, z. B. solche, in denen $R^1$ eine veresterte Carboxygruppe oder $CH_2OH$ bedeutet, sind auch als Zwischenprodukte zur Herstellung der wirksameren Verbindungen geeignet. Bevorzugte Verbindungen sind solche, in denen R einen aromatischen Ring, insbesondere einen Phenylring mit einem Orthosubstituenten, vorzugsweise ein Halogenatom, insbesondere ein Fluoratom, in bezug auf die Stelle der Bindung an die gesamte Ringstruktur darstellt, außerdem solche Verbindungen, in denen $R^1$ eine Carboxylgruppe bedeutet, die durch eine niedere Alkyl- oder die Benzylgruppe verestert ist. Weitere bevorzugte Verbindungen sind solche, in denen A Sauerstoff bedeutet.

Die physiologisch verträglichen Salze dieser Erfindung umfassen die Salze der Alkali- und Erdalkalimetalle und von nicht toxischen organischen Basen, z. B. von Äthanolamin, Diäthanolamin oder N-Methylglucamin.

Die Verbindungen gemäß der Erfindung können durch die folgenden Mehrschrittreaktionen hergestellt werden, wobei, wenn nicht anders angegeben, R, $R^1$ bis $R^8$, X, m und n die obigen Bedeutungen haben und Y für Chlor oder Brom steht.

a) Ein Phenol oder Alkoxybenzol der Formel II

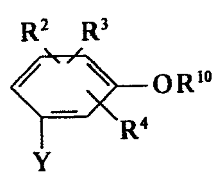

worin $R^{10}$ Wasserstoff oder $C_{1-4}$-Alkyl bedeutet, wird unter Friedel-Crafts-Bedingungen mit einem

Säurehalogenid der Formel RCOZ, worin R die obige Bedeutung hat und Z Chlor, Brom oder Fluor bedeutet, zu einer Verbindung der Formel

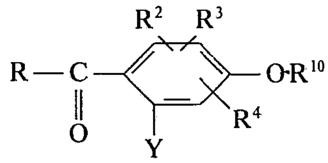

(III)

umgesetzt.

In einer bevorzugten Ausführungsform verwendet man 1,2-Di-chloräthan als Lösungsmittel und Aluminiumchlorid als Friedel-Crafts-Katalysator.

$a_2$) Eine Verbindung der Formel R—H, worin R

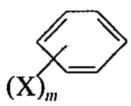

oder gegebenenfalls substituiertes Thienyl oder Furyl bedeutet, wird unter Friedel-Crafts-Bedingungen mit einem Säurehalogenid der Formel

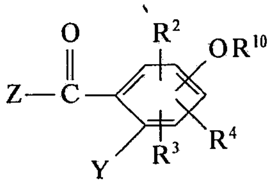

(IV)

worin Y, Z und $R^{10}$ die unter $a_1$) angegebene Bedeutung haben, zu einer Verbindung der Formel

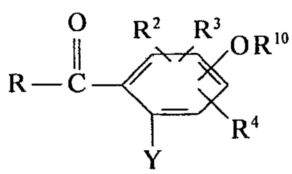

(IIIa)

umgesetzt.

Gemäß einer bevorzugten Ausführungsform werden 1,2-Di-chloräthan als Lösungsmittel und Aluminiumchlorid als Friedel-Crafts-Katalysator verwendet.

$a_3$) Eine Verbindung der Formel

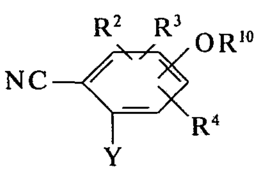

(IV)

worin $R^{10}$ $C_{1-4}$-Alkyl bedeutet, wird mit einer Verbindung der Formel R—MgZ oder R—Li, worin R nicht für Wasserstoff steht, umgesetzt und die erhaltene Verbindung zu einer Verbindung der IIIa hydrolysiert.

Die Verwendung von Tetrahydrofuran als Lösungsmittel und eine Temperatur von −70°C bis Raumtemperatur werden bevorzugt.

$a_4$) Eine Verbindung der Formel IIIa, worin R Wasserstoff bedeutet und $R^{10}$ $C_{1-4}$-Alkyl bedeutet, wird gemäß Methode $a_3$) zu einer Verbindung der Formel

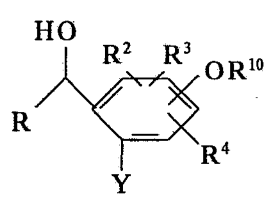

(V)

worin R nicht Wasserstoff bedeutet, umgesetzt.

$a_5$) Eine Verbindung, hergestellt gemäß Methode $a_4$), wird zu einer Verbindung der Formel IIIa, worin R nicht Wasserstoff bedeutet, oxidiert, z. B. unter Verwendung von Chromtrioxyd in Eisessig.

$a_6$) Eine Verbindung der Formel III oder IIIa, worin $R^{10}$ $C_{1-4}$-Alkyl bedeutet, kann in bekannter Weise desalkyliert werden, wobei man eine entsprechende Verbindung der Formel III oder IIIa erhält, in welcher $R^{10}$ Wasserstoff bedeutet, z. B. durch Behandlung mit Aluminiumchlorid in Benzol.

$a_7$) Eine Verbindung der Formel III oder IIIa wird mit Hydroxylamin-hydrochlorid in einem Lösungsmittel wie Pyridin behandelt, wobei die entsprechende Verbindung der Formel

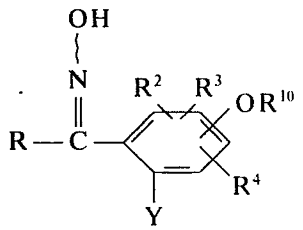

(VI)

erhalten wird.

$a_8$) Eine Verbindung der Formel VI wird durch eine Behandlung mit einer Base in Gegenwart eines Lösungsmittels bei einer Temperatur von Raumtemperatur bis zur Rückflußtemperatur des Reaktionsmediums zu einer entsprechenden bicyclischen Verbindung der Formel

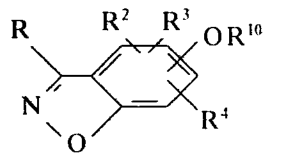

(VII)

cyclisiert, vorzugsweise mit Natriumhydrid als Base, Dimethylformamid als Lösungsmittel und bei Rückflußtemperatur.

$a_9$) Ein Diphenol oder Dialkoxybenzol der Formel

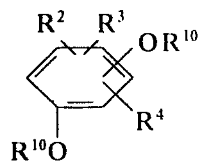

(VIII)

worin $R^2$, $R^3$, $R^4$ und $R^{10}$ die obige Bedeutung haben, wird gemäß Methode $a_1$) zu einer Verbindung der Formel

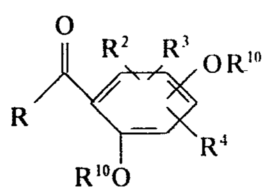

(IX)

umgesetzt.

$a_{10}$) Eine Verbindung der Formel R–H, worin R

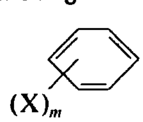

oder gegebenenfalls substituiertes Thienyl oder Furyl bedeutet, wird unter Friedel-Crafts-Bedingungen mit einem Säurehalogenid der Formel

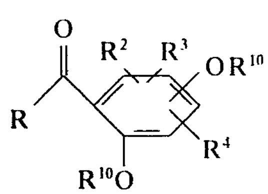

(X)

worin Z, $R^2$, $R^3$, $R^4$ und $R^{10}$ die obige Bedeutung haben, zu einer Verbindung der Formel

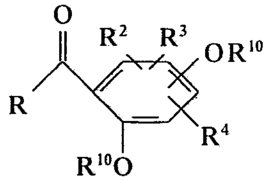

(XI)

umgesetzt.

$a_{11}$) Eine Verbindung der Formel

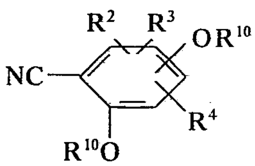

(XII)

worin $R^{10}$ $C_{1-4}$-Alkyl bedeutet, wird gemäß Methode $a_3$) zu einer entsprechenden Verbindung der Formel XI umgesetzt.

$a_{12}$) Eine Verbindung der Formel XI, worin R Wasserstoff und $R^{10}$ $C_{1-4}$-Alkyl bedeutet, wird gemäß Methode $a_3$) zu einer Verbindung der Formel

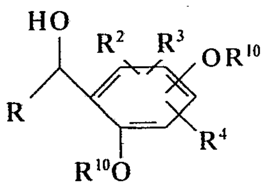

(XIII)

worin R nicht Wasserstoff bedeutet, umgesetzt.

$a_{13}$) Eine nach Methode $a_{12}$) hergestellte Verbindung wird zu einer Verbindung der Formel XI, in welcher R nicht Wasserstoff bedeutet, umgesetzt, z. B. unter Verwendung von Chromtrioxyd in Eisessig.

$a_{14}$) Eine Verbindung der Formel XI, worin $R^{10}$ $C_{1-4}$-Alkyl darstellt, kann teilweise zu einer entsprechenden Verbindung der Formel XI, in welcher $R^{10}$ in Ortho-Stellung zur Carbonylgruppe Wasserstoff bedeutet, oder vollständig zu einer Verbindung der Formel XI, in welcher beide $R^{10}$-Gruppen Wasserstoff bedeuten, dealkyliert werden. Im ersteren Fall wird ein Äquivalent und im zweiten Fall zwei Äquivalente Aluminiumchlorid verwendet, z. B. in Benzol als Lösungsmittel.

$a_{15}$) Eine Verbindung der Formel XI, worin mindestens der $R^{10}$-Rest in o-Stellung zur Carbonylgruppe Wasserstoff bedeutet, wird gemäß Methode g) in eine Verbindung der Formel

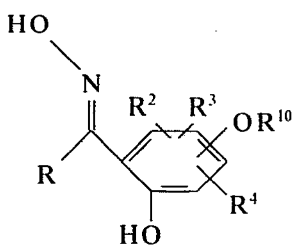

(XIV)

umgewandelt.

$a_{16}$) Eine Verbindung der Formel XIV wird zu einer Verbindung der Formel

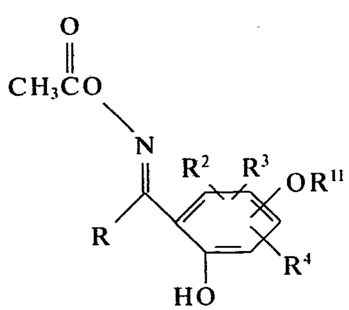

(XV)

worin $R^{11}$ Wasserstoff, $C_{1-4}$-Alkyl oder Acetyl bedeutet, acetyliert, vorzugsweise in Essigsäureanhydrid als Reagenz und Lösungsmittel.

$a_{17}$) Eine Verbindung der Formel XV wird mit einer Base in Gegenwart eines Lösungsmittels bei Raumtemperatur bis zur Rückflußtemperatur des Reaktionsgemisches cyclisiert zur entsprechenden bicyclischen Verbindung der Formel

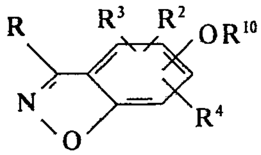

(VII)

$a_{18}$) Eine Verbindung der Formel VII, worin $R^{10}$ $C_{1-4}$-Alkyl bedeutet, wird zu einer entsprechenden Verbindung, in welcher $R^{10}$ Wasserstoff bedeutet, dealkyliert, z. B. durch eine Behandlung mit Pyridinhydrochlorid bei 170 bis 200°C.

$a_{19}$) Eine Verbindung der Formel VII, worin $R^{10}$ Wasserstoff bedeutet, wird mit einer Verbindung der Formel

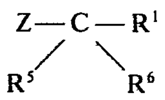

in welcher $R^1$ eine freie oder veresterte Carboxylgruppe, CN, $CH_2OH$ oder $CH(OR^9)_2$, worin $R^9$ $C_{1-4}$-Alkyl ist, in Gegenwart einer Base und eines Lösungsmittels, zu einer Verbindung der Formel

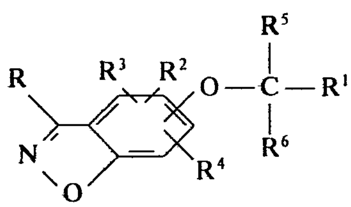

(Ia)

in welcher $R^1$ die obige Bedeutung hat, umgesetzt, vorzugsweise mit Natriumhydrid als Base und Dimethylformamid als Lösungsmittel.

$b_1$) Ein Phenoxyalkansäureester oder -nitril der Formel

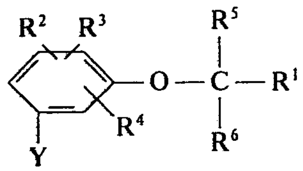

(XVI)

wird gemäß Methode $a_1$) zu einer Verbindung der Formel

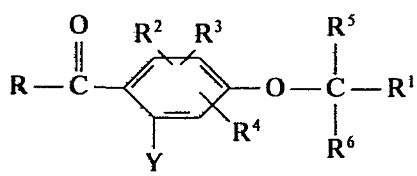

(XVII)

umgesetzt, vorzugsweise mit Aluminiumchlorid als Katalysator und Schwefelkohlenstoff als Lösungsmittel.

$b_2$) Eine Verbindung der Formel III oder IIIa, worin $R^{10}$ für Wasserstoff steht, wird gemäß Methode $a_{19}$) in eine Verbindung der Formel

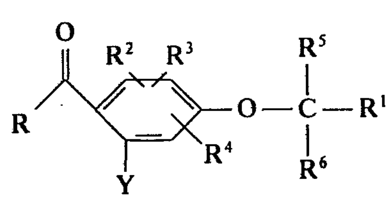

(XVIII)

in welcher $R^1$ eine freie oder veresterte Carboxylgruppe, CN, $CH_2OH$ oder $CH(OR^9)_2$ bedeutet, überführt.

$b_3$) Eine Verbindung der Formel XVIII wird gemäß Methode $a_7$) in eine Verbindung der Formel

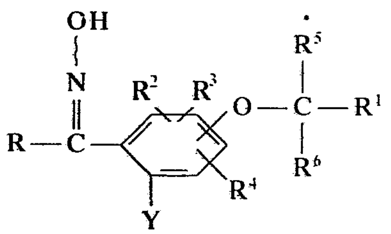

(XIX)

umgewandelt.

$b_4$) Eine Verbindung der Formel XIX wird gemäß Methode $a_9$) zu einer entsprechenden Verbindung der Formel Ia cyclisiert.

$c_1$) Eine Verbindung der Formel IX, worin $R^{10}$ für H steht, wird gemäß Methode $a_{19}$) zu einer Verbindung der Formel

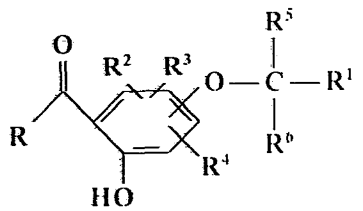

(XX)

umgewandelt.

$c_2$) Eine Verbindung der Formel XX wird gemäß Methode $a_4$) in eine Verbindung der Formel

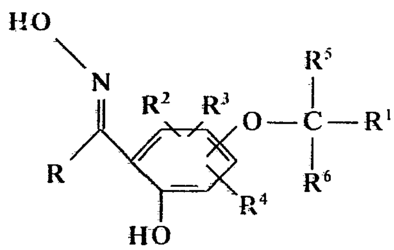

(XXI)

umgewandelt.

$c_3$) Eine Verbindung der Formel XXI wird gemäß Methode $a_{16}$) in eine Verbindung der Formel

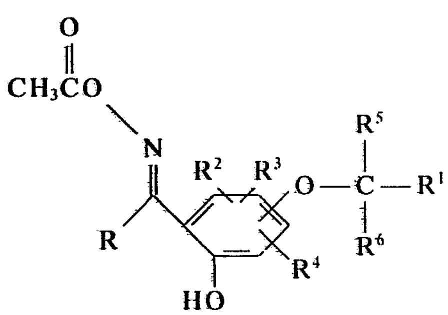

(XXII)

umgewandelt.

$c_4$) Eine Verbindung der Formel XXII wird gemäß Methode $a_{17}$) zu einer Verbindung der Formel Ia cyclisiert.

$d_1$) Eine Verbindung der Formel XI, worin R für Wasserstoff steht, hergestellt nach Methode $a_{14}$), wird mit Hydroxylamin-o-sulfonsäure in einem Lösungsmittel, z. B. Wasser, zu einer Verbindung der Formel VII umgesetzt.

$d_2$) Eine Verbindung der Formel XX, worin R für Wasserstoff steht, wird gemäß Methode $d_1$) in einer Verbindung der Formel Ia umgewandelt.

$e_1$) Eine Verbindung der Formel VII, in welcher $R^{10}$ für Wasserstoff steht und X nicht Hydroxy bedeutet, wird mit einem Dialkylthiocarbamoylhalogenid in Gegenwart einer Base zu einer Verbindung der Formel

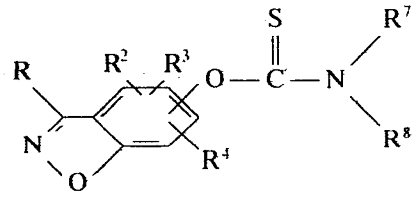

(XXIII)

worin $R^7$ und $R^8$ für niedriges Alkyl stehen, umgesetzt, vorzugsweise mit Dimethylthiocarbamoylchlorid in Dimethylformamid als Lösungsmittel und Natriumhydrid als Base.

$e_2$) Eine Verbindung der Formel XXIII wird durch Erhitzen im geschmolzenen Zustand thermisch zu einer Verbindung der Formel

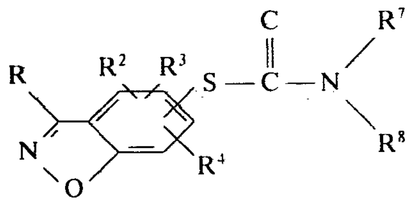

(XXIV)

umlagert.

$e_3$) Eine Verbindung der Formel XXIV wird nach einer üblichen Methode zu einer Verbindung der Formel

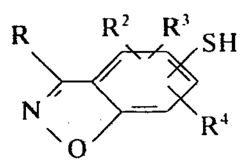

(XXV)

hydrolysiert, z. B. mit verdünnter Natronlauge.

$e_4$) Eine Verbindung der Formel XXV wird gemäß Methode $a_{19}$) in eine Verbindung der Formel

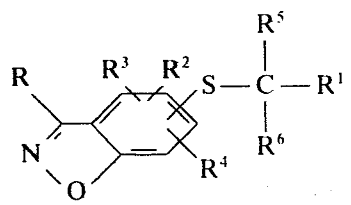

(Ib)

umgewandelt.

$f_1$) Eine Verbindung der Formel

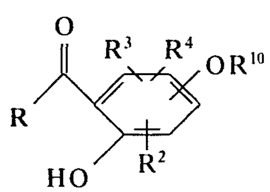

(XXVI)

wie in Methode $a_{14}$) beschrieben, worin $R^{10}$ $C_{1-4}$-Alkyl ist und X nicht Hydroxyl bedeutet, wird analog zu den Methoden $e_1$), $e_2$) und $e_3$) behandelt, wobei eine Verbindung der Formel

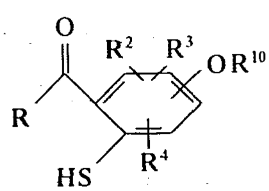

(XXVII)

erhalten wird.

$f_2$) Eine Verbindung der Formel XXVII wird analog zu den Methoden $a_7$), $a_{16}$), $a_{17}$) und $a_{18}$) behandelt, wobei eine Verbindung der Formel

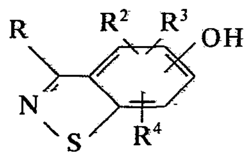

(XXVIII)

erhalten wird.

$f_3$) Eine Verbindung der Formel XXVIII wird analog zur Methode $a_{19}$) zu einer Verbindung der Formel

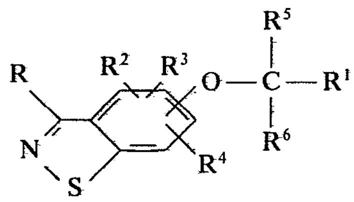

(Ic)

umgesetzt.

$g_1$) Eine Verbindung der Formel XXVIII wird analog zu den Methoden $e_1$), $e_2$) und $e_3$) zu einer Verbindung der Formel

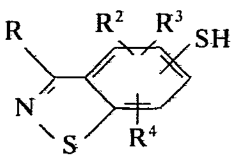

(XXIX)

umgesetzt.

$g_2$) Eine Verbindung der Formel XXIX wird gemäß Methode $a_{19}$) zu einer Verbindung der Formel

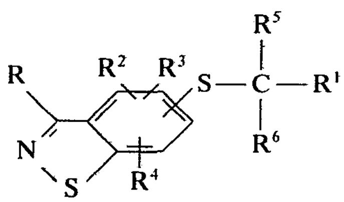

(Id)

umgesetzt.

h) Eine Verbindung der Formel Ia, Ib, Ic oder Id, worin $R^1$ für einen Carbonsäureester oder CN steht, läßt sich in eine entsprechende Verbindung, worin $R^1$ für COOH steht, umwandeln, z. B. durch Hydrolyse mit einer Base wie Natriumhydroxyd.

i) Eine Verbindung der Formel Ia, Ib, Ic oder Id, worin $R^1$ $CH(OR^9)_2$ bedeutet, läßt sich in eine entsprechende Verbindung mit $R^1$ = CHO umwandeln, z. B. durch Hydrolyse mit verdünnter Mineralsäure.

k) Eine Verbindung der Formel Ia, worin A für Sauerstoff steht, X, $R^2$, $R^3$ und $R^4$ nicht $C_{1-4}$-Alkyl bedeuten und $R^1$ für $CH_2OH$ oder CHO steht, kann in eine entsprechende Verbindung, worin $R^1$ für COOH steht, umgewandelt werden, z. B. durch Oxydation mit Kaliumpermanganat.

l) Eine Verbindung der Formel

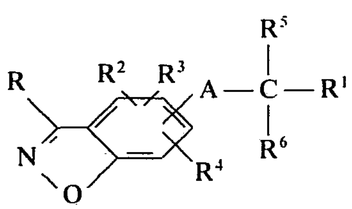

(I)

worin $R^1$ für COOH steht, läßt sich in eine Verbindung umwandeln, worin $R^1$ = COZ bedeutet, z. B. durch eine Behandlung mit $SOZ_2$.

m) Eine Verbindung der Formel I, worin $R^1$ für COZ steht, läßt sich durch Behandlung mit

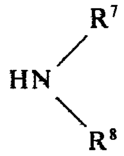

oder $NH_2OH$ in Gegenwart eines Säurefängers in eine Verbindung der Formel I umwandeln, worin $R^1$

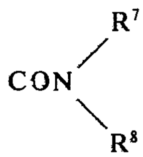

oder CONHOH bedeutet.

n) Eine Verbindung der Formel I, worin $R^1$ für CN steht, läßt sich in eine entsprechende Verbindung umwandeln, worin

$R^1$ = 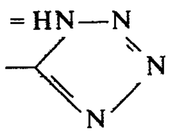

darstellt, z. B. durch Behandlung mit $HN_3$ in Dimethylformamid.

o) Eine Verbindung der Formel I, worin $R^1$

COZ 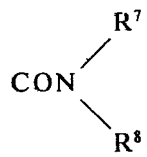 CONHOH 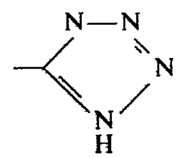

oder eine andere, nicht besonders definierte Gruppe bedeutet, die hydrolytisch in eine COOH-Gruppe umgewandelt werden kann, wird durch saure oder basische Hydrolyse in eine Verbindung der Formel I, worin $R^1$ den Rest COOH bedeutet, umgewandelt.

p) Eine Verbindung der Formel I, worin $R^1$ für COOH steht, läßt sich durch Behandlung mit einer geeigneten Alkali- oder Eralkalibase in einem Lösungsmittel in ein Salz umwandeln.

q) Eine Verbindung der Formel

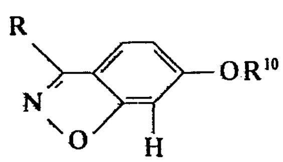

(XXXI)

worin

R = 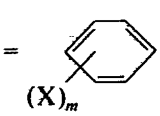

bedeutet, $R^{10}$ für $C_{1-4}$-Alkyl steht und X nicht Hydroxy oder Amino bedeutet, wird mit einer starken Base und anschließend mit einem geeigneten elektrophilen Mittel behandelt, wobei man eine Verbindung der Formel

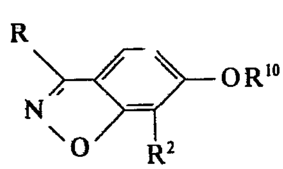

(XXXII)

erhält, worin $R^2$ für Halogen oder $C_{1-4}$-Alkyl steht. Eine bevorzugte Base ist n-Butyllithium und bevorzugte elektrophile Mittel sind, z. B. Brom, Jod, Chlor, N-Halosuccinimide und Alkylhalogenide.

r) Eine Verbindung der Formel

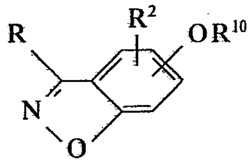

(XXIII)

worin $R^2$ Wasserstoff oder Halogen, $R^{10}$ für Wasserstoff oder $C_{1-4}$-Alkyl, R für

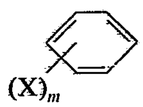

und X für Halogen stehen, wird mit elementarem Halogen in einem Lösungsmittel wie Essigsäure zu einer Verbindung der Formel

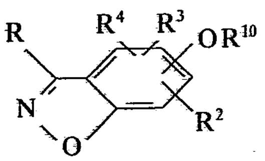

(VII)

worin $R^3$ und $R^4$ beide Halogen sein können, umgesetzt.

s) Eine Verbindung der Formel

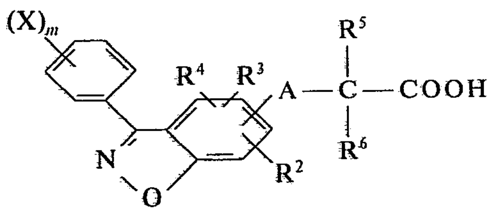

(Ie)

worin m 1 oder 2 ist und X nicht $NO_2$ bedeutet, kann durch eine Behandlung mit Salpetersäure in Eisessig nitriert werden, wobei die entsprechende Verbindung der Formel

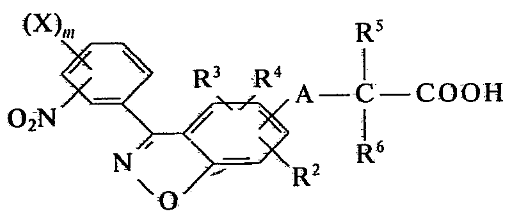

(If)

erhalten wird.

t) Eine Verbindung der Formel

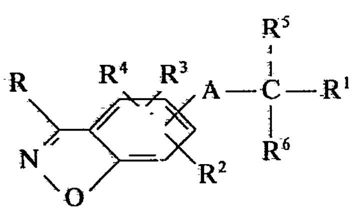

worin R einen Rest der Formel

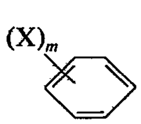

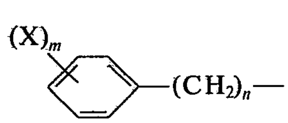

oder

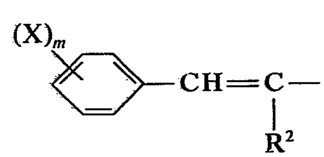

darstellt, und X für Alkoxy steht, kann zu einer entsprechenden Verbindung, in welcher X Hydroxy bedeutet, dealkyliert werden, z. B. durch eine vorsichtige Behandlung mit Bortribromid.

u) Eine Verbindung der Formel VII, worin R für Pyridyl steht, kann mit einem entsprechenden Oxydationsmittel zu einer entsprechenden Verbindung der Formel VII, in welcher R

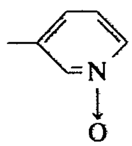

bedeutet, oxydiert werden, z. B. mit 3-Chlorperbenzoesäure.

Die Reaktionszeiten und genauen Reaktionsbedingungen in allen obigen Reaktionsstufen sind abhängig von den verwendeten Reaktionspartnern und Lösungsmitteln.

Alle Ausgangsmaterialien sind entweder bekannt oder durch bekannte Methoden aus leicht zugänglichen Substanzen einfach herzustellen.

Eine in den Stufen $a_2$), $a_4$), $a_5$), $a_{12}$) und $a_{13}$) zur Herstellung einer erfindungsgemäßen Verbindung, worin die $OCH_2R^1$-Gruppe in 5-Stellung steht, geeignete Ausgangssubstanz ist, z. B. 2-Chlor-5-methoxybenzoesäure. Eine solche Substanz läßt sich aus 2-Chlor-5-nitroanilin durch allgemein bekannte Methoden herstellen, wie Diazotisierung und Behandlung mit CuCN zu 2-Chlor-5-nitro-benzonitril mit nachfolgender Reduzierung mit Eisenspänen in wäßrig-äthanolischer Salzsäure zu 5-Amino-2-chlorbenzonitril, Diazotisierung zu 2-Chlor-5-hydroxy-benzonitril, nachfolgende Methylation mit Dimethylsulfat zu 2-Chlor-5-methoxy-benzonitril und schließlich Hydrolyse zu 2-Chlor-5-methoxybenzoesäure. Andere geeignete, substituierte Alkoxy-o-halogen-benzoesäuren, Alkoxy(hydroxy)-o-alkoxy-(hydroxy)benzoesäuren, -aldehyde und -nitrile und eine Reihe von Haloalkoxy(hydroxy)- und Dialkoxy-(hydroxy)-benzole können in einfacher Weise durch ähnliche Reaktionsfolgen oder andere bekannte Reaktionen hergestellt werden.

Auf Grund ihrer diuretischen Wirkung sind die erfindungsgemäßen Verbindungen als Diuretika geeignet. Die diuretische Wirksamkeit wird an Mäusen in Anlehnung an die Methode von C. M. Kagawa und M. J. Kalm, Arch. Intern. Pharmacodyn. 137, 241 (1962), bestimmt. Einer Gruppe von 6 Mäusen werden die erfindungsgemäßen Verbindungen oral verabreicht, und das durchschnittlich ausgeschiedene Urinvolumen wird mit der durchschnittlich ausgeschiedenen Urinmenge einer Kontrollgruppe von 6 Mäusen, die oral 1000 mg/kg Harnstoff, ein bekanntes Diuretikum, erhalten haben, verglichen. Eine diuretische Wirksamkeit ist gegeben, wenn der Quotient der Urinmengen, die durch eine der geprüften Verbindungen bzw. durch Harnstoff ausgeschieden werden, größer als 1 ist (vgl. Spalte 2).

Die diuretischen Wirksamkeiten einiger Verbindungen gemäß der Erfindung und der Standarddiuretika Thienylsäure und Ethacrynsäure, sind in der folgenden Tabelle angegeben.

Tabelle I

| Verbindung | Dosis MG/KG P.O. | Quotient der ausgeschiedenen Urinmengen |
|---|---|---|
| {[7-Chlor-3-(2-fluorphenyl)-1,2-benzisoxazol-6-yl]oxy}-essigsäure | 4<br>32 | 2.2<br>6.1 |
| {[7-Chlor-3-(2-thienyl)-1,2-benzisoxazol-6-yl]oxy}-essigsäure | 64 | 1.2 |
| {[7-Chlor-3-phenyl-1,2-benzisoxazol-6-yl]oxy}-essigsäure | 64 | 2.0 |
| {[7-Chlor-3-(2-furyl)-1,2-benzisoxazol-6-yl]oxy}-essigsäure | 64 | 1.1 |
| {[7-Chlor-3-(4-tolyl)-1,2-benzisoxazol-6-yl]oxy}-essigsäure | 4<br>64 | 1.2<br>2.0 |
| {[7-Chlor-3-(4-chlorphenyl)-1,2-benzisoxazol-6-yl]oxy}-essigsäure | 64 | 1.5 |
| {[7-Chlor-3-(5-methyl-2-furyl)-1,2-benzisoxazol-6-yl]oxy}-essigsäure | 64 | 2.0 |
| {[7-Chlor-3-(3-furyl)-1,2-benzisoxazol-6-yl]oxy}-essigsäure | 64 | 2.0 |
| {[7-Chlor-3-(2-chlorphenyl)-1,2-benzisoxazol-6-yl]oxy}-essigsäure | 4<br>16<br>32 | 1.3<br>2.4<br>5.6 |
| {[7-Chlor-3-(2-tolyl)-1,2-benzisoxazol-6-yl]oxy}-essigsäure | 64 | 2.3 |
| [(3-Benzyl-7-chlor-1,2-benzisoxazol-6-yl)]-essigsäure | 64 | 1.2 |
| {[7-Chlor-3-(1-naphthyl)-1,2-benzisoxazol-6-yl]oxy}-essigsäure | 64 | 1.1 |
| {[7-Chlor-3-(3-methyl-2-thienyl)-1,2-benzisoxazol-6-yl]oxy}-essigsäure | 64 | 1.4 |
| {[7-Chlor-3-(2,6-difluorphenyl)-1,2-benzisoxazol-6-yl]oxy}-essigsäure | 4<br>64 | 3.1<br>7.0 |
| {[3-(2-Fluorphenyl)-1,2-benzisoxazol-6-yl]oxy}-essigsäure | 4<br>64 | 1.3<br>4.6 |
| {[3-(2-Fluorphenyl)-7-methyl-1,2-benzisoxazol-6-yl]oxy}-essigsäure | 8<br>64 | 2.1<br>6.4 |
| {[3-(2-Fluorphenyl)-7-iod-1,2-benzisoxazol-6-yl]oxy}-essigsäure | 64 | 2.3 |
| {[7-Brom-3-(2-fluorphenyl)-1,2-benzisoxazol-6-yl]oxy}-essigsäure | 4<br>64 | 1.7<br>6.2 |
| {[7-Chlor-3-(trans-β-fluorstyryl)-1,2-benzisoxazol-6-yl]oxy}-essigsäure | 64 | 1.0 |
| {[5,7-Dichlor-3-(2-fluorphenyl)-1,2-benzisoxazol-6-yl]thio}-essigsäure | 64 | 3.1 |
| Tienylsäure | 64<br>16 | 1.8<br>1.2 |
| Ethacrynsäure | 64 | 2.5 |

Um eine diuretische Wirkung zu erhalten, wird den Patienten eine tägliche Dosis von 0,1–500 mg/kg, vorzugsweise 1,0–200 mg/kg Körpergewicht, oral, parenteral oder intravenös verabreicht.

Die erfindungsgemäßen Verbindungen sind auch als blutdrucksenkende Mittel geeignet. Die blutdrucksenkende Wirksamkeit wird an der Hochdruckratte durch die indirekte Schwanz-Manschetten-Methode von A. Schwartz Ed., Methods in Pharmacology, Band I, Seite 135, Appleton-Century-Crofts, New York 1971, bestimmt. Zu diesem Zweck wird eine Gruppe von 5 Tieren oral mit dem Medikament 3 Tage lang behandelt, und die Resultate werden mit denen einer Kontrollgruppe verglichen. Der Blutdruckabfall wird am dritten Tag nach der Verabreichung gemessen. Die blutdrucksenkende Wirksamkeit einiger Verbindungen der Erfindung, ausgedrückt als Abnahme des Hauptarteriendruckes in mm Hg ist in Tabelle II angegeben.

Tabelle II

| Verbindung | MG/KG P.O. | Blutdruckabfall |
|---|---|---|
| {[7-Chlor-3-phenyl-1,2-benzisoxazol-6-yl]oxy}-essigsäure | 50 | 15 |
| {[7-Chlor-3-(2-furyl)-1,2-benzisoxazol-6-yl]oxy}-essigsäure | 50 | 22 |
| {[7-Chlor-3-(4-tolyl)-1,2-benzisoxazol-6-yl]oxy}-essigsäure | 50 | 16 |
| {[7-Chlor-3-(4-chlorphenyl)-1,2-benzisoxazol-6-yl]oxy}-essigsäure | 50 | 16 |
| {[7-Chlor-3-(5-methyl-2-thienyl)-1,2-benzisoxazol-6-yl]oxy}-essigsäure | 50<br>10 | 53<br>35 |
| [(3-Benzyl-7-chlor-1,2-benzisoxazol-6-yl)oxy]-essigsäure | 50 | 27 |
| {[7-Chlor-3-(1-naphthyl)-1,2-benzisoxazol-6-yl]oxy}-essigsäure | 50 | 39 |
| {[7-Chlor-3-(2,3-dimethylphenyl)-1,2-benzisoxazol-6-yl]oxy}-essigsäure | 50 | 21 |
| {[7-Chlor-3-(4-fluorphenyl)-1,2-benzisoxazol-6-yl]oxy}-essigsäure | 50 | 19 |
| {[7-Chlor-3-(2-pyridyl)-1,2-benzisoxazol-6-yl]oxy}-essigsäure | 50 | 21 |
| {[3-(2-Fluorphenyl)-7-methyl-1,2-benzisoxazol-6-yl]oxy}-essigsäure | 50 | 28 |
| {[7-Chlor-3-(trans-β-fluorstyryl)-1,2-benzisoxazol-6-yl]oxy}-essigsäure | 50 | 27 |

Um eine blutdrucksenkende Wirkung zu erreichen, wird den Patienten eine tägliche Dosis von 0,1–500 mg/kg, vorzugsweise 1,0–200 mg/kg Körpergewicht, oral, parenteral oder intravenös verabreicht.

Auf Grund ihrer Fähigkeit, die Ausscheidung von Harnsäure zu steigern, sind die Verbindungen der Erfindung auch als uricosurische Mittel geeignet. Zur Bestimmung der uricosurischen Wirksamkeit bekommen Gruppen von jeweils 6 Wistarratten 25 ml/kg einer Suspension oder Lösung der Testverbindung in destilliertem Wasser oral verabreicht. Eine entsprechende Kontrollgruppe erhält nur die gleiche Menge Wasser. Der Urin wird während 5 Stunden gesammelt und der Gehalt an Harnsäure in einem Abbot Biochromatic Analyzer unter Verwendung von Uricosquant®-Reagenz bestimmt. Die Ergebnisse von jeder Gruppe sind als durchschnittliche Harnsäureausscheidung in mg/kg Ratte ausgedrückt und werden mit den Werten von Kontrollgruppen verglichen. Die entsprechenden Werte sind in Tabelle III zusammengefaßt.

Tabelle III

| Verbindung | Dosis MG/KG P.O. | mg Harnsäure-ausscheidung/KG |
|---|---|---|
| {[7-Chlor-3-(2-thienyl)-1,2-benzisoxazol-6-yl]oxy}-essigsäure | 256 | 3.5 |
| {[7-Chlor-3-phenyl)-1,2-benzisoxazol-6-yl]oxy}-essigsäure | 128 | 2.6 |
| {[7-Chlor-3-(2-furyl)-1,2-benzisoxazol-6-yl]oxy}-essigsäure | 128 | 2.5 |
| {[7-Chlor-3-(5-methyl-2-thienyl)-1,2-benzisoxazol-6-yl]oxy}-essigsäure | 128 | 3.4 |
| {[7-Chlor-3-(3-furyl)-1,2-benzisoxazol-6-yl]oxy}-essigsäure | 128 | 2.7 |
| {[7-Chlor-3-(2-fluorphenyl)-1,2-benzisoxazol-6-yl]oxy}-essigsäure | 64<br>128<br>256<br>512 | 3.3<br>4.0<br>5.7<br>13.5 |
| {[7-Chlor-3-(4-fluorphenyl)-1,2-benzisoxazol-6-yl]oxy}-essigsäure | 32 | 3.0 |
| {[7-Chlor-3-(2,6-difluorphenyl)-1,2-benzisoxazol-6-yl]oxy}-essigsäure | 64 | 2.5 |
| {[7-Chlor-3-(2,4-difluorphenyl)-1,2-benzisoxazol-6-yl]oxy}-essigsäure | 64 | 4.4 |
| {[3-(2-fluorphenyl)-1,2-benzisoxazol-6-yl]oxy}-essigsäure | 128 | 2.5 |
| {[5,7-Dichlor-3-(2-fluorphenyl)-1,2-benzisoxazol-6-yl]thio}-essigsäure | 128 | 3.8 |
| Tienylsäure | 64 | 2.9 |
| Ethacrynsäure | ohne Wirkung | |

Um eine urikosurische Wirkung zu erreichen, wird den Patienten eine tägliche Dosis von 0,1 bis 500 mg/kg, vorzugsweise 1,0–200 mg/kg Körpergewicht, oral, parenteral oder intravenös verabreicht.

Eine besondere Bedeutung der obigen Verbindungen liegt in ihrer doppelten Wirksamkeit als Diuretika und Uricosurika. Es ist bekannt, daß während einer diuretischen Behandlung mit bekannten Diuretika in vielen Fällen die Harnsäurekonzentration im Blut eines Patienten steigt. Ein erhöhter Harnsäurespiegel ist ein ernstes Problem bei Gichtpatienten. Darüber hinaus wird ein erhöhter Harnsäurespiegel mehr und mehr als Risikofaktor bei Herzkrankheiten angesehen. Daher kann die diuretische Wirkung mit gleichzeitiger Ausscheidung von Harnsäure als ein Hauptvorteil der erfindungsgemäßen Verbindungen angesehen werden.

Die Erfindung betrifft insbesondere die folgenden Verbindungen:

{[7-Chlor-3-(4-methoxyphenyl)-1,2-benzisoxazol-6-yl]oxy}-essigsäure;
{[7-Chlor-3-(4-hydroxy-2-fluorophenyl)-1,2-benzisoxazol-6-yl]oxy}-essigsäure;
{[7-Chlor-3-(3-trifluormethylphenyl)-1,2-benzisoxazol-6-yl]oxy}-essigsäure;
{[7-Chlor-3-(4-nitrophenyl)-1,2-benzisoxazol-6-yl]oxy}-essigsäure;
{[4,5-Dichlor-3-(2-fluorphenyl)-1,2-benzisoxazol-6-yl]oxy}-essigsäure;
{[4,7-Dichlor-3-(2-fluorphenyl)-1,2-benzisoxazol-6-yl]oxy}-essigsäure;
[(4-Methyl-3-phenyl-1,2-benzisoxazol-6-yl)oxy]essigsäure;
[(5-Methyl-3-phenyl-1,2-benzisoxazol-6-yl)oxy]essigsäure;
[(3-Phenyl-1,2-benzisoxazol-4-yl)oxy]essigsäure;
[(3-Phenyl-1,2-benzisoxazol-5-yl)oxy]essigsäure;
[(3-Phenyl-1,2-benzisoxazol-7-yl)oxy]essigsäure;
{[7-Chlor-3-(2-fluorphenyl)-1,2-benzisoxazol-6-yl]oxy}-essigsäurebenzylester;
{[3-(2-Thienyl)-1,2-benzisoxazol-6-yl]oxy}-essigsäure-n-propylester;

[(3-Phenyl-1,2-benzisoxazol-7-yl)oxy]essigsäure-t-butylester;
{[7-Brom-3-(2-fluor-4-hydroxyphenyl)-1,2-benzisoxazol-6-yl]oxy}-essigsäure;
2-{[7-Brom-3-(2-fluorphenyl)-1,2-benzisoxazol-6-yl]oxy}-äthanol;
5-{[7-Chlor-3-(2-fluorphenyl)-1,2-benzisoxazol-6-yl]oxy}-methyltetrazol;
{[7-Brom-5-chlor-3-(2-fluorphenyl)-1,2-benzisoxazol-6-yl]oxy}-essigsäure;
{[7-Brom-5-methyl-3-(2-fluorphenyl)-1,2-benzisoxazol-6-yl]oxy}-essigsäure;
{[7-Chlor-3-(2-fluorphenyl)-1,2-benzisoxazol-6-yl]oxy}-essigsäureglycerylester;
{[7-Brom-3-(2,6-difluorphenyl)-1,2-benzisoxazol-6-yl]oxy}-essigsäure;
{[7-Methyl-3-(2,5-difluorphenyl)-1,2-benzisoxazol-6-yl]oxy}-essigsäure;
{[7-Brom-3-(2,3-difluorphenyl)-1,2-benzisoxazol-6-yl]oxy}-essigsäure;
{[7-Brom-3-(2,5-difluorphenyl)-1,2-benzisoxazol-6-yl]oxy}-essigsäure;
{[7-Chlor-3-(3-fluor-2-thienyl)-1,2-benzisoxazol-6-yl]oxy}-essigsäure;
{[7-Fluor-3-(2-fluorphenyl)-1,2-benzisoxazol-6-yl]oxy}-essigsäure.

Wirksame Mengen der erfindungsgemäßen Verbindungen können einem Patienten auf verschiedene Weise verabreicht werden, z. B. oral in Form von Kapseln oder Tabletten, parenteral in Form von sterilen Lösungen oder Suspensionen und in einigen Fällen auch intravenös als sterile Lösungen. Die freien Säuren, die selbst wirksam sind, können formuliert werden und aus Gründen der Stabilität, besserer Kristallisierbarkeit, besserer Löslichkeit etc. auch in Form ihrer pharmazeutisch verträglichen Salze verabreicht werden.

Für eine orale Verabreichung können die wirksamen Verbindungen der Erfindung mit einem Verdünnungsmittel oder eßbaren Träger vermischt, in Gelatinekapseln eingeschlossen oder zu Tabletten verpreßt werden. Für eine oral therapeutische Verabreichung können die wirksamen Verbindungen in Träger eingearbeitet und in Form von Tabletten, Pastillen, Kapseln, Elixieren, Suspensionen, Syrups, Waffeln, Kaugummi usw. verwendet werden. Diese Präparate sollen mindestens 0,5% an wirksamer Substanz enthalten, in Abhängigkeit der besonderen Form kann der Gehalt jedoch zwischen 4 und 70% des Gewichtes der Einheit schwanken. Die Menge an aktiver Verbindung in solchen Präparaten ist so bemessen, daß eine geeignete Dosierung erreicht werden kann. Bevorzugte Mischungen und Präparate enthalten pro oraler Einheitsdosis zwischen 1,0 und 300 Milligramm der wirksamen Verbindung.

Die Tabletten, Pillen, Kapseln, Pastillen usw. können außerdem die folgenden Bestandteile enthalten: Bindemittel wie mikrokristalline Cellulose, Tragantgummi oder Gelatine, Träger wie Stärke oder Laktose, Zerfallmittel wie Alginsäure, Maisstärke usw., Gleitmittel wie Magnesiumstearat oder kolloidales Siliziumdioxyd, Süßstoff wie Rohrzucker oder Saccharin, oder ein Aromastoff wie Pfefferminz, Methylsalicylat oder Orangenaroma. Im Fall einer Einheitsdosis in Kapselform können diese, neben den genannten Substanzen, auch einen flüssigen Träger enthalten, z. B. ein Öl. Tabletten oder Dragees können z. B. auch mit Zucker, Schellack oder anderen darmlöslichen Überzügen versehen sein. Ein Syrup kann neben der wirksamen Verbindung noch Rohrzucker als Süßstoff, bestimmte Konservierungsmittel, Farbstoffe und Geschmacksstoffe enthalten. Die zur Herstellung der Mischungen verwendeten Materialien sollten pharmazeutisch rein und in den zugegebenen Mengen ungiftig sein.

Für eine parenteral therapeutische Verabreichung können die wirksamen Verbindungen gemäß der Erfindung in eine Lösung oder Suspension eingearbeitet werden. Solche Präparate sollen mindestens 0,1% der aktiven Verbindung enthalten, jedoch kann der Gehalt zwischen 0,5 und 30 Gew.-% schwanken. Die Präparate haben einen solchen Gehalt an wirksamer Verbindung, daß eine geeignete Einheitsdosis erhalten werden kann. Die Mischungen und Präparate gemäß der Erfindung enthalten vorzugsweise zwischen 0,5 und 100 mg aktive Substanz pro parenteraler Dosiereinheit.

Die Lösungen und Suspensionen können die folgenden Komponenten enthalten: Ein steriles Verdünnungsmittel, wie Wasser zur Injektion, Salzlösung, nichtflüchtige Öle, Polyäthylenglykol, Glycerin, Propylenglykol oder andere synthetische Lösungsmittel, antibakterielle Mittel, wie Benzylalkohol; Antioxydantien wie Ascorbinsäure oder Natriumbisulfit; Chelierungsmittel wie Äthylendiamintetraessigsäure; Puffer wie Acetate, Zitrate oder Phosphate, und Mittel zur Einstellung der Tonizität wie Kochsalz oder Dextrose. Die parenteralen Präparate können in Ampullen, Einwegspritzen oder Mehrfachdosis-Fläschchen aus Glas oder Plastik abgefüllt werden.

Die folgenden Beispiele veranschaulichen die Erfindung:

## Beispiel 1

a) 26,5 g Aluminiumchlorid werden portionsweise innerhalb von 30 Minuten zu einer Lösung von 31,5 g 2-Fluorbenzoylchlorid in 100 ml Dichloräthan zugegeben. Nach beendeter Zugabe wird die Mischung zuerst gelb und dann dunkel. Zu der dunklen Mischung wird dann eine Lösung von 32 g 2,3-Dichloranisol in 50 ml 1,2-Dichloräthan zugetropft. Nach der Zugabe wird die Mischung 2 Stunden gerührt und dann auf eine Mischung von 100 ml konzentrierter Salzsäure und 100 ml zerkleinertes Eis gegossen.
Die organische Phase der Zweiphasenmischung wird im Vakuum verdampft und die wäßrige Mischung mit Äther extrahiert. Die vereinigten Ätherextrakte werden mit 10%iger Kaliumkarbonatlösung und dann mit Wasser gewaschen, getrocknet, und der Ather wird abgedampft, wobei ein weißer Feststoff zurückbleibt, der aus einer Mischung von Äther und Hexan umkristallisiert wird. Das erhaltene 2'-Fluor-4-methoxy-2,3-dichlorbenzophenon hat einen Schmelzpunkt von 74 bis 77°C.

b) Eine Mischung aus 38,5 g 2'-Fluor-4-methoxy-2,3-dichlorbenzophenon und 34,7 g Aluminiumchlorid in 250 m Benzol wird unter Rückfluß 5 Stunden zum Sieden erhitzt und dann auf eine Mischung aus 100 ml konzentrierter Salzsäure und 100 ml Eis gegossen. Die Zweiphasenmischung wird mit Äthylacetat extrahiert und die vereinigten Extrakte werden getrocknet und zur Trockne eingedampft, wobei ein fester Rückstand erhalten wird. Der Rückstand wird mit Hexan verrieben, und die erhaltene feste Substanz wird aus einer Äther-Hexan-Mischung umkristallisiert, wobei 2,3-Dichlor-4-hydroxy-2'-fluor-benzophenon mit einem Schmelzpunkt von 128–131°C erhalten wird.

c) Eine Lösung von 31,8 g 2,3-Dichlor-4-hydroxy-2'-fluor-benzophenon und 15,3 g Hydroxylaminhydrochlorid in 150 ml Pyridin wird unter Rückfluß 64 Stunden zum Sieden erhitzt. Danach wird das Pyridin im Vakuum verdampft und eine 5%ige wäßrige Salzsäurelösung zugegeben. Die angesäuerte Lösung wird mit Äthylacetat extrahiert, und die vereinigten Extrakte werden getrocknet und zur Trockne eingedampft. Die feste Substanz wird aus wäßriger Äthanollösung umkristallisiert, wobei 2,3-Dichlor-4-hydroxy-2'-fluorbenzophenonoxim mit einem Schmelzpunkt von 168 bis 175°C erhalten wird.

d) Eine Lösung von 18,4 g 2,3-Dichlor-4-hydroxy-2'-fluorbenzophenonoxim und 3,6 g Natriumhydrid in 120 ml Dimethylformamid und 120 ml Benzol wird 3 Stunden bei einer Temperatur von 80–85°C gehalten. Nachdem die Lösung sich auf Raumtemperatur abgekühlt hat, wird eine Lösung von 11,0 g Äthylbromacetat in 20 ml Dimethylformamid zugetropft. Nach vollständiger Zugabe wird die Mischung 30 Minuten gerührt, und zur Zersetzung von einem möglichen Überschuß an Natriumhydrid wird Wasser zugefügt. Die Mischung wird mit Äthylacetat extrahiert, die vereinigten Extrakte getrocknet und zur Trockne eingedampft. Die zurückbleibende feste Substanz wird wiederholt aus 95%igem Äthanol umkristallisiert, wobei reiner {[7-Chlor-3-(2-fluorphenyl)-1,2-benzisoxazol-6-yl]oxy}-essigsäureäthylester mit einem Schmelzpunkt von 102–104°C erhalten wird.

Analyse:

Berechnet für $C_{17}H_{13}ClFNO_4$:
58,38% C; 3,75% H; 4,01% N.
Gefunden:
58,11% C; 3,62% H; 3,86% N.

Beispiel 2

Eine Mischung aus 10,0 g {[7-Chlor-3-(2-fluorphenyl)-1,2-benzisoxazol-6-yl]oxy}-essigsäureäthylester, 100 ml 10%ige Natronlauge und 350 ml Äthanol wird 3,5 Stunden unter Rückfluß erhitzt. Danach wird das Äthanol im Vakuum entfernt und der Rückstand mit 5%iger Salzsäure angesäuert, wobei eine feste Substanz ausfällt, die abfiltriert und getrocknet wird. Das getrocknete Produkt wird aus 95%igem Äthanol umkristallisfiert. Die erhaltene {[7-Chlor-3-(2-fluorphenyl)-1,2-benzisoxazol-6-yl]-oxy}essigsäure hat einen Schmelzpunkt von 190–191°C.

Beispiel 3

a) 7,1 g 2-Fluorbenzoylchlorid werden bei einer Temperatur unter °C zu einer Mischung aus 12,5 g Aluminiumchlorid und 45 ml Schwefelkohlenstoff zugetropft und die Mischung 1,5 Stunden auf der niedrigen Temperatur gehalten. Bei dieser Temperatur werden 5 g 2,3-Dichlor-phenoxyessigsäure portionsweise zugegeben, und nach der Zugabe wird die Mischung für weitere 30 Minuten auf der niedrigen Temperatur gehalten. Nach dem Erreichen von Raumtemperatur wird die Mischung 28 Stunden unter Rückfluß zum Sieden erhitzt. Der Schwefelkohlenstoff wird von der Lösung abdekantiert, wobei ein dunkel orangefarbiger Rückstand erhalten wird, der auf eine Mischung von 500 ml Eis und Wasser und 100 ml konzentrieter Salzsäure gegossen wird. Der gebildete rosa Niederschlag wird abfiltriert, mit 300 ml warmen Wasser (50°C) gewaschen und in einem Vakuumofen getrocknet. Das getrocknete Produkt wird zweimal aus wäßrigem Äthanol umkristallisiert. Die erhaltene 2,3-Dichlor-4-(2-fluorbenzoyl)-phenoxyessigsäure hat einen Schmelzpunkt von 153–156°C.

b) Eine Mischung aus 1,0 g 2,3-Dichlor-4-(2-fluorbenzoyl)-phenoxyessigsäure und 1 g Hydroxylamin in 10 ml Pyridin wird 2 Stunden unter Rückfluß erhitzt, das Lösungsmittel wird im Vakuum entfernt und der Rückstand 16 Stunden mit 5%iger Salzsäure gerührt. Das erhaltene Produkt wird

abfiltriert und aus wäßrigem Äthanol umkristallisiert. Die erhaltene 2,3-Dichlor-4-(2-fluorbenzohydroximoyl)-phenoxyessigsäure hat einen Schmelzpunkt von 91–96°C.

c) Eine Mischung aus 0,3 g 2,3-Dichlor-4-(2-fluorbenzohydroximoyl)-phenoxyessigsäure und 0,05 g Natriumhydrid in 5 ml Benzol und 5 ml Dimethylformamid wird 3 Stunden unter Rückfluß erhitzt. Nach dem Abkühlen wird die Mischung mit 5%iger Salzsäure versetzt, wodurch sich das Benzol abscheidet, welches im Vakuum verdampft wird. Der zurückbleibende Niederschlag wird abfiltriert und aus wäßrigem Äthanol umkristallisiert. Die erhaltene {[7-Chlor-3-(2-fluorphenyl)-1,2-benzisoxazol-6-yl]oxy}essigsäure hat einen Schmelzpunkt von 188–189°C.

Analyse:

Berechnet für $C_{15}H_9ClFNO_4$:
56,00% C; 2,83% H; 4,36% N.
Gefunden:
55,94% C; 2,86% H; 4,32% N.

Beispiel 4

a) Eine Mischung aus 3,8 g (2,3-Dichlor-4-hydroxyphenyl)-2'-thienylmethanon und 2,0 g Hydroxylaminhydrochlorid in 20 ml Pyridin wird für 6 Stunden unter Rückfluß erhitzt. Danach wird das Pyridin im Vakuum verdampft und der Rückstand mit 5%iger Salzsäure versetzt. Die angesäuerte Mischung wird mit Äthylacetat extrahiert und der Extrakt mit Wasser gewaschen, getrocknet und zur Trockne eingedampft. Der Rückstand wird aus Äthanol und Wasser umkristallisiert. Das erhaltene (2,3-Dichlor-4-hdydroxyphenyl)-2'-thienylmethanon-oxim schmilzt bei 179–183°C.

b) 0,62 g Natriumhydrid wird zu einer Mischung aus 3,0 g (2,3-Dichlor-4-hydroxyphenyl)-2'-thienylmethanon-oxim in 30 ml Dimethylformamid und 30 ml Toluol gegeben, und die Mischung wird 2 Stunden auf 100°C und dann 2,5 Stunden auf 115°C erhitzt. Nach dem Abkühlen wird die Mischung tropfenweise mit einer Lösung von 1,9 g Äthylbromacetat in 10 ml Dimethylformamid versetzt. Nach beendeter Zugabe wird die Reaktionsmischung 2,25 Stunden gerührt, worauf Wasser zur Zersetzung von überschüssigem Natriumhydrid zugegeben wird. Die Reaktionsmischung wird mit Äthylacetat extrahiert, der Extrakt mit Wasser gewaschen, getrocknet und eingedampft. Der Rückstand wird aus Äthanol umkristallisiert. Der erhaltene {[7-Chlor-3-(2-thienyl)-1,2-benzisoxazol-6-yl]oxy}essigsäureäthylester schmilzt bei 142–143°C.

Analyse:

Berechnet für $C_{15}H_{12}ClNO_4S$:
53,33% C; 3,58% H; 4,15% N.
Gefunden:
53,28% C; 3,58% H; 4,17% N.

Beispiel 5

a) Eine Lösung von 18,0 g 2,3-Dichlor-4-hydroxybenzophenon und 9,3 g Hydroxyaminhydrochlorid in 100 ml Pyridin wird 2 Stunden unter Rückfluß erhitzt. Danach wird das Pyridin im Vakuum verdampft und die zurückbleibende Flüssigkeit zwischen 5%iger Salzsäure und Äthylacetat verteilt. Der Äthylacetatextrakt wird mit Wasser gewaschen, getrocknet und eingedampft, wobei das 2,3-Dichlor-4-hydroxybenzophenon-oxim erhalten wird.
Das 2,3-Dichlor-4-hydroxybenzophenon wird mit Benzoylchlorid an Stelle von 2-Fluorbenzoylchlorid unter den Bedingungen des Beispiels 1a) und 1b) hergestellt.

b) Eine Lösung von 2,3-Dichlor-4-hydroxybenzophenonoxim und 2,6 g Natriumhydrid in 50 ml Dimethylformamid und 50 ml Toluol wird auf 118°C erhitzt und 50 Minuten bei dieser Temperatur gehalten. Nach dem Abkühlen werden 7,9 g Äthylbromacetat in 50 ml Dimethylformamid zugetropft. Nach beendeter Zugabe wird die Mischung 40 Minuten bei Raumtempeatur gerührt. Wasser wird zu der gut gerührten Mischung zugetropft, um überschüssiges Natriumhydrid zu zersetzen. Das Toluol wird im Vakuum verdampft, der gebildete Niederschlag wird abfiltriert und mit Äther gewaschen. Der erhaltene [7-Chlor-3-phenyl-1,2-benzisoxazol-6-yl)oxy]-essigsäureäthylester schmilzt bei 130–132°C.

Analyse:

Berechnet für $C_{17}H_{14}ClNO_4$:
61,54% C; 4,25% H; 4,22% N.
Gefunden:
61,34% C; 4,15% H; 4,17% N.

Beispiel 6

6 ml 7 n-Natriumhydroxyd werden zu einer Lösung von 8,3 g [7-Chlor-3-phenyl-1,2-benzisoxazol-6-yl)oxy]essigsäureäthylester (Beispiel 5) in 160 ml Äthanol zugegeben, und die Reaktionsmischung wird für 45 Minuten unter Rückfluß erhitzt. Der Niederschlag wird abfiltriert, mit Äthanol und dann mit Äther gewaschen, in 200 ml heißem Wasser suspendiert, und die Suspension wird mit konzentrierter Salzsäure angesäuert. Die angesäuerte Mischung wird 1 Stunde gerührt und der graue Niederschlag abfiltriert. Es wird aus Äthylacetat umkristallisiert, wobei die reine [(7-Chlor-3-phenyl-1,2-benzisoxazol-6-yl)-oxy]essigsäure mit einem Schmelzpunkt von 219–221°C erhalten wird.

Analyse:

Berechnet für $C_{15}H_{10}ClNO_4$:
59,32% C; 3,32% H; 4,61% N.
Gefunden:
59,33% C; 3,38% H; 4,57% N.

Beispiel 7

19,8 g 3,2-Dichlor-4-hydroxyphenyl-2'-furylmethanon wird in 200 ml Dimethylformamid gelöst, und 4,8 g Natriumhydrid werden zu der Lösung zugegeben. Nachdem die Wasserstoffentwicklung nachgelassen hat, wird die Reaktionsmischung auf 130°C erhitzt. Die Mischung wird dann auf 5°C abgekühlt, und zu der gekühlten Mischung wird eine Lösung von 16,7 g Äthylbromacetat in 20 ml Dimethylformamid zugegeben. Die Reaktionsmischung wird 45 Minuten gerührt und dann, zur Bildung eines kristallinen Produktes, in Wasser gegossen. Die Kristalle werden abfiltriert, mit Äthanol und dann mit Äther gewaschen und schließlich aus Äthanol/Äthylacetat umkristallisiert. Der erhaltene [(7-Chlor-3-(2-furyl)-1,2-benzisoxazol-6-yl)-oxy]essigsäureäthylester schmilzt bei 151–152°C.

Analyse:

Berechnet für $C_{15}H_{12}ClNO_5$:
56,00% C; 3,76% H; 4,35% N.
Gefunden:
55,91% C; 3,83% H; 4,32% N.

Beispiel 8

10 ml einer 50%igen Natriumhydroxydlösung werden zu einer Lösung von 15,0 g [(7-Chlor-3-(2-furyl)-1,2-benzisoxazol-6-yl)-oxy]essigsäureäthylester (Beispiel 7) in 500 ml siedendem 95%igem Äthanol zugegeben, wobei das Natriumsalz unmittelbar ausfällt. Weitere 300 ml 95%iges Äthanol werden zugegeben und die Mischung für 30 Minuten zum Sieden erhitzt. Nach leichtem Abkühlen werden 100 ml einer 5%igen Salzsäure zugegeben. Das Reaktionsprodukt scheidet sich beim Abkühlen aus, worauf 250 ml Wasser zugegeben werden und die verdünnte Mischung mit Eis gekühlt wird. Der Niederschlag wird abfiltriert und aus Isopropanol umkristallisiert. Die erhaltene {[7-Chlor-3-(2-furyl)-1,2-benzisoxazol-6-yl]-oxy}essigsäure schmilzt bei 230–233°C.

Analyse:

Berechnet für $C_{13}H_8ClNO_5$:
53,17% C; 2,74% H; 4,77% N.
Gefunden:
52,83% C; 2,83% H; 4,74% N.

Beispiel 9

a) Eine Mischung aus 28,3 g (2,3-Dichlor-4-hydroxyphenyl)-(5'-methyl-2'-thienyl)methanon und 14,0 g Hydroxyaminhydrochlorid in 300 ml Pyridin wird 16 Stunden unter Rückfluß erhitzt, worauf das Lösungsmittel im Vakuum entfernt wird. Der Rückstand wird mit 5%iger Salzsäure behandelt, mit einer Mischung aus Dichloräthan und Äther extrahiert, getrocknet, und das Lösungsmittel wird verdampft. Das erhaltene Produkt wird mit Hexan gerieben, wobei das gewünschte (2,3-Dichlor-4-hydroxyphenyl)-(5'-methyl-2'-thienyl)-methanonoxim erhalten wird.

b) 5,3 g Natriumhydrid werden zu einer Mischung des obigen Oxims in 200 ml Dimethylformamid zugegeben, und die Reaktionsmischung wird für 1,5 Stunden bei 120°C, für 1 Stunde bei 130°C und für 1 Stunde bei °C gehalten und dann auf 35°C abgekühlt. Die gekühlte Mischung wird mit 18,3 g Bromessigsäureäthylester in 20 ml Dimethylformamid versetzt und 20 Minuten gerührt. Sie wird dann in gesättigte Natriumchloridlösung gegossen, wobei ein kristallines Produkt erhalten wird, welches abfiltriert wird. Die Kristalle werden mit Methanol und dann mit Äther gewaschen und aus Isopropanol umkristallisiert. Der erhaltene {[7-Chlor-3-(5-methyl-2-thienyl)-1,2-benzisoxazol-6-yl]-oxy}essigsäureäthylester schmilzt bei 149–150°C.

Analyse:

Berechnet für $C_{16}H_{14}ClNO_4S$:
54,62% C; 4,04% H; 3,98% N.
Gefunden:
54,60% C; 3,97% H; 3,98% N.

Beispiel 10

Wenn man in dem Verfahren von Beispiel 9a) das (2,3-Dichlor-4-hydroxyphenyl)-(5'-methyl-2'-thienyl)methanon durch (2,3-Dichlor-4-hydroxyphenyl)-(5'-methyl-2'-furyl)-methanon ersetzt und die Umsetzung gemäß Beispiel 9b) fortsetzt, erhält man den {[7-Chlor-3-(5-methyl-2-furyl)-1,2-benzisoxazol-6-yl]oxy}essigsäureäthylester mit einem Schmelzpunkt von 139–141°C.

Analyse:

Berechnet für $C_{16}H_{14}ClNO_5$:
57,23% C; 4,20% H; 4,17% N.
Gefunden:
57,23% C; 4,18% H; 3,93% N.

Beispiel 11

10 ml einer 50%igen Natriumhydroxydlösung werden zu einer Suspension von 15,0 g {[7-Chlor-3-(5-methyl-2-furyl)-1,2-benzisoxazol-6-yl]oxy}essigsäureäthylester (Beispiel 10) in 800 ml Äthanol gegeben. Die Reaktionsmischung wird unter kräftigem Rühren 1,5 Stunden unter Rückfluß erhitzt und dann mit 100 ml 5%iger Salzsäure versetzt. Die erhaltene Lösung wird abgekühlt und mit Wasser verdünnt, sobald das Produkt anfängt, auszukristallisieren. Die Kristalle werden abfiltriert und aus Methanol umkristallisiert. Die erhaltene {[7-Chlor-3-(5-methyl-2-furyl)-1,2-benzisoxazol-6-yl]oxy}-essigsäure schmilzt bei 217–219°C.

Analyse:

Berechnet für $C_{14}H_{10}ClNO$:
54,65% C; 3,28% H; 4,55% N.
Gefunden:
54,74% C; 3,40% H; 4,49% N.

Beispiel 12

a) Eine Mischung von 2,3-Dichlor-4-hydroxy-4'-methylbenzophenon und 12,5 g Hydroxylaminhydrochlorid in 200 ml Pyridin wird 2 Stunden unter Rückfluß erhitzt, worauf das Pyridin im Vakuum verdampft wird. Der Rückstand wird zwischen Äthylacetat und 5%iger Salzsäure verteilt, der Äthylacetatextrakt mit Wasser gewaschen, getrocknet und zur Trockne eingedampft, wobei das 2,3-Dichlor-4-hydroxy-4'-methylbenzophenonoxim erhalten wird.

b) Eine Mischung des obigen Oxims mit 5,2 g Natriumhydrid in 300 ml Dimethylformamid wird 3 Stunden bei 87°C gehalten. Nach Abkühlen auf Raumtemperatur werden 16,0 g Äthylbromacetat in 50 ml Dimethylformamid zugetropft. Nach beendeter Zugabe wird die Mischung 30 Minuten gerührt und dann in Wasser gegossen, wobei der {[7-Chlor-3-(4-tolyl)-1,2-benzisoxazol-6-yl]-oxy}-essigsäureäthylester, welcher bei 157–159°C schmilzt, ausfällt.

Beispiel 13

Eine Mischung aus 20 g {[7-Chlor-3-(4-tolyl)-1,2-benzisoxazol-6-yl]oxy}-essigsäureäthylester (Beispiel 12) und 15 ml 50%iges Natriumhydroxid in 60 ml Äthanol wird 1 Stunde unter Rückfluß erhitzt. Die heiße Mischung wird dann mit 500 ml Wasser verdünnt und mit konzentrierter Salzsäure angesäuert. Die angesäuerte Suspension wird erst 30 Minuten gerührt, dann filtriert, und der Filterkuchen wird aus Dimethylformamid umkristallisiert. Die erhaltene {[7-Chlor-3-(4-tolyl)-1,2-benzisoxazol-6-yl]oxy-}essigsäure schmilzt bei 247–260°C.

Analyse:

Berechnet für $C_{17}H_{14}ClNO_4$:
60,48% C; 3,78% H; 4,41% N.
Gefunden:
60,39% C; 3,77% H; 4,38% N.

Beispiel 14

a) Eine Mischung aus 41 g (2,3-Dichlor-4-hydroxyphenyl)-4'-chlorbenzophenon und 18,9 g Hydroxylaminhydrochlorid in 300 ml Pyridin wird 2 Stunden unter Rückfluß erhitzt. Das Pyridin wird dann im Vakuum entfernt und der Rückstand zwischen Äthylacetat und 5%iger Salzsäure verteilt. Der Äthylacetatanteil wird mit Wasser gewaschen, getrocknet und zur Trockne eingedampft, wobei man das 2,3-Dichlor-4-hydroxy-4'-chlorbenzophenonoxim erhält.
b) Eine Mischung aus 41,5 g des obigen Oxims und 7,9 g Natriumhydrid in 300 ml Dimethylformamid wird 2 Stunden bei 111°C gehalten. Nach dem Abkühlen auf Raumtemperatur wird eine Mischung aus 23 g Äthylbromacetat in 50 ml Dimethylformamid zugetropft. Nach beendeter Zugabe wird die Reaktionsmischung 30 Minuten gerührt und dann 16 Stunden stehengelassen. Beim Eingießen der Mischung in Wasser erhält man den {[7-Chlor-3-(4-chlorphenyl)-1,2-benzisoxazol-6-yl]oxy}-essigsäureäthylester als Niederschlag, der abfiltriert wird und bei 179°C schmilzt.

Beispiel 15

Eine Mischung aus 25 g {[7-Chlor-3-(4-chlorphenyl)-1,2-benzisoxazol-6-yl]oxy}-essigsäureäthylester (Beispiel 14) und 20 ml 50%iger Natronlauge in 400 ml Äthanol wird 1 Stunde unter Rückfluß gekocht, die heiße Mischung wird mit 300 ml Wasser verdünnt und dann mit konzentrierter Salzsäure angesäuert. Die angesäuerte Mischung wird 30 Minuten gerührt, filtriert, und der Filterkuchen wird aus einer Dimethylformamid/Äthylacetat-Mischung umkristallisiert. Die erhaltene {[7-Chlor-3-(4-chlorphenyl)-1,2-benzisoxazol-6-yl]oxy}essigsäure schmilzt bei 254–257°C.

Analyse:

Berechnet für $C_{15}H_9Cl_2NO_4$:
53,28% C; 2,68% H; 4,14% N.
Gefunden:
53,01% C; 2,39% H; 4,03% N.

Wenn nicht anders angegeben, wird in den obigen Beispielen das Oximvorprodukt aus dem entsprechenden Keton in ähnlicher Weise wie im Beispiel 1c) hergestellt.

Beispiel 16

Eine Suspension von 0,72 g {[7-Chlor-3-(2-thienyl)-1,2-benzisoxazol-6-yl]oxy}-essigsäureäthylester (Beispiel 4) und 10 ml konzentrierter wäßriger Natronlauge in 40 ml Äthanol wird 1 Stunde unter Rückfluß zum Sieden erhitzt und dann das Äthanol durch Verdampfen im Vakuum entfernt. Die erhaltene Suspension wird mit konzentrierter Salzsäure angesäuert und 30 Minuten bei Raumtemperatur gerührt. Das Rohprodukt wird abfiltriert und aus Äthanol umkristallisiert. Die erhaltene {[7-Chlor-3-(2-thienyl)-1,2-benzisoxazol-6-yl]oxy}-essigsäure schmilzt bei 217–220°C.

Analyse:

Berechnet für $C_{13}H_8ClNO_4S$:
50,41% C; 2,60% H; 4,52% N.
Gefunden:
50,13% C; 2,47% H; 4,48% N.

Beispiel 17

a) Eine Reaktionsmischung aus 32,8 g 3-Methyl-2-thiophencarbonsäurechlorid und 35,4 g 2,3-Dichloranisol und 26,7 g Aluminiumchlorid in 200 ml Schwefelkohlenstoff werden 40 Stunden unter Rückfluß erhitzt und dann in eine Mischung aus Eis und Salzsäure gegossen. Das erhaltene kristalline Produkt wird abfiltriert, dann mit Hexan gewaschen und aus einem Toluol-Hexan-Gemisch umkristallisiert. Das erhaltene (2,3-Dichlor-4-methoxy)-(3-methyl-2-thienyl)methanon schmilzt bei 136–138°C.

Analyse:

Berechnet für $C_{13}H_{10}Cl_2O_2S$:
51,84% C; 3,35% H; 10,65% S.
Gefunden:
51,81% C; 3,35% H; 10,85% S.

b) Eine Mischung von 0,6 g (2,3-Dichlor-4-Methoxy)-(3-methyl-2-thienyl)methanon und $NH_2OH \cdot HCl$ in Pyridin wird in das entsprechende Oxim verwandelt. Das Oxim (Isomerenmischung) (37,8 g) wird dann in 70 ml Dimethylformamid gelöst, und die Lösung wird zu einer Suspension von 3,3 g Natriumhydrid in 100 ml Dimethylformamid gegeben. Nach beendeter Reaktion wird die Mischung in Wasser gegossen. Die wäßrige Mischung wird mit verdünnter Salzsäure auf einen pH-Wert von 6–7 eingestellt. Der erhaltene Niederschlag wird abfiltriert, gut mit Äther gewaschen und aus einer Mischung von Toluol und Hexan umkristallisiert. Das erhaltene 7-Chlor-3-(3-methyl-2-thienyl)-6-methoxy-1,2-benzisoxazol schmilzt bei 154–156°C.

Analyse.

Berechnet für $C_{13}H_{10}ClNO_2S$:
55,81% C; 3,60% H; 5,01% N; 11,46% S.
Gefunden:
55,90% C; 3,54% H; 4,98% N; 11,22% S.

c) Eine Mischung aus 13,3 g 7-Chlor-3-(3-methyl-2-thienyl)-6-methoxy-1,2-benzisoxazol und 25 g $BBr_3$ in $CH_2Cl_2$ wird 18 Stunden unter Rückfluß erhitzt, dann in Wasser gegossen und mit Äther extrahiert. Der Ätherextrakt wird getrocknet, eingedampft und mit Hexan gerieben. Das erhaltene 7-Chlor-6-hydroxy-3-(3-methyl-2-thienyl)-1,2-benzisoxazol schmilzt bei 197–198°C.

Analyse:

Berechnet für $C_{12}H_8ClNO_2S$:
54,24% C; 3,03% H; 5,27% N.
Gefunden:
54,22% C; 3,05% H; 5,08% N.

d) Eine Mischung aus 10,3 g 7-Chlor-6-hydroxy-3-(3-methyl-2-thienyl)-1,2-benzisoxazol in 60 ml DMF wird zu einer Suspension von 1,1 g NaH in 40 ml DMF gegeben. 6,7 g Bromessigsäureäthylester wird zugefügt und die Reaktionsmischung 30 Minuten auf 50°C erwärmt. Nach Zugabe von 100 ml $H_2O$ und 25%iger wäßriger Natronlauge wird die Reaktionsmischung 3 Stunden auf 90°C erhitzt. Sie wird dann in Wasser gegossen und mit konzentrierter Salzsäure angesäuert. Die angesäuerte Mischung wird mit Äther extrahiert, mit Wasser gewaschen und getrocknet. Nach dem Verdampfen und Umkristallisieren aus einer Essigsäureäthylester/Hexan-Mischung erhält man die {[7-Chlor-3-(3-methyl-2-thienyl)-1,2-benzisoxazol-6-yl]oxy}-essigsäure.

Analyse:

Berechnet für $C_{14}H_{10}ClNO_4S$:
51,93% C; 3,11% H; 4,33% N.
Gefunden:
51,93% C; 3,05% H; 4,28% N.

Beispiel 18

Eine Mischung aus 15,0 g [7-Chlor-3-(5-methyl-2-thienyl)-1,2-benzisoxazol-6-yl]oxy-essigsäureäthylester (Beispiel 9b) und 10 ml 50%ige Natronlauge in 800 ml Äthanol wird 30 Minuten unter Rückfluß erhitzt, wonach 100 ml 5%iger Salzsäure zur Bildung einer homogenen Lösung zugegeben werden. Beim Abkühlen der Lösung scheidet sich ein kristallines Produkt ab, worauf weiteres Wasser zugegeben wird. Das Reaktionsprodukt wird abfiltriert und aus Isopropanol umkristallisiert. Die erhaltene {[7-Chlor-3-(5-methyl-2-thienyl)-1,2-benzisoxazol-6-yl]oxy}-essigsäure schmilzt bei 235–238°C.

Analyse:

Berechnet für $C_{14}H_{10}ClNO_4S$:
51,93% C; 3,11% H; 4,33% N; 9,91% S.
Gefunden:
51,69% C; 3,14% H; 4,20% N; 10,02% S.

Beispiel 19

18,0 g 3-Furoylchlorid und 24,7 g 2,3-Dichloranisol werden in 125 ml Schwefelkohlenstoff gelöst und mit 18,7 g $AlCl_3$ behandelt, zuerst bei 5°C und dann bei Raumtemperatur. Nach 5 Stunden wird die Reaktionsmischung mit Eis/HCl abgeschreckt und mit $CH_2Cl_2$ extrahiert. Trocknen und Verdampfen ergibt ein kristallines Produkt, das mit Hexan verrieben wird. Das erhaltene 4-(3-furoyl)-2,3-dichloranisol schmilzt bei 118–122°C.

Durch Zugabe von 16 ml konzentrierter HCl (0,186 Mol) zu 14,2 g Pyridin, Erhitzen der Mischung auf 210°C unter Stickstoff und Abdestillieren des Wassers wird eine Schmelze hergestellt. 5,0 g Anisol werden portionsweise zugegeben, die Mischung wird eine weitere Stunde erhitzt, die Lösung auf Eis gegossen und mit Äthylacetat extrahiert. Nach dem Trocknen und Verdampfen erhält man das 4-(3-Furoyl)-2,3-dichlorphenol, welches bei 138–142°C schmilzt.

Das Phenol wird mit Hydroxylaminhydrochlorid in Pyridin vermischt und 3 Stunden unter Rückfluß erhitzt. Das Pyridin wird verdampft, die erhaltene Mischung mit Salzsäure angesäuert und dann mit Essigsäureäthylester extrahiert. Der Äthylacetatextrakt wird mit Wasser gewaschen, getrocknet und zur Trockne eingedampft. Man erhält ein Oxim, welches in 100 ml DMF gelöst und zu einer Suspension von 7,0 g NaH in 100 ml DMF gegeben wird. Die Reaktionsmischung wird 2 Stunden auf 120°C erhitzt, dann auf 45°C abgekühlt, worauf 24,0 g Bromessigsäureäthylester in 20 ml DMF zugegeben werden. Die Reaktionsmischung wird mit Kochsalzlösung abgeschreckt, die erhaltene feste Substanz abfiltriert und erst mit Äthanol und dann mit Äther gewaschen, wobei man den rohen Phenoxyester erhält. Hydrolyse des Rohesters für 45 Minuten unter Rückfluß in 500 ml Äthanol, welches 10 ml 50%ige Natronlauge enthält, ergibt, nach Ansäuern und Abkühlen, eine kristalline Säure. Die Säure wird in siedendem Methanol suspendiert und die Suspension mit DMF bis zur Lösung versetzt. Nach der Zugabe von Wasser setzt sofort Kristallisation ein und man erhält die {[7-Chlor-3-(3-furyl)-1,2-benzisoxazol-6-yl]oxy}-essigsäure mit einem Schmelzpunkt von 225–227°C.

Analyse:

Berechnet für $C_{13}H_8ClNO_5$:
53,17% C; 2,74% H; 4,77% N.
Gefunden:
53,36% C; 2,85% H; 4,71% N.

Beispiel 20

a) Eine Lösung von 24,6 g 2,6-Difluorbenzoylchlorid in 100 ml 1,2-Dichloräthan wird innerhalb von 30 Minuten portionsweise mit 18,5 g $AlCl_3$ versetzt, und zu der Mischung wird eine Lösung von 22,5 g 2,3-Dichloranisol in 100 ml 1,2-Dichloräthan zugegeben. Die Mischung wird 1 Stunde gerührt und dann auf 100 ml konzentrierte HCl und Eis gegossen. Die organische Schicht wird

abgetrennt und die wäßrige Schicht mit $CHCl_3$ extrahiert. Der organische Extrakt wird mit Wasser gewaschen, getrocknet ($Na_2SO_4$) und eingedampft. Man erhält ein Öl, das aus Hexan kristalliert. Das feste 2,3-Dichlor-4-methoxy-2',6'-difluorbenzophenon schmilzt bei 94–96°C nach Umkristallisation aus Äther.

Analyse:

Berechnet für $C_{14}H_8Cl_2F_2O_2$:
53,02% C; 2,54% H; 11,98% F.
Gefunden:
53,21% C; 2,50% H; 12,08% F.

b) Eine Mischung aus 50,5 g 2,3-Dichlor-4-methoxy-2',6'-difluorbenzophenon, 44,27 g Hydroxylaminhydrochlorid in 200 ml Pyridin wird 48 Stunden unter Rückfluß erhitzt. Das Pyridin wird im Vakuum verdampft und der Rückstand zwischen 5%iger Salzsäure und Äthylacetate verteilt. Der Extrakt wird mit Wasser gewaschen, über $Na_2SO_4$ getrocknet und verdampft. Man erhält ein Isomerengemisch. Eine Analysenprobe von 2,3-Dichlor-4-methoxy-2',6'-difluorbenzophenon-oxim, Schmelzpunkt 173–189°C, wird aus 95%igem Äthanol umkristallisiert.

Analyse:

Berechnet für $C_{14}H_9Cl_2F_2NO_2$:
50,62% C; 2,73% H; 4,22% N.
Gefunden:
50,84% C; 2,68% H; 4,14% N.

c) Eine Mischung von 5 g NaH in 200 ml DMF wird unter Stickstoff tropfenweise mit 48 g 2,3-Dichlor-4-methoxy-2',6'-difluorbenzophenon-oxim in 250 ml DMF versetzt, wobei die Temperatur auf etwa 40°C gehalten wird. Nach der Zugabe wird die Mischung 30 Minuten gerührt und dann in Eiswasser gegossen. Das Rohprodukt, eine Mischung der Isomeren, wird abfiltriert und an Silikagel mit $CHCl_3$ als Elutionsmittel chromatographiert. Das erhaltene 7-Chlor-3-(2,6-difluorphenyl)-6-methoxy-1,2-benzisoxazol wird zur Analyse aus Toluol umkristallisiert. Es schmilzt bei 175 bis 179°C.

Analyse:

Berechnet für $C_{14}H_8ClF_2NO_2$:
56,87% C; 2,73% H; 4,74% N.
Gefunden:
56,99% C; 2,64% H; 4,64% N.

d) Eine Mischung aus 10,8 g 7-Chlor-3-(2,6-difluorphenyl)-6-methoxy-1,2-benzisoxazol und 40,3 g Pyridinhydrochlorid wird 1 Stunde auf 200°C erhitzt und dann in kräftig gerührtes Eiswasser gegossen. Das ausgefallene 7-Chlor-3-(2,6-difluorphenyl)-6-hydroxy-1,2-benzisoxazol wird abfiltriert und getrocknet. Eine aus Toluol umkristallisierte Analysenprobe schmilzt bei 216 bis 220°C.

Analyse:

Berechnet für $C_{13}H_6ClF_2NO_2$:
55,43% C; 2,15% H; 4,97% N.
Gefunden:
55,59% C; 2,29% H; 4,96% N.

e) Eine Mischung aus 1,18 g NaH und 9,2 g 7-Chlor-3-(2,6-difluorphenyl)-6-hydroxy-1,2-benzisoxazol in 15 ml DMF wird 1 Stunde gerührt. Eine Lösung von 6,06 g Bromessigsäureäthylester in 40 ml DMF wird zugetropft und die Mischung für ½ Stunde gerührt. Nach Zugabe von 10 ml 50%iger Natronlauge wird die Reaktionsmischung 1 Stunde auf 80°C erwärmt. Die warme Lösung wird mit konzentrierter Salzsäure angesäuert. {[7-Chlor-3-(2,6-difluorphenyl)-1,2-benzisoxazol-6-yl]oxy}-essigsäure wird durch Zugabe von Wasser ausgefällt, abfiltriert, getrocknet und aus Toluol-Acetonitril umkristallisiert. Sie hat einen Schmelzpunkt von 170–172°C.

Analyse:

Berechnet für $C_{15}H_8ClF_2NO_4$:
53,04% C; 2,37% H; 4,12% N.
Gefunden:
53,17% C; 2,38% H; 4,18% N.

Beispiel 21

a) Eine Mischung aus 20,36 g $\alpha$-Fluorzimtsäurechlorid und 14,4 g $AlCl_3$ in 100 ml 1,2-Dichloräthan wird 1 Stunde gerührt und tropfenweise mit 17,7 g 2,3-Dichloranisol in 100 ml 1,2-Dichloräthan versetzt. Die Reaktionsmischung wird 1 Stunde auf etwa 80°C erwärmt und dann auf 100 ml konzentrierte Salzsäure und Eis gegossen. Die wäßrige Schicht wird mit $CHCl_3$ extrahiert, und die vereinigten organischen Schichten werden mit Wasser gewaschen, über $Na_2SO_4$ getrocknet und verdampft, bis eine feste Substanz erhalten wird. Diese wird mit Hexan gerieben, wobei man das 4-(Trans-$\alpha$-fluorcinnamoyl)-2,3-dichloranisol erhält. Eine Probe wird aus Toluol umkristallisiert, sie schmilzt bei 137–138°C.

Analyse:

Berechnet für $C_{16}H_{11}Cl_2FO_2$:
59,10% C; 3,41% H; 5,48% F.
Gefunden:
59,38% C; 3,46% H; 5,86% F.

b) Eine Mischung aus 26,6 g 4-(Trans-$\alpha$-fluorcinnamoyl)-2,3-dichloranisol und 22,7 g Hydroxylaminohydrochlorid in Pyridin wird 18 Stunden zum Sieden unter Rückfluß erhitzt. Das Pyridin wird im Vakuum verdampft und der Rückstand mit 5%iger HCl verrieben. Der Niederschlag wird abfiltriert, getrocknet und mit Äther-Hexan gewaschen. Man erhält eine Mischung der Isomeren. Eine Probe von 4-(Trans-$\alpha$-fluorcinnamoyl)-2,3-dichloranisoloxim wird umkristallisiert und schmilzt bei 222 bis 238°C.

Analyse:

Berechnet für $C_{16}H_{12}Cl_2FNO_2$:
56,49% C; 3,56% H; 4,12% N.
Gefunden:
56,47% C; 3,51% H; 4,02% N.

c) Eine Mischung aus 0,43 g NaH und 4 g 4-(Trans-$\alpha$-fluorcinnamoyl)-2,3-dichloranisol-oxim in 60 ml DMF wird eine halbe Stunde gerührt und dann in Eiswasser gegossen. Der gebildete Niederschlag wird abfiltriert und getrocknet. Das erhaltene 7-Chlor-3-(trans-$\beta$-fluorstyryl)-6-methoxy-1,2-benzisoxazol wird durch Umkristallisieren aus Toluol gereinigt. Es schmilzt bei 155–161°C.

Analyse:

Berechnet für $C_{16}H_{11}ClFNO_2$:
63,27% C; 3,65% H; 4,61% N.
Gefunden:
63,18% C; 3,42% H; 4,65% N.

d) Eine Mischung aus 1,4 g 7-Chlor-3-(trans-$\beta$-fluorstyryl)-6-methoxy-1,2-benzisoxazol und 5,6 g Pyridinhydrochlorid wird 1 Stunde auf 200°C erhitzt und in kräftig gerührtes Eiswasser gegossen. Der 7-Chlor-3-(trans-$\beta$-fluorstyryl)-6-hydroxy-1,2-benzisoxazol-Niederschlag wird abfiltriert und getrocknet. Er schmilzt bei 226–229°C.

Analyse:

Berechnet für $C_{15}H_9ClFNO_2$:
62,19% C; 3,13% H; 4,84% N.
Gefunden:
62,43% C; 3,17% H; 4,92% N.

e) 6,77 g 7-Chlor-3-(trans-$\beta$-fluorstyryl)-6-hydroxy-1,2-benzisoxazol in 50 ml DMF wird unter

Stickstoff zu einer Mischung von 0,84 g NaH und 100 ml DMF zugetropft, die Mischung wird eine Stunde gerührt und tropfenweise mit 4,2 g Bromessigsäureäthylester versetzt. Die Reaktionsmischung wird ½ Stunde gerührt, mit 15 ml 50%iger Natronlauge versetzt und dann 1 Stunde auf 80°C erwärmt. Die Reaktionsmischung wird mit konzentrierter Salzsäure angesäuert. Nach Zugabe von Wasser bildet sich ein Niederschlag, der abfiltriert und getrocknet wird. Das Rohprodukt wird aus 95%igem Äthanol umkristallisiert. Die erhaltene {[7-Chlor-3-(trans-β-fluorstyryl)-1,2-benzisoxazol-6-yl]oxy}-essigsäure schmilzt bei 200–203°C.

Analyse:

Berechnet für $C_{17}H_{11}ClFNO_4$:
58,72% C; 3,18% H; 4,03% N.
Gefunden:
59,03% C; 3,29% H; 4,01% N.

Beispiel 22

a) Eine Lösung von 52,23 g 2,4-Difluorbenzoylchlorid in 200 ml 1,2-Dichloräthan wird in 30 Minuten mit 40 g $AlCl_3$ versetzt, und eine Lösung von 48,3 g 2,3-Dichloranisol in 100 ml 1,2-Dichloräthan wird zugetropft. Man beobachtet eine schwache Gasentwicklung, und die Temperatur steigt auf etwa 30°C. Die Reaktionsmischung wird auf 43°C erwärmt, und die Gasentwicklung dauert etwa 30 Minuten. Die Mischung wird auf eine Mischung aus konzentrierter Salzsäure und Eis gegossen. Die organische Phase wird abgetrennt, und die wäßrige Phase wird zweimal mit $CHCl_3$ extrahiert. Die vereinigten organischen Phasen werden mit Wasser gewaschen, getrocknet ($Na_2SO_4$) und eingedampft, wobei ein Öl erhalten wird, welches mit Hexan gerieben wird. Das 2,3-Dichlor-4-methoxy-2',4'-difluorbenzophenon, aus Äther umkristallisiert, hat einen Schmelzpunkt von 139–141°C.

Analyse:

Berechnet für $C_{14}H_8Cl_2F_2O_2$:
53,02% C; 2,54% H; 11,98% N.
Gefunden:
53,09% C; 2,43% H; 11,84% N.

b) 67 g 2,3-Dichlor-4-methoxy-2',4'-difluorbenzophenon in 300 ml Pyridin werden mit 58 g Hydroxylaminhydrochlorid versetzt, und die erhaltene Mischung wird 18 Stunden unter Rückfluß erhitzt. Das Pyridin wird im Vakuum verdampft und der Rückstand zwischen 5%iger HCl und Äthylacetat verteilt. Der Äthylacetatextrakt wird mit Wasser gewaschen, über $Na_2SO_4$ getrocknet und eingedampft. Man erhält das 2,3-Dichlor-4-methoxy-2',6'-difluorbenzophenon-oxim in der Form eines Isomerengemisches. Eine Probe wird aus 95%igem Äthanol umkristallisiert. Sie schmilzt bei 180–186°C.

Analyse:

Berechnet für $C_{14}H_9Cl_2F_2NO_2$:
50,62% C; 2,73% H; 4,22% N.
Gefunden:
50,63% C; 2,80% H; 4,55% N.

c) Eine Mischung von 5,7 g NaH in 100 ml DMF wird tropfenweise mit einer Lösung von 53 g 2,3-Dichlor-4-methoxy-2',6'-difluorbenzophenon in 250 ml DMF versetzt, wobei die Temperatur bei etwa 30°C gehalten wird. Die Mischung wird 1½ Stunden gerührt, und durch Zugabe von Wasser wird ein Niederschlag ausgefällt. Das Rohprodukt, eine Mischung von Isomeren, wird an Silikagel mit Toluol-Hexan als Elutionsmittel chromatographiert. Das reine 7-Chlor-3-(2,4-difluorphenyl)-6-methoxy-1,2-benzisoxazol wird aus Toluol umkristallisiert und schmilzt bei 189–191°C.

Analyse:

Berechnet für $C_{14}H_8ClF_2NO_2$:
56,87% C; 2,73% H; 4,74% N.
Gefunden:
56,94% C; 2,77% H; 4,66% N.

d) Eine feste Mischung aus 7,1 g 7-Chlor-3-(2,4-difluorphenyl)-6-methoxy-1,2-benzisoxazol und 27,8 g Pyridinhydrochlorid wird 1 Stunde auf 200°C erhitzt und zur Fällung des Niederschlags in kräftig gerührtes Eiswasser gegossen. Das abfiltriete und getrocknete 7-Chlor-3-(2,4-difluorphenyl)-6-hydroxy-1,2-benzisoxazol wird aus Toluol umkristallisiert und schmilzt bei 186 bis 190°C.

Analyse:

Berechnet für $C_{13}H_6ClF_2NO_2$:
55,43% C; 2,15% H; 4,97% N.
Gefunden:
55,68% C; 2,16% H; 4,92% N.

e) Eine Mischung aus 0,86 g NaH in 100 ml DMF wird unter Stickstoff tropfenweise mit einer Lösung von 6,9 g 7-Chlor-3-(2,4-difluorphenyl)-6-hydroxy-1,2-benzisoxazol in 50 ml DMF versetzt, und 4,41 g Bromessigsäureäthylester in 25 ml DMF werden zugegeben. Die Mischung wird ½ Stunde gerührt, 15 ml 50%ige NaOH wird zugegeben und das Ganze 1 Stunde auf 80—85°C erwärmt. Konzentrierte Salzsäure wird dann zugegeben, bis die Mischung sauer ist. Die {[7-Chlor-3-(2,4-difluorphenyl)-1,2-benzisoxazol-6-yl]oxy}-essigsäure wird durch Zugabe von Wasser ausgefällt und aus Toluol-Acetonitril umkristallisiert. Sie schmilzt bei 200—203°C.

Analyse:

Berechnet für $C_{15}H_8ClF_2NO_4$:
53,04% C; 2,37% H; 4,12% N.
Gefunden:
53,24% C; 2,55% H; 4,39% N.

## Beispiel 23

a) 94,64 g Dimethylsulfat werden innerhalb von 40 Minuten zu einer gekühlten und gerührten Lösung von 122,25 g 2,5-Dichlorphenol und 31,5 g Natriumhydroxyd in 300 ml Wasser gegeben. Die Mischung wird 1 Stunde bei Raumtemperatur gerührt, 2 Stunden zum Rückfluß erhitzt und dann auf Raumtemperatur abgekühlt. Die organische Schicht wird abgetrennt und mit den Ätherextrakten der zurückbleibenden wäßrigen Schicht vereinigt. Die Ätherlösung wird getrocknet (gesättigtes NaCl, $Na_2SO_4$, $K_2CO_3$) und der Äther entfernt, wobei man das 2,5-Dichloranisol erhält. Durch Destillation erhält man eine farblose Flüssigkeit (115°C im Saugvakuum).
Eine Lösung von 69,7 g o-Fluorbenzoylchlorid in 25 ml Schwefelkohlenstoff wird bei Raumtemperatur zu einer Suspension von 58,67 g Aluminiumchlorid in 450 ml Schwefelkohlenstoff zugegeben, und die Mischung wird bei Raumtemperatur mit 70,81 g 2,5-Dichloranisol in 25 ml Schwefelkohlenstoff versetzt. Die Mischung wird 4 Stunden bei Zimmertemperatur gerührt, gekühlt, und 58 g $AlCl_3$ werden zugegeben. Die erhaltene Mischung wird zwei Stunden unter Rückfluß gekocht und dann 18 Stunden bei Raumtemperatur gerührt. Sie wird anschließend auf eine Mischung von Eis und HCl gegossen und mit Methylendichlorid extrahiert. Das erhaltene Öl wird mit Hexan/Äther gerieben, wobei sich eine feste Substanz bildet. Durch Umkristallisierung aus Diisopropyläther erhält man eine feste Substanz, die zum Teil bei 94°C und dann bei 120°C schmilzt. Die feste Substanz, die nach Entfernung des Lösungsmittels vom Filtrat zurückbleibt, wird mit Äther-Petroläther behandelt, worauf man das 2,5-Dichlor-4-(2-fluorbenzoyl)anisol erhält, welches, nach Umkristallisierung aus Methanol, bei 100—103°C schmilzt.

Analyse:

Berechnet für $C_{14}H_9Cl_2FO_2$:
56,21% C; 3,04% H.
Gefunden:
56,26% C; 3,00% H.

b) Eine Mischung aus 3 g 2,5-Dichlor-4-(2-Fluorbenzoylanisol und 1,4 g Hydroxylaminhydrochlorid in 25 ml Pyridin wird mehrere Stunden unter Rückfluß gekocht, bis kein Keton mehr zurückbleibt. Das Pyridin wird im Hochvakuum entfernt, der Rückstand mit Wasser verdünnt und mit Chloroform extrahiert. Die Chloroformlösung wird getrocknet, das Chloroform entfernt und der zurückbleibende Feststoff aus Äther umkristallisiert. Das erhaltene (z)-2'-Fluor-2,5-dichlor-4-methoxybenzophenon-oxim schmilzt bei 188°C.

Analyse:

Berechnet für $C_{14}H_{10}Cl_2FNO_2$:
53,52% C; 3,21% H; 4,64% N.
Gefunden:
53,44% C; 3,22% H; 4,36% N.

c) Eine gerührte Suspension von 0,6 g Natriumhydrid in 20 ml DMF wird tropfenweise mit einer Lösung von 3,14 g (z)-2'-Fluor-2,5-dichlor-4-methoxybenzophenon-oxim in 20 ml DMF versetzt. Die Mischung wird 18 Stunden bei Raumtemperatur gerührt, in Eis/Wasser gegossen und mit Chloroform extrahiert. Die Chloroformlösung wird mit Wasser und gesättigter Natriumchloridlösung gewaschen und über $Na_2SO_4$–$MgSO_4$ getrocknet. Nach dem Entfernen des Chloroforms erhält man einen Rückstand, der beim Behandeln mit Äther-Petroläther festes 5-Chlor-3-(2-fluorphenyl)-6-methoxy-1,2-benzisoxazol mit einem Schmelzpunkt von 165–166°C ergibt.

Analyse:

Berechnet für $C_{14}H_9ClNO_2$:
60,55% C; 3,27% H; 5,04% N; 12,77% Cl.
Gefunden:
60,71% C; 3,14% H; 4,99% N; 12,75% Cl.

d) Eine Lösung von 1 g 5-Chlor-3-(2-fluorphenyl)-6-methoxy-1,2-benzisoxazol und 1,3 ml Bortribromid in 30 ml Dichloräthan wird für etwa 24 Stunden unter Rückfluß erhitzt. Die Reaktionsmischung wird in Eis/Wasser gegossen und mit Dichlormethan extrahiert. Das feste 5-Chlor-3-(2-fluorphenyl)-6-hydroxy-1,2-benzisoxazol schmilzt bei 180–182°C.

Analyse:

Berechnet für $C_{13}H_7FClNO_2$:
59,22% C; 2,68% H; 5,31% N.
Gefunden:
58,90% C; 2,68% H; 5,17% N.

e) Eine Lösung von 14,8 g 5-Chlor-3-(2-fluorphenyl)-6-hydroxy-1,2-benzisoxazol in 40 ml DMF wird bei Raumtemperatur zu einer Suspension von 3,0 g Natriumhydrid in 40 ml DMF getropft, und die Mischung wird ½ Stunde bei Raumtemperatur gerührt. Dann wird eine Lösung von 10,31 g Bromessigsäureäthylester in 40 ml DMF zugetropft und das Ganze 18 Stunden bei Raumtemperatur gerührt. Weitere 0,3 g NaH suspendiert in DMF und 1 g Bromessigsäureäthylester werden nacheinander zugegeben, die Mischung wird 1 ½ Stunden auf 50°C erwärmt, abgekühlt, auf eine Mischung von Eis und Wasser gegossen und mit Äther extrahiert. Die Ätherextrakte werden mit Wasser und gesättigter Natriumchloridlösung gewaschen, über Natriumsulfat getrocknet, und das Lösungsmittel wird entfernt. Nach Anreiben der festen Substanz mit Äther-Petroläther (1 : 1) erhält man den {[5-Chlor-3-(2-fluorphenyl)-1,2-benzisoxazol-6-yl]oxy}-essigsäureäthylester, welcher bei 114–155°C schmilzt.

Analyse:

Berechnet für $C_{17}H_{13}ClFNO_4$:
58,37% C; 3,75% H; 4,00% N.
Gefunden:
58,15% C; 3,66% H; 3,93% N.

f) Eine Mischung aus 14 g {[5-Chlor-3-(2-fluorphenyl)-1,2-benzisoxazol-6-yl]oxy}-essigsäureäthylester, 8 g Natriumhydroxyd, 500 ml Äthanol und 300 ml Wasser wird 5 Stunden unter Rückfluß gekocht, in einem Eisbad abgekühlt und mit konzentrierter Salzsäure angesäuert. Die Suspension wird mit Chloroform extrahiert, der Extrakt mit konzentriertem Natriumchlorid getrocknet, und das Chloroform wird entfernt. Die erhaltene feste Substanz, die bei 214–215°C schmilzt, wird aus einer Mischung aus Acetonitril-Toluol (3 : 2) umkristallisiert, wobei man die {[5-Chlor-3-(2-fluorphenyl)-1,2-benzisoxazol-6-yl]oxy}-essigsäure erhält.

Analyse:

Berechnet für $C_{15}H_9ClFNO_4$:
56,00% C; 2,82% H; 4,35% N.
Gefunden:
55,79% C; 2,69% H; 4,27% N.

Beispiel 24

a) Eine Lösung von 57,6 g 3,4-Dichlorbenzoylchlorid in 150 ml 1,2-Dichloräthan wird portionsweise innerhalb von 30 Minuten mit 36,7 g $AlCl_3$ versetzt, und zu dem erhaltenen Gemisch werden 44,26 g 2,3-Dichloranisol in 150 ml 1,2-Dichloräthan zugetropft. Es tritt Gasentwicklung ein, und die Mischung wird 1 Stunde auf 60°C erwärmt. Sie wird dann auf 150 ml konzentrierte Salzsäure und 150 ml Eis gegossen und mit $CHCl_3$ und danach mit Äthanol extrahiert. Die gebildete organische Schicht wird mit 10%igem $K_2CO_3$ und mit Wasser gewaschen, über $Na_2SO_4$ getrocknet und eingedampft. Das erhaltene 2,3-Dichlor-4-methoxy-3',4'-dichlorbenzophenon schmilzt bei 113 bis 140°C.

b) Eine Mischung aus 53 g 2,3-Dichlor-4-methoxy-3',4'-dichlorbenzophenon und 40 g $AlCl_3$ in 400 ml Benzol wird 5 Stunden unter Rückfluß erhitzt, auf Raumtemperatur abgekühlt und bei dieser Temperatur etwa 18 Stunden gehalten. Die Mischung wird dann auf 200 ml konzentrierte HCl und 200 ml Eis gegossen und ½ Stunde bei Zimmertemperatur gerührt. Die Mischung wird mit Essigsäureäthylester versetzt, die Essigester/Benzol-Schicht mit Wasser gewaschen und über $Na_2SO_4$ getrocknet. Beim Eindampfen erhält man das 2,3-Dichlor-4-hydroxy-3',4'-dichlorbenzophenon, welches bei 179–180°C schmilzt.

c) Eine Mischung von 35,43 g 2,3-Dichlor-4-hydroxy-3',4'-dichlorbenzophenon und 15,29 g Hydroxylaminhydrochlorid in 300 ml Pyridin wird 2 Stunden unter Rückfluß erhitzt, das Pyridin wird im Vakuum verdampft und der Rückstand zwischen Äthylacetat und 5%iger HCl verteilt. Der Rückstand wird mit Wasser gewaschen, über $Na_2SO_4$ getrocknet und eingedampft. Man erhält das entsprechende Oxim.
Eine Lösung von 35 g des Oxims in 300 ml DMF wird mit 6,0 g NaH versetzt, und die Mischung wird eine Stunde lang auf eine Innentemperatur von 103°C und dann 45 Minuten lang auf eine Innentemperatur von 100°C erhitzt. Die Reaktionsmischung wird auf Raumtemperatur abgekühlt und tropfenweise mit einer Lösung von 18,37 g Bromessigsäureäthylester in 50 ml DMF versetzt. Die Mischung wird 64 Stunden gerührt, auf Wasser gegossen, der gebildete Niederschlag wird abfiltriert und mit Hexan gewaschen. Das Rohprodukt wird aus Äthanol umkristallisiert. Der erhaltene {[7-Chlor-3-(3,4-dichlorphenyl)-1,2-benzisoxazol-6-yl]oxy}-essigsäureäthylester schmilzt bei 123–125°C.
Eine Suspension von 19 g des Esters in 400 ml Äthanol wird mit 20 ml 50%iger NaOH versetzt, worauf sich ein Niederschlag bildet. Die heterogene Mischung wird 1 Stunde unter Rückfluß erhitzt, die heiße Mischung zuerst mit 400 ml Wasser unnd dann mit konzentrierter Salzsäure versetzt, bis sie sauer ist. Die Suspension wird ½ Stunde gerührt, filtriert und der Niederschlag aus DMF-Äthylacetat umkristallisiert. Die erhaltene {[7-Chlor-3-(3,4-dichlorphenyl)-1,2-benzisoxazol-6-yl]oxy}-essigsäure schmilzt bei 222–224°C.

Analyse:

Berechnet für $C_{15}H_8Cl_3NO_4$:
48,35% C; 2,16% H; 3,76% N.
Gefunden:
58,40% C; 2,03% H; 3,93% N.

Beispiel 25

a) Die Friedel-Crafts-Reaktion von Beispiel 24a wird mit 48,13 g o-Chlorbenzoylchlorid, 36,7 g $AlCl_3$ und 44,26 g Dichloranisol wiederholt. Man erhält eine weiße, kristalline Substanz von 2,3-Dichlor-4-methoxy-2'-chlorbenzophenon, die bei 117–120°C schmilzt.

b) Das Verfahren von Beispiel 24b wird mit 68 g 2,3-Dichlor-4-methoxy-2'-chlorbenzophenon und 58,6 g $AlCl_3$ in 500 ml Benzol wiederholt. Das erhaltene Produkt wird aus Toluol umkristallisiert. Das 2,3-Dichlor-4-hydroxy-2'-chlorbenzophenon schmilzt bei 74–77°C.

c) Eine Lösung von 53 g 2,3-Dichlor-4-hydroxy-2'-chlorbenzophenon in 350 ml Pyridin wird mit 25,02 g Hydroxylaminhydrochlorid versetzt und die Mischung 2 Stunden unter Rückfluß erhitzt. Das Pyridin wird im Vakuum verdampft und der Rückstand zwischen Essigester und 5%iger HCl verteilt. Der Essigesterextrakt wird mit Wasser gewaschen, über $Na_2SO_4$ getrocknet und ein-

gedampft, wobei man das entsprechende Oxim erhält. Eine Lösung von 49 g des Oxims in 300 ml DMF wird mit 9,12 g NaH versetzt, und die Mischung wird 1 Stunde auf eine Innentemperatur von 80–84°C erhitzt. Die Reaktionsmischung wird dann auf Raumtemperatur abgekühlt und tropfenweise mit einer Lösung von 27,5 g Bromessigsäureäthylester in 50 ml DMF versetzt. Die Mischung wird ½ Stunde gerührt, und Wasser wird zugegeben, um den Überschuß an NaH zu zersetzen. Das Reaktionsprodukt wird mit Essigester extrahiert, der Essigesterextrakt mit 10%iger NaOH gewaschen, über $Na_2SO_4$ getrocknet und eingedampft. Chromatographie der erhaltenen Mischung an Silikagel mit $CHCl_3$ als Elutionsmittel ergibt den {[7-Chlor-3-(2-chlorphenyl)-1,2-benzisoxazol-6-yl]oxy}-essigsäureäthylester.

Eine Lösung von 5,86 g des Esters in 200 ml heißem Äthanol wird mit 6 ml 50%iger Natronlauge versetzt. Es bildet sich ein Niederschlag, und die Suspension wird eine Stunde unter Rückfluß erhitzt. Nach Zugabe von 200 ml Wasser wird die Mischung mit konzentrierter Salzsäure angesäuert und 18 Stunden bei Raumtemperatur gerührt. Die ausgefallene feste Substanz wird abfiltriert und aus Toluol umkristallisiert. Die erhaltene {[7-Chlor-3-(2-chlorphenyl)-1,2-benzisoxazol-6-yl]oxy}-essigsäure schmilzt bei 165–168°C.

Analyse:

Berechnet für $C_{15}H_9Cl_2NO_4$:
53,28% C; 2,68% H; 4,14% N.
Gefunden:
53,50% C; 2,67% H; 3,95% N.

Beispiel 26

a) Die Friedel-Crafts-Reaktion von Beispiel 24a wird mit 35,4 g 2,3-Dichloranisol, 32,4 g o-Toluoylchlorid und 28 g $AlCl_3$ in 125 ml 1,2-Dichloräthan wiederholt. Man erhält das 2,3-Dichlor-4-methoxy-2'-methylbenzophenon.

b) Das Verfahren von Beispiel 24b wird mit 38 g 2,3-Dichlor-4-methoxy-2'-methylbenzophenon und 34,6 g $AlCl_3$ in 300 ml Benzol wiederholt. Das erhaltene Produkt wird aus Toluol umkristallisiert, wobei man das 2,3-Dichlor-4-hydroxy-2'-methylbenzophenon erhält.

c) Eine Lösung von 28 g 2,3-Dichlor-4-hydroxy-2'-methylbenzophenon in 250 ml Pyridin wird mit 13,9 g Hydroxylaminhydrochlorid versetzt und die Mischung 48 Stunden unter Rückfluß gekocht. Das Pyridin wird im Vakuum verdampft und der Rückstand zwischen Essigester und 5%iger HCl verteilt. Der Essigesterextrakt wird mit Wasser gewaschen, über $Na_2SO_4$ getrocknet und eingedampft, wobei man das entsprechende Oxim enthält. Eine Lösung von 12 g des Oxims in 50 ml DMF und 50 ml Toluol wird mit 2,43 g NaH versetzt, die Mischung wird unter Stickstoff 5¼ Stunde auf eine Innentemperatur von 95–98°C erhitzt. Nach Zugabe von weiteren 50 ml DMF wird die Innentemperatur noch 2 Stunden auf 95°C gehalten. Die Reaktionsmischung wird auf 30°C abgekühlt, und 7,5 g Äthylbromacetat werden zugetropft. Nach beendeter Zugabe wird die Mischung 1 Stunde gerührt. Wasser wird dann zugegeben, das Reaktionsprodukt wird mit Äthylacetat extrahiert, über $Na_2SO_4$ getrocknet und eingedampft. Man erhält den {[7-Chlor-3-(2-tolyl)-1,2-benzisoxazol-6-yl]oxy}-essigsäureäthylester.

Eine Lösung von 13,95 g des Esters in 500 ml heißem Äthanol wird mit 13 ml 50%ige NaOH versetzt. Es bildet sich ein Niederschlag. Die erhaltene Suspension wird 1½ Stunden unter Rückfluß gekocht. 500 ml Wasser werden zugegeben, und die Mischung wird mit konzentrierter HCl angesäuert. Beim Abkühlen fällt ein Niederschlag aus, der abfiltriert und aus Toluol umkristallisiert wird. Die erhaltene {[7-Chlor-3-(2-tolyl)-1,2-benzisoxazol-6-yl]oxy}-essigsäure schmilzt bei 179 bis 181°C.

Analyse:

Berechnet für $C_{16}H_{12}ClNO_4$:
60,48% C; 3,81% H; 4,41% N.
Gefunden:
60,76% C; 3,91% H; 4,26% N.

Beispiel 27

a) Die Friedel-Crafts-Reaktion von Beispiel 24a wird mit 33,7 g 2,3-Dimethylbenzoylchlorid, 54 g 2,3-Dichloranisol und 26,7 g $AlCl_3$ wiederholt, wobei man das 2,3-Dichlor-4-methoxy-2',3'-dimethylbenzophenon erhält.

b) Das Verfahren von Beispiel 24b wird mit 39 g 2,3-Dichlor-4-methoxy-2',3'-dimethylbenzophenon in 300 ml Benzol wiederholt, und das Reaktionsprodukt wird aus Toluol umkristallisiert. Man erhält das 2,3-Dichlor-4-hydroxy-2',3'-dimethylbenzophenon.

c) Eine Mischung von 31 g 2,3-Dichlor-4-hydroxy-2',3'-dimethylbenzophenon und 30,5 g Hydroxylaminhydrochlorid in 250 ml Pyridin wird etwa 1 Woche unter Rückfluß gekocht. Das Pyridin wird im Vakuum verdampft und der Rückstand zwischen Äthylacetat und 5%iger HCl verteilt. Der Äthylacetatextrakt wird gewaschen, über $Na_2SO_4$ getrocknet und eingedampft. Reiben mit Hexan gibt das 2,3-Dichlor-4-hydroxy-2',3'-dimethylbenzophenon-oxim.

d) Eine Lösung von 5 g 2,3-Dichlor-4-hydroxy-2',3'-dimethylbenzophenon-oxim in 70 ml DMF wird mit 0,96 g NaH versetzt. Die Innentemperatur wird 7 Stunden auf 95–120°C gehalten, und die Mischung wird dann 1½ Stunden auf 130°C erhitzt. Die Reaktionsmischung wird abgekühlt, 2,94 g Äthylbromacetat werden zugetropft, die Mischung wird 1 Stunde gerührt, und Wasser wird zugetropft. Das gebildete Reaktionsprodukt wird abfiltriert und aus 95%igem Äthanol umkristallisiert. Der erhaltene {[7-Chlor-3-(2,3-dimethylphenyl)-1,2-benzisoxazol-6-yl]oxy}-essigsäureäthylester schmilzt bei 89–91°C.

Analyse:

Berechnet für $C_{19}H_{18}ClNO_4$:
63,42% C; 5,04% H; 3,89% N.
Gefunden:
63,25% C; 5,02% H; 3,79% N.

e) Eine Suspension von 2 g {[7-Chlor-3-(2,3-dimethylphenyl)-1,2-benzisoxazol-6-yl]oxy}-essigsäureäthylester, 50 ml Äthanol und 5 ml 50%ige NaOH wird 1 Stunde unter Rückfluß gekocht. Wasser wird zu der heißen Lösung gegeben, und die Lösung wird mit konzentrierter HCl angesäuert. Nach Kühlen und Zugabe von Wasser fällt die {[7-Chlor-3-(2,3-dimethylphenyl)-1,2-benzisoxazol-6-yl]oxy}-essigsäure aus. Sie schmilzt bei 170–172°C.

Analyse:

Berechnet für $C_{17}H_{14}ClNO_4$:
61,54% C; 4,25% H; 4,22% N.
Gefunden:
61,65% C; 4,38% H; 4,01% N.

Beispiel 28

a) 29,3 g 2-Brompyridin in 150 ml trockenem THF werden zu 75 ml 2,6 M n-Butyllithium gekühlt auf −65°C gegeben. Dann werden 38,0 g 2,3-Dichlor-4-methoxybenzaldehyd in 300 ml THF hinzugefügt, und man läßt die Reaktionsmischung auf Raumtemperatur kommen. Sie wird dann in Wasser gegossen, und das kristalline Produkt wird abfiltriert, mit Äther gewaschen und getrocknet zu *a*-(2,3-Dichlor-4-methoxyphenyl)-2-pyridinmethanol, Fp. 174–176°C.

b) *a*-(2,3)-dichlor-4-methoxyphenyl)-2-pyridinmethanol (31,86 g) wird in 600 ml Essigsäure und 100 ml Wasser gelöst. Chromsäureanhydrid (11,0 g) wird portionsweise während 5 Minuten zugegeben. Nach 3 Stunden wird die Reaktionsmischung in Wasser gegossen und mit Äther extrahiert. Die vereinigte organische Phase wird gründlich mit 10%iger $NaHCO_3$ gewaschen, dann mit Sole und getrocknet. Nach Entfernung des Lösungsmittels im Vakuum ergibt sich ein kristallines Produkt von (2,3-Dichlor-4-methoxyphenyl)-(2-pyridyl)methanon, Fp. 104–107°C.

Analyse:

Berechnet für $C_{13}H_9Cl_2NO_2$:
55,34% C; 3,21% H; 4,97% N.
Gefunden:
55,29% C; 3,26% H; 4,93% N.

c) (2,3-Dichlor-4-methoxyphenyl)-(2-pyridyl)methanon (32,0 g) wird 4 Stunden in 300 ml Äthanol, das 30 g Hydroxylaminhydrochlorid enthält, unter Rückfluß gekocht. Die Reaktionsmischung wird in Wasser gegossen, mit $NH_4OH$ basisch gestellt und mit Äthylacetat extrahiert. Nach Trocknung und Verdampfung wird eine Rohoximmischung von E-(2-Pyridyl)(2,3-dichlor-4-methoxyphenyl)-methanonoxim und Z-(2-Pyridyl)(2,3-dichlor-4-methoxyphenyl)methanonoxim erhalten.
Die Oximmischung wird in 200 ml DMF gelöst und zu einer Suspension von 3,1 g NaH in 150 ml DMF gegeben. Nach 15 Minuten wird die Reaktionsmischung in Wasser gegossen und das Produkt

abfiltriert. Einmalige Umkristallisierung aus Isopropanol trennt nicht umgesetztes E-Oxim ab und ergibt 7-Chlor-6-methoxy-3-(2-pyridyl)-1,2-benzisoxazol, Fp. 164–166°C.

Analyse:

Berechnet für $C_{13}H_9ClN_2O_2$:
59,89% C; 3,48% H; 10,75% N.
Gefunden:
59,53% C; 3,37% H; 10,63% N.

d) 7-Chlor-6-methoxy-3-(2-pyridyl)-1,2-benzisoxazol (24,4 g) werden 1 Stunde lang in 450 ml 48%iger HBr am Rückfluß gekocht. Das ausgefällte Hydrobromid-Salz wird abfiltriert, mit Äther gewaschen und mit 10%iger NaH-$CO_3$-Lösung neutralisiert. Die freie Base wird abfiltriert und getrocknet, wobei 7-Chlor-6-hydroxy-3-(2-pyridyl)-1,2-benzisoxazol, Fp. 209–211°C, erhalten wird.

Analyse:

Berechnet für $C_{12}H_7ClN_2O_2$:
58,43% C; 2,85% H; 11,36% N.
Gefunden:
58,16% C; 2,81% H; 11,42% N.

e) 7-Chlor-6-hydroxy-3-(2-pyridyl)-1,2-benzisoxazol (10,0 g) wird in 80 ml DMF gelöst und zu einer eiskalten Suspension von NaH (1,1 g) in 50 ml DMF gegeben. Nach Beendigung der Wasserstoffentwicklung wird Bromessigsäureäthylester (7,5 g) in 20 ml DMF zugegeben. Nach weiteren 90 Minuten werden 200 ml Wasser und 10 ml 50%ige NaOH zugegeben und die Reaktionsmischung für 45 Minuten auf 65°C erwärmt. Dann wird die Mischung in Wasser gegossen, auf pH 1–2 angesäuert, ein festes Produkt wird abfiltriert, getrocknet und ergibt dann [7-Chlor-3-(2-pyridyl)-1,2-benzisoxazole-6-yl]oxy-essigsäure, Fp. 255–256°C.

Analyse:

Berechnet für $C_{14}H_9ClN_2O_4$:
55,18% C; 2,48% H; 9,20% N.
Gefunden:
55,35% C; 2,88% H; 9,45% N.

## Beispiel 29

a) 7-Chlor-6-hydroxy-3-(2-pyridyl)-1,2-benzisoxazol (Beispiel 28d) (9,9 g) wird bei 60°C in 600 ml Eisessig gelöst, und dann wird m-Chlorperbenzoesäure (8,3 g, 85%ig) portionsweise zugegeben. Nach Erwärmung auf 60°C für insgesamt 14 Stunden wird die Reaktionsmischung in 2 l Wasser gegossen, das Produkt wird abfiltriert und erst mit Methanol, dann mit Äther gewaschen. Auf diese Weise wird 7-Chlor-6-hydroxy-3-(2-pyridyl)-1,2-benzisoxazol-1'-oxid nach Umkristallisieren aus DMF/Wasser in der Form eines Hemihydrats vom Schmelzpunkt 214°C erhalten.

Analyse:

Berechnet für $C_{12}H_7ClN_2O_3 \cdot O \cdot 5\ H_2O$:
53,05% C; 2,97% H; 10,31% N.
Gefunden:
53,14% C; 2,82% H; 10,47% N.

b) 7-Chlor-6-hydroxy-3-(2-pyridyl)-1,2-benzisoxazol-1'-oxid (7,30 g) wird in 200 ml DMF gelöst und zu einer Suspension von NaH (1,33 g) in 50 ml DMF gegeben. Nach 15 Minuten wird Bromessigsäureäthylester (5,0 g) in 20 ml DMF zugegeben und die Reaktionsmischung für 10 Stunden auf 50°C erwärmt. Anschließend wird die Mischung mit Wasser abgeschreckt, angesäuert, der Niederschlag abfiltriert und gut getrocknet. Der Niederschlag wird erneut mit 1,33 g NaH und 5,0 g Bromessigsäureäthylester in DMF behandelt. Nach 30 Minuten werden 200 ml Wasser und 10 ml 50%ige NaOH zugegeben, und die Reaktionsmischung wird für 30 Minuten auf 50°C erwärmt und dann angesäuert.
{[7-Chlor-3-(2-pyridyl)-1,2-benzisoxazol-6-yl]oxy}-essigsäure-1'-oxyd wird erhalten, Fp. 214°C (Z.).

Analyse:

Berechnet für $C_{14}H_9ClN_2O_5$:
52,43% C; 2,83% H; 8,74% N.
Gefunden:
52,35% C; 2,79% H; 8,93% N.

Beispiel 30

a) m-Dimethoxybenzol (27,6 g) und o-Fluorbenzoylchlorid (31,7 g) wird in 200 ml Dichloräthan gelöst, und $AlCl_3$ (28,0 g) wird portionsweise zugegeben. Nach 2 Stunden werden weitere 56 g $AlCl_3$ zugegeben, und die Reaktionsmischung wird für 1,5 Stunden auf 60°C erwärmt. Dann wird sie in Wasser gegossen und mit Essigsäureäthylester extrahiert. Trocknung und Verdampfung ergeben ein kristallines Produkt. Nach Anreiben mit Toluol wird 2,4-Dihydroxy-2'-fluorbenzophenon vom Schmp. 109–111°C erhalten.

Analyse:

Berechnet für $C_{13}H_9FO_3$:
67,24% C; 3,91% H; 8,18% F.
Gefunden:
66,85% C; 3,75% H; 8,35% F.

b) 2,4-Dihydroxy-2'-fluorbenzophenon (25,0 g) wird 18 Stunden lang in 250 ml Pyridin, das 17,1 g Hydroxylamin-Hydrochlorid enthält, am Rückfluß gekocht. Die Reaktionsmischung wird dann zwischen Äther und 5%iger HCl verteilt, und die organische Phase wird abgetrennt. Nach Kristallisieren aus Toluol, Trocknung und Verdampfung wird ein reines Isomeres von 2,4-Dihydroxy-2'-fluorbenzophenon-[E-oxim], Fp. 170–172°C, erhalten.

Analyse:

Berechnet für $C_{13}H_{10}FNO_3$:
63,16% C; 4,08% H; 5,67% N.
Gefunden:
63,56% C; 4,28% H; 5,56% N.

c) Das E-Oxim 2,4-Dihydro-2'-fluorbenzophenon-oxim (18,6 g) wird in 18,0 ml Essigsäureanhydrid für 5 Stunden auf 50°C erwärmt, und dann für 6 Stunden auf 60°C. Weitere 3,0 ml Essigsäureanhydrid werden zugegeben und die Reaktionsmischung dann 73 Stunden bei Raumtemperatur stehengelassen. Das kristalline Produkt, das sich von der Reaktionsmischung abtrennt, wird mit kaltem Äther gewaschen, und man erhält reines E-4-Acetoxy-2-hydroxy-2'-fluorbenzophenon-O-acetyl-oxim, Fp. 132–134°C.

Analyse:

Berechnet für $C_{17}H_{14}FNO_5$:
61,63% C; 4,26% H; 4,23% N.
Gefunden:
61,80% C; 4,08% H; 4,07% N.

d) E-4-Acetoxy-2-hydroxy-2'-fluorbenzophenon-O-acetyloxim (18,4 g) wird in 80 ml DMF gelöst und zu einer eiskalten Suspension von NaH (3,3 g) in 100 ml DMF gegeben. Nach 90 Minuten wird die Reaktionsmischung in Wasser gegossen, und ein geringer unlöslicher Niederschlag wird abfiltriert. Nach Ansäuern des wäßrigen Filtrats und Extraktion mit Äther, Trocknung und Verdampfung wird 3-(2-Fluorphenyl)-6-hydroxy-1,2-benzisoxazol, Fp. 206–210°C, erhalten.

Analyse:

Berechnet für $C_{13}H_8FNO_2$:
68,12% C; 3,52% H; 6,11% N.
Gefunden:
68,43% C; 3,59% H; 6,14% N.

e) 3-(2-Fluorphenyl)-6-hydroxy-1,2-benzisoxazol (9,7 g) wird in 80 ml DMF gelöst und zu einer eisgekühlten Suspension von NaH (1,4 g) in 50 ml DMF gegeben. Nach Beendigung der Wasserstoffentwicklung wird Bromessigsäureäthylester (7,5 g) in 20 ml DMF zugegeben, und man läßt die Reaktionsmischung auf Raumtemperatur kommen. Nach 2 Stunden werden 200 ml Wasser und 10 ml 50%ige NaOH zugegeben, und die Reaktionsmischung wird auf 50°C erwärmt. Nach weiteren 30 Minuten und nach Ansäuern und Filtration wird ein Produkt gesammelt, dessen Umkristallisation aus Toluol/$CH_3CN$ {[3-(2-Fluorphenyl)-1,2-benzisoxazol-6-yl]oxy}-essigsäure, Fp. 182 bis 184°C, ergibt.

Analyse:

Berechnet für $C_{15}H_{10}FNO_4$:
62,72% C; 3,51% H; 4,88% N.
Gefunden:
62,56% C; 3,61% H; 5,06% N.

Beispiel 31

a) Das Verfahren von Beispiel 24a wird mit 31,55 g p-Fluorbenzoylchlorid, 32 g 2,3-Dichloranisol und 35,22 g $AlCl_3$, alles zusammen in 1,2-Dichloräthan, wiederholt. Das entstandene Rohprodukt wird mit warmem Hexan verrieben, gekühlt und filtriert, und ergibt 2,3-Dichlor-4-methoxy-4'-fluorbenzophenon.

b) Eine Mischung aus 39,5 g 2,3-Dichlor-4-methoxy-4'-fluorbenzophenon und 160 g Pyridin-Hydrochlorid wird 1 Stunde lang auf 200°C erhitzt. Unter Rühren wird die Reaktionsmischung in Eiswasser gegossen, so daß sich ein Niederschlag bildet, der abfiltriert und etwa 18 Stunden lang getrocknet wird. 2,3-Dichlor-4-hydroxy-4'-fluorbenzophenon wird erhalten.

c) Zu einer Lösung von 35 g 2,3-Dichlor-4-hydroxy-4'-fluorbenzophenon in 250 ml Pyridin werden 34,6 g Hydroxylamin-HCl zugegeben. Die Mischung wird 4 Stunden unter Rückfluß gekocht, das Pyridin wird im Vakuum verdampft und der Rückstand verteilt zwischen 5%iger HCl und Essigester. Der Essigester-Extrakt wird mit Wasser gewaschen, über $Na_2SO_4$ getrocknet und eingedampft. 2,3-Dichlor-4-hydroxy-4'-fluorbenzophenon-oxim werden als Isomerenmischung erhalten. Fp. 150–156°C.

Analyse:

Berechnet für $C_{13}H_8Cl_2FNO_2$:
52,02% C; 2,69% H; 4,67% N.
Gefunden:
52,19% C; 2,74% H; 4,69% N.

d) Eine Lösung von 20 g 2,3-Dichlor-4-hydroxy-4'-fluorbenzophenonoxim in 100 ml DMF werden in einer $N_2$-Atmosphäre zu einer Mischung von 4,0 g NaH in 50 ml DMF getropft. Die Reaktionsmischung wird auf eine Innentemperatur von 96°C erhitzt und 2 Stunden auf dieser Temperatur gehalten. Dann wird sie auf 40°C gekühlt, 12,3 g Bromessigsäureäthylester werden zugetropft, und die Mischung wird 1 Stunde lang gerührt. 20 ml 50%ige NaOH und 100 ml Wasser werden zugegeben, und der Ansatz wird für 1 Stunde auf 80–90°C erhitzt. Konz. HCl wird so lange zugegeben, bis die Reaktionsmischung sauer ist, dann wird sie ½ Stunde lang gerührt und schließlich Wasser zugegeben. Ein festes Produkt wird durch Filtration gesammelt und umkristallisiert. Reine {[7-Chlor-3-(4-fluorphenyl)-1,2-benzisoxazol-6-yl]oxy}-essigsäure, Fp. 233–237°C, wird erhalten.

Analyse:

Berechnet für $C_{15}H_9ClFNO_4$:
56,00% C; 2,83% H; 4,36% N.
Gefunden:
55,76% C; 2,90% H; 4,29% N.

Beispiel 32

a) 2,6-Dimethoxytoluol (20,0 g) und o-Fluorbenzoylchlorid (19,8 g) wird in 250 ml Dichloräthan gelöst und auf 5°C gekühlt. $AlCl_3$ wird portionsweise zugegeben, und nach Beendigung der Zugabe läßt man die Reaktionsmischung für 30 Minuten auf Raumtemperatur kommen, dann kocht man sie für 30 Minuten unter Rückfluß. Anschließend wird in 5%ige HCl gegossen und 18 Stunden lang

stehengelassen. Extraktion mit Ather, anschließende Trocknung und Einengung ergeben ein kristallines Material, das nach Waschen mit Hexan 2'-Fluor-2-hydroxy-4-methoxy-3-methylbenzophenon, Fp. 118–120°C, ergibt.

Analyse:

Berechnet für $C_{15}H_{13}FO_3$:
69,22% C; 5,04% H; 7,30% F.
Gefunden:
69,23% C; 5,01% H; 6,98% F.

b) 2'-Fluor-2-hydroxy-4-methoxy-3-methylbenzophenon (30,0 g) wird 18 Stunden lang in 350 ml Pyridin, das 32,0 g Hydroxylamin-Hydrochlorid enthält, am Rückfluß gekocht. Das Lösungsmittel wird im Vakuum entfernt, und der Rückstand wird zwischen Äther und 5%iger HCl verteilt. Trocknung und Einengung des Äthers ergeben ein Rohprodukt, das als Schmelze für 30 Minuten auf 200°C in einer Wasserstoff-Atomosphäre erhitzt wird. Die Schmelze läßt man dann abkühlen, und die feste Masse wird gut mit Hexan angerieben. E-2'-Fluor-2-hydroxy-4-methoxy-3-methylbenzophenon-oxim, Fp. 167–169°C, wird erhalten.

Analyse:

Berechnet für $C_{15}H_{14}FNO_3$:
65,44% C; 5,13% H; 5,09% N.
Gefunden:
65,49% C; 5,26% H; 4,83% N.

c) E-2'-Fluor-2-hydroxy-4-methoxy-3-methylbenzophenon-oxim (20,0 g) wird 1 Stunde lang mit 12 ml Essigsäureanhydrid im Dampfbad erwärmt. Das Essigsäureanhydrid wird im Vakuum verdampft, und das Produkt wird zwischen Äther und Wasser verteilt; der Äther wird anschließend mit 10%iger $NaHCO_3$ gewaschen. Verdampfung und Einengen des kristallinen Produkts mit Hexan ergeben E-2'-Fluor-2-hydroxy-4-methoxy-3-methylbenzophenon-O-acetyloxim, Fp. 83–86°C.

Analyse:

Berechnet für $C_{12}H_{16}FNO_4$:
64,34% C; 5,08% H; 4,42% N.
Gefunden:
64,30% C; 5,08% H; 4,26% N.

d) E-2'-Fluor-2-hydroxy-4-methoxy-3-methylbenzophenon-O-acetyloxim (22,0 g) wird in 100 ml DMF gelöst und zu einer Suspension von 2,5 g NaH in 100 ml DMF gegeben. Mittels eines Eisbades wird die Reaktionstemperatur unter 30°C gehalten. Nach 40 Minuten wird die Reaktionsmischung in Wasser gegossen und mit Äther extrahiert. Nach gründlichem Waschen mit Wasser wird der Äther getrocknet und verdampft. Ein kristallines Produkt entsteht, das mit kaltem Hexan gewaschen wird. 3-(2-Fluorphenyl)-6-methoxy-7-methyl-1,2-benzisoxazol, Fp. 105–108°C, wird erhalten.

Analyse:

Berechnet für $C_{15}H_{12}FNO_2$:
70,03% C; 4,70% H; 5,45% N.
Gefunden:
69,96% C; 4,76% H; 3,36% N.

e) 3-(2-fluorphenyl)-6-methoxy-7-methyl-1,2-benzisoxazol (16,1 g) wird 2 Stunden lang mit 64 g Pyridin-Hydrochlorid auf 200°C erhitzt. Die Schmelze wird in Wasser gegossen und mit Essigester extrahiert. Nach Waschen mit 5%iger HCl wird der Essigester getrocknet und eingedampft. Man erhält 3-(2-Fluorphenyl)-6-hydroxy-7-methyl-1,2-benzisoxazol, Fp. 216–219°C.

Analyse:

Berechnet für $C_{14}H_{10}FNO_2$:
69,13% C; 4,14% H; 5,76% N.
Gefunden:
68,91% C; 4,03% H; 5,82% N.

f) 3-(2-Fluorphenyl)-6-hydroxy-7-methyl-1,2-benzisoxazol (10,0 g) wird in 90 ml DMF gelöst und mit 8,0 g Äthylbromacetat und 6,7 g $K_2CO_3$ behandelt. Die Reaktionsmischung wird 2 Stunden lang auf 60°C erwärmt, und man läßt sie dann auf Raumtemperatur abkühlen. Nach 18 Stunden bei Raumtemperatur werden Wasser (200 ml) und 50%ige NaOH (15 ml) zugegeben, und die Lösung wird 90 Minuten lang auf 90°C erhitzt. Dann wird die Mischung in Wasser gegossen, angesäuert und mit Äther extrahiert. Nach Trocknung und Verdampfung ergibt sich ein kristallines Produkt {[3-(2-Fluorphenyl)-7-methyl-1,2-benzisoxazol-6-yl]oxy}-essigsäure, Fp. 158–160°C.

Analyse:

Berechnet für $C_{16}H_{12}FNO_4$:
63,78% C; 4,02% H; 4,65% N.
Gefunden:
63,89% C; 4,06% H; 4,58% N.

Beispiel 33

a) m-Chloranisol (28,5 g) und o-Fluorbenzoylchlorid (31,7 g) werden in 200 ml Dichloräthan gelöst und bei 10°C mit 26,7 g $AlCl_3$ behandelt. Nach 45 Minuten wird die Reaktionsmischung auf Eis gegossen und mit Äther extrahiert. Nach Trocknung und Verdampfung und anschließendem Anreiben mit Hexan wird ein Material erhalten, das 2-Chlor-2'-fluor-4-methoxybenzophenon enthält. Umkristallisieren aus $Et_2O$/Hexan ergibt 2-Chlor-2'-fluor-4-methoxybenzophenon, Fp. 77–79°C.

Analyse:

Berechnet für $C_{14}H_{10}ClFO_2$:
63,53% C; 3,81% H; 7,18% F.
Gefunden:
63,77% C; 3,75% H; 7,25% F.

b) 2-Chlor-2'-Fluor-4-methoxybenzophenon (15,5 g) wird 3 Stunden in 150 ml Pyridin, das 10,0 g Hydroxylamin-Hydrochlorid enthält, unter Rückfluß gekocht. Das Pyridin wird im Vakuum entfernt, und der Rückstand wird zwischen Äther und 5%iger HCl verteilt. Trocknung und Eindampfen der organischen Phase ergeben eine isomere Oximmischung. Die Mischung wird in 50 ml DMF gelöst und zu einer Suspension von 1,5 g NaH in 30 ml DMF gegeben. Nach Erwärmung auf 60°C für 30 Minuten wird die Reaktionsmischung in Wasser gegossen und mit Äther extrahiert. Einengen im Vakuum ergibt einen Feststoff, aus dem nach Umkristallisieren in Äther 3-(2-Fluorphenyl)-6-methoxy-1,2-benzisoxazol, Fp. 101–103°C, entsteht.

Analyse:

Berechnet für $C_{14}H_{10}FNO_2$:
69,13% C; 4,14% H; 5,76% N.
Gefunden:
69,08% C; 4,29% H; 5,65% N.

c) 10 g 3-(2-Fluorphenyl)-6-methoxy-1,2-benzisoxazol werden in 400 ml trockenem THF gelöst (bei −40°C) und mit 21 ml 2,2 M n-Butyllithium behandelt. Nach einstündigem Rühren wird Jod (11,7 g) in 90 ml Äther zugegeben. Die Reaktionsmischung wird in $Na_2S_2O_3$ gegossen, anschließend in $H_2O$. Trocknung und Einengen ergeben 3-(2-Fluorphenyl)-7-iod-6-methoxy-1,2-benzisoxazol, Fp. 135–138°C.

Analyse:

Berechnet für $C_{14}H_9FINO_2$:
45,55% C; 2,46% H; 3,80% N; 34,38% I.
Gefunden:
44,94% C; 2,43% H; 3,70% N; 34,09% I.

d) 3-(2-Fluorphenyl)-7-iodo-6-methoxy-1,2-benzisoxazol (8,2 g) wird 18 Stunden in 130 ml $CH_2Cl_2$, das 6,6 ml $BBr_3$ enthält, unter Rückfluß gekocht. Die Reaktionsmischung wird dann in Wasser gegossen und mit Äther extrahiert. Trocknung und Eindampfung ergeben ein kristallines Produkt, das gut mit Hexan verrieben wird. 3-(2-Fluorphenyl)-6-hydroxy-7-iod-1,2-benzisoxazol, Fp. 212 bis 214°C, wird erhalten.

Analyse:

Berechnet für $C_{13}H_7FINO_2$:
43,97% C; 1,99% H; 3,95% N; 35,74% I.
Gefunden:
44,19% C; 2,00% H; 3,86% N; 35,12% I.

e) 3-(2-Fluorphenyl)-6-hydroxy-7-iod-1,2-benzisoxazol (7,80 g) in 80 ml DMF wird bei 60°C mit 6,6 g $K_2CO_3$ und 7,9 g Äthylbromacetat behandelt. Nach einer Stunde wird die Temperatur auf 90°C erhöht, und 80 ml Wasser und 8 ml 50%ige NaOH werden zugegeben. Nach weiteren 30 Minuten wird die Mischung in Wasser gegossen und angesäuert, anschließend mit Äther extrahiert, getrocknet und eingedampft. Ergebnis: {[3-(2-Fluorphenyl)-7-iod-1,2-benzisoxazol-6-yl]-oxy}-essigsäure, Fp. 178–180°C.

Analyse:

Berechnet für $C_{15}H_9FINO_4$:
43,61% C; 2,20% H; 3,39% N.
Gefunden:
43,25% C; 2,12% H; 3,28% N.

## Beispiel 34

a) 10 g 3-(2-Fluorphenyl)-6-methoxy-1,2-benzisoxazol aus Beispiel 33b werden in 400 ml trockenem THF gelöst und bei −40°C mit 21 ml 2,2 M n-Butyllithium behandelt. Nach einstündigem Rühren wird Brom (2,5 ml) zugetropft. Die Reaktionsmischung wird in Wasser gegossen, mit Äther extrahiert und mit $Na_2S_2O_3$-Lösung gewaschen. Trocknung und Eindampfen ergeben ein Material, das das gewünschte bromierte Produkt enthält, d. h. 7-Brom-3-(2-fluorphenyl)-6-methoxy-1,2-benzisoxazol, Fp. 150–153°C.

Analyse:

Berechnet für $C_{14}H_9BrFNO_2$:
52,19% C; 2,82% H; 4,35% N; 24,81% Br.
Gefunden:
51,97% C; 2,83% H; 4,28% N; 25,17% Br.

b) 7-Brom-3-(2-fluorphenyl)-6-methoxy-1,2-benzisoxazol (7,20 g) wird 18 Stunden in 130 ml $CH_2Cl_2$, das 6,6 ml $BBr_3$ enthält, unter Rückfluß gekocht. Danach wird die Reaktionsmischung in Wasser gegossen und mit Essigester extrahiert. Eindampfung und Verreiben mit Hexan ergeben das entsprechende Phenol, Fp. 231–234°C.
Das Phenol (6,30 g) in 80 ml DMF wird bei 60°C mit 6,6 g $K_2CO_3$ und 7,9 g Bromessigsäureäthylester behandelt. Nach einer Stunde werden 80 ml Wasser und 8 ml 50%ige NaOH zugegeben, und die Temperatur wird auf 90°C erhöht. Nach weiteren 45 Minuten wird die Reaktionsmischung angesäuert und mit Essigester extrahiert. Eindampfen und Umkristallisieren aus Toluol/$CH_3CN$ ergeben {[7-Brom-3-(2-fluorphenyl)-1,2-benzisoxazol-6-yl]-oxy}-essigsäure, Fp. 180–182°C.

Analyse:

Berechnet für $C_{15}H_9BrFNO_4$:
49,20% C; 2,48% H; 3,83% N.
Gefunden:
49,19% C; 2,47% H; 3,88% N.

## Beispiel 35

a) 3,06 g $AlCl_3$ werden portionsweise zu einer Mischung von 4 g 2-Chlor-resorcin-dimethyläther und 3,7 g 2,3-Difluorbenzoylchlorid in 60 ml 1,2-Dichloräthan bei 5°C gegeben. Man läßt die Mischung auf Raumtemperatur kommen und kocht sie dann 30 Minuten lang am Rückfluß. Dann wird die Reaktionsmischung in konz. HCl/Eis gegossen und etwa 72 Stunden stehengelassen. Die wäßrige Schicht wird mit zusätzlichem organischen Lösungsmittel extrahiert, über $Na_2SO_4$ getrocknet und eingedampft. Ergebnis: 3-Chlor-2-hydroxy-4-methoxy-2',3'-difluorbenzophenon, Fp. 161–162°C.

Analyse:

Berechnet für $C_{14}H_9ClF_2O_3$:
56,30% C; 3,04% H; 12,72% F.
Gefunden:
56,26% C; 3,06% H; 12,56% F.

b) Zu einer Lösung von 24 g 3-Chlor-2-hydroxy-4-methoxy-2',3'-difluorbenzophenon in 160 ml Pyridin werden 22 g Hydroxylamin-HCl gegeben. Die Mischung wird 2 Stunden unter Rückfluß gekocht und das Pyridin im Vakuum verdampft. Der Rückstand wird zwischen Essigester und 5%iger HCl verteilt. Der Essigester-Extrakt wird mit Wasser gewaschen, über $Na_2SO_4$ getrocknet und eingedampft, so daß ein hellgelber Feststoff entsteht, der aus 2 Isomeren besteht. Der Feststoff wird während etwa 13 Minuten bei 205°C geschmolzen. Der Rückstand wird in heißem Essigester gelöst und zur Trockne eingedampft. Ergebnis: E-3-Chlor-2',3'-difluor-2-hydroxy-4-methoxybenzophenon-oxim, Fp. 198–199°C.

Analyse:

Berechnet für $C_{14}H_{10}ClF_2NO_3$:
53,60% C; 3,21% H; 4,47% N.
Gefunden:
53,95% C; 3,29% H; 4,42% N.

c) Eine Mischung von 1,5 g E-3-Chlor-2',3'-difluor-2-hydroxy-4-methoxybenzophenon-oxim und 0,67 g Essigsäureanhydrid wird 30 Minuten auf 60°C erwärmt. Die Mischung geht in Lösung und erstarrt dann. Der Rückstand wird zwischen Essigester und 10%iger $NaHCO_3$ verteilt. Der Essigesterextrakt wird gewaschen, über $Na_2SO_4$ getrocknet und eingedampft. Ergebnis: E-3-Chlor-2',3'-difluor-2-hydroxy-4-methoxybenzophenon-O-acetyloxim, Fp. 136–139°C.

Analyse:

Berechnet für $C_{16}H_{12}ClF_2NO_4$:
54,02% C; 3,40% H; 3,94% N.
Gefunden:
53,89% C; 3,48% H; 3,97% N.

d) Zu einer Mischung von 1,4 g NaH in 200 ml DMF wird eine Lösung von 19 g E-3-Chlor-2',3'-difluor-2-hydroxy-4-methoxybenzophenon-O-acetyloxim in 200 ml DMF unter einer $N_2$-Atmosphäre getropft. Die Mischung wird ½ Stunde gerührt und dann ½ Stunde lang auf 45°C erwärmt. Wasser wird zugegeben, worauf ein Produkt ausfällt, das nach Filtration und Trocknung 7-Chlor-3-(2,3-difluorphenyl)-6-methoxy-1,2-benzisoxazol, Fp. 184–189°C, ergibt.

Analyse:

Berechnet für $C_{14}H_8ClF_2NO_2$:
56,87% C; 2,73% H; 4,74% N.
Gefunden:
56,95% C; 2,84% H; 4,77% N.

e) Eine feste Mischung von 12,4 g 7-Chlor-3-(2,3-difluorphenyl)-6-methoxy-1,2-benzisoxazol und 50 g Pyridin-HCl wird 45 Minuten lang bei 200°C erhitzt. Dann wird sie in kräftig gerührtes Eiswasser gegossen, wobei 7-Chlor-6-hydroxy-3-(2,3-difluorphenyl)-1,2-benzisoxazol ausfällt, Fp. 250 bis 254°C.

Analyse:

Berechnet für $C_{13}H_6ClF_2NO_2$:
55,43% C; 2,15% H; 4,97% N.
Gefunden:
55,60% C; 2,25% H; 4,91% N.

f) Zu einer Lösung von 12 g 7-Chlor-6-hydroxy-3-(2,3-difluorphenyl)-1,2-benzisoxazol in 120 ml DMF werden 6,36 g $K_2CO_3$ gegeben und anschließend 7,83 g $BrCH_2CO_2-C_2H_5$. Die Reaktionsmischung wird 2 Stunden auf 60°C erwärmt und dann 18 Stunden stehengelassen. 200 ml Wasser und 15 ml 50%ige NaOH werden zugegeben. Die Mischung wird 90 Minuten lang auf 90°C

erwärmt, in Wasser gegossen und angesäuert. Das Produkt wird mit Essigester extrahiert, über $Na_2SO_4$ getrocknet und eingedampft. Ergebnis: {[7-Chlor-3-(2,3-difluorphenyl)-1,2-benzisoxazol-6-yl]oxy}-essigsäure, Fp. 183–187°C.

Analyse:

Berechnet für $C_{15}H_8ClF_2NO_4$:
53,04% C; 2,37% H; 4,12% N.
Gefunden:
53,20% C; 2,49% H; 3,88% N.

Beispiel 36

a) 3,17 g Hydroxylamin-HCl werden zu einer Lösung von 10 g (0,33 M) 2'-Fluor-4-methoxy-2,3-dichlorbenzophenon aus Beispiel 1a in 100 ml Pyridin gegeben. Die Mischung wird etwa 64 Stunden lang unter Rückfluß gekocht. Das Pyridin wird verdampft und der Rückstand zwischen 5%iger HCl und Essigester verteilt. Der Essigester-Extrakt wird mit Wasser gewaschen, über $Na_2SO_4$ getrocknet und eingedampft und ergibt dann 2,3-Dichlor-4-methoxy-2'-fluorbenzophenon, Fp. 195 bis 197°C.

Analyse:

Berechnet für $C_{14}H_{10}Cl_2FNO_2$:
53,52% C; 3,21% H; 4,46% N.
Gefunden:
53,55% C; 3,10% H; 4,42% N.

b) Unter einer $N_2$-Atmosphäre wird 0,67 g NaH zu einer Lösung von 8 g 2,3-Dichlor-4-methoxy-2'-fluorbenzophenoxim in 50 ml DMF gegeben. Die Mischung wird 1 Stunde gerührt, Wasser wird zugegeben, woraufhin 7-Chlor-3-(2-fluorphenyl)-6-methoxy-1,2-benzisoxazol ausfällt, das abfiltriert und getrocknet wird. Schmp. 155–158°C.

Analyse:

Berechnet für $C_{14}H_9ClFNO_2$:
60,55% C; 3,26% H; 5,05% N.
Gefunden:
60,63% C; 3,15% H; 5,01% N.

c) Eine feste Mischung von 2 g 7-Chlor-3-(2-fluorphenyl)-6-methoxy-1,2-benzisoxazol und 20 g Pyridin-Hydrochlorid wird 1 Stunde lang bei 190–210°C gehalten. Die heiße Reaktionsmischung wird in kräftig gerührtes Eiswasser gegossen und dann leicht angesäuert. Durch Filtrieren und Trocknen wird 7-Chlor-6-hydroxy-3-(2-fluorphenyl)-1,2-benzisoxazol, Fp. 140–141°C, erhalten.

Analyse:

Berechnet für $C_{13}H_7ClFNO_2$:
59,22% C; 2,68% H; 5,31% N.
Gefunden:
59,16% C; 2,65% H; 5,23% N.

d) Das Verfahren von Beispiel 35f kann wiederholt werden mit 7-Chlor-6-hydroxy-3-(2-fluorphenyl)-1,2-benzisoxazol. Ergebnis: {[7-Chlor-3-(2-fluorphenyl)-1,2-benzisoxazol-6-yl]oxy}-essigsäureäthylester, das Produkt von Beispiel 1d.

Beispiel 37

a) 2-Chlor-resorcin-dimethyläther (22,0 g) und o-Fluorbenzoylchlorid (20,2 g) werden in 250 ml Dichloräthan gelöst, in einem Eisbad gekühlt und mit $AlCl_3$ (18,6 g) behandelt. 15 Minuten nach Beendigung der Zugabe wird die Reaktionsmischung unter Rückfluß 30 Minuten lang gekocht. Sie wird dann in Wasser gegossen und mit Essigester extrahiert. Verdampfung und Verreiben mit Hexan ergeben: 3-Chlor-2'-fluor-2-hydroxy-4-methoxybenzophenon, Fp. 132–133°C.

Analyse:

Berechnet für $C_{14}H_{10}ClFO_3$:
59,90% C; 3,59% H; 12,63% Cl.
Gefunden:
59,77% C; 3,59% H; 12,51% Cl.

b) 3-Chlor-2'-fluor-2-hydroxy-4-methoxybenzophenon (24,6 g) wird in 300 ml Pyridin, das 12,2 g Hydroxylamin-Hydrochlorid enthält, 18 Stunden lang unter Rückfluß gekocht. Die Reaktionsmischung wird im Vakuum eingeengt und zwischen Äther und 5%iger HCl verteilt. Die organische Phase wird getrocknet und eingedampft, und das resultierende kristalline Produkt 45 Minuten lang bei 205°C geschmolzen, anschließend abgekühlt und aus Toluol umkristallisiert. Ergebnis: E-3-Chlor-2'-fluor-2-hydroxy-4-methoxybenzophenonoxim, Fp. 184–186°C.

Analyse:

Berechnet für $C_{14}H_{11}ClFNO_3$:
56,86% C; 3,75% H; 4,79% N.
Gefunden:
56,67% C; 3,68% H; 4,66% N.

c) E-3-Chlor-2'-fluor-2-hydroxy-4-methoxybenzophenonoxim (18,1 g) wird 30 Minuten lang mit 9 ml Essigsäureanhydrid auf 60°C erwärmt, und die Reaktionsmischung wird anschließend zwischen Äther und 10%iger $NaHCO_3$ verteilt sowie mit 10%iger $NaHCO_3$ gewaschen, bis das Gewaschene basisch bleibt. Trocknung, Eindampfung und Verreiben mit Hexan ergeben E-3-Chlor-2'-fluor-2-hydroxy-4-methoxybenzophenon-O-acetyloxim, Fp. 125–128°C.

Analyse:

Berechnet für $C_{16}H_{13}ClFNO_4$:
56,90% C; 3,88% H; 4,15% N.
Gefunden:
56,79% C; 3,85% H; 4,20% N.

d) Das Verfahren von Beispiel 35d wird wiederholt. Ergebnis: 7-Chlor-3-(2-fluorphenyl)-6-methoxy-1,2-benzisoxazol. Danach können die Verfahrensweisen der Beispiele 36c und 36d benutzt werden, um {[7-Chlor-3-(2-fluorphenyl)-1,2-benzisoxazol-6-yl]oxy}-essigsäureäthylester zu erhalten.

## Beispiel 38

a) 5,0 g 7-Chlor-6-hydroxy-3-(2-fluorphenyl)-1,2-benzisoxazol des Beispiels 36c in 30 ml DMF werden unter Rühren zu 1,1 g NaH in 30 ml DMF getropft. Nach einer Stunde werden 2,6 ml 2-Brompropionsäureäthylester zugegeben, und die Lösung wird 1,5 Stunden gerührt. Danach wird sie auf Eis gegossen, wobei 2-{[7-Chlor-3-(2-fluorphenyl)-1,2-benzisoxazol-6-yl]oxy}-propionsäureäthylester ausfällt, das filtriert und getrocknet wird. Fp. 79°C.

Analyse:

Berechnet für $C_{18}H_{15}ClFNO_4$:
59,50% C; 4,13% H; 3,85% N; 9,60% Cl.
Gefunden:
59,48% C; 4,09% H; 4,00% N; 9,48% Cl.

b) 6,8 g 2-{[7-Chlor-3-(2-fluorphenyl)-1,2-benzisoxazol-6-yl]oxy}-propionsäureäthylester wird in 35 ml Methanol gelöst, und 25 ml einer 15%igen NaOH-Lösung werden zugegeben. Die Suspension wird 2 Stunden lang erhitzt. Dann wird die Reaktionsmischung auf Eis gegossen, mit HCl angesäuert, wobei ein fester Niederschlag ausfällt, der filtriert und im Vakuum getrocknet wird. Ergebnis: 2-{[7-Chlor-3-(2-fluorphenyl)-1,2-benzisoxazol-3-yl]oxy}-propionsäure, Fp. 159 bis 161°C.

Analyse:

Berechnet für $C_{16}H_{11}ClFNO_4$:
57,2% C; 3,28% H; 4,19% N; 10,55% Cl.
Gefunden:
56,8% C; 3,24% H; 4,17% N; 10,51% Cl.

Beispiel 39

a) Zu einer Mischung von 21 g 2,5-Difluorbenzoylchlorid und 20,4 g 2-Chlor-resorcin-dimethyläther in 250 ml 1,2-Dichloräthan werden bei 5–10°C 15,7 g $AlCl_3$ in Portionen gegeben. Man läßt die Reaktionsmischung auf Raumtemperatur kommen und kocht sie dann 30 Minuten unter Rückfluß. Anschließend wird sie in konz. HCl und Eis gegossen und etwa 1 Stunde gerührt. Das Produkt wird mit Essigester extrahiert, über $Na_2SO_4$ getrocknet und eingedampft. Ergebnis: 3-Chlor-2',5'-difluor-2-hydroy-4-methoxybenzophenon, Fp. 178–180°C.

Analyse:

Berechnet für $C_{14}H_9ClF_2O_3$:
56,30% C; 3,04% H; 12,72% N.
Gefunden:
56,16% C; 3,01% H; 12,75% N.

b) Eine Mischung von 28 g 3-Chlor-2',5'-difluor-2-hydroxy-4-methoxybenzophenon und 26 g Hydroxylamin-HCl in 250 ml Pyridin wird 3 Stunden unter Rückfluß gekocht. Das Pyridin wird verdampft und der Rückstand zwischen Essigester und 5%iger HCl verteilt. Der Essigester-Extrakt wird mit Wasser gewaschen, über $Na_2SO_4$ getrocknet und eingedampft. Ein festes Produkt in Form einer Isomerenmischung wird erhalten. Der Feststoff wird etwa 30–45 Minuten auf 200–210°C erhitzt und aus Toluol umkristallisiert. Ergebnis: E-3-Chlor-2',5'-difluor-2-hydroxy-4-methoxybenzophenonoxim, Fp. 209–210°C.

Analyse:

Berechnet für $C_{14}H_{10}ClFNO_3$:
53,60% C; 3,29% H; 4,47% N.
Gefunden:
53,61% C; 3,22% H; 4,43% N.

c) Eine Mischung von 19 g E-3-Chlor-2',5'-difluor-2-hydroxy-4-methoxybenzophenonoxim wird mit 9 ml Essigsäureanhydrid 30 Minuten auf 60°C erwärmt. Beim Abkühlen erstarrt die Mischung. Der Rückstand wird zwischen Essigester und 10%iger $NaHCO_3$ verteilt. Der Essigester-Extrakt wird mit Wasser gewaschen, über $Na_2SO_4$ getrocknet und eingedampft. Ergebnis: E-3-Chlor-2',5'-difluor-2-hydroxy-4-methoxybenzophenon-O-acetyloxim, Fp. 130–131°C.

Analyse:

Berechnet für $C_{16}H_{12}ClFNO_4$:
54,02% C; 3,40% H; 3,94% N.
Gefunden:
53,76% C; 3,37% H; 3,89% N.

d) Zu einer Suspension von NaH/200 ml DMF werden unter $N_2$-Atomosphäre 19,5 g E-3-Chlor-2',5'-difluor-2-hydroxy-4-methoxybenzophenon-O-acetyloxim in 50 ml DMF getropft. Die Mischung wird 30 Minuten gerührt und Wasser wird zugegeben, wobei 7-Chlor-3-(2,5-difluorphenyl)-6-methoxy-1,2-benzisoxazol, Fp. 159–161°C, ausfällt.

Analyse:

Berechnet für $C_{14}H_8ClF_2NO_2$:
56,87% C; 2,73% H; 4,74% N.
Gefunden:
56,74% C; 2,70% H; 4,70% N.

e) Eine Mischung von 10 g 7-Chlor-3-(2,5-difluorphenyl)-6-methoxy-1,2-benzisoxazol und 40 g

Pyridin-HCl wird 45 Minuten auf 200°C erhitzt. Die heiße Mischung wird in kräftig gerührtes Eiswasser gegossen, wobei ein Produkt ausfällt, das nach Filtrieren und Trocknen 7-Chlor-3-(2,5-difluorphenyl)-6-hydroxy-1,2-benzisoxazol, Fp. 256–257°C, ergibt.

Analyse:

Berechnet für $C_{13}H_6ClF_2NO_2$:
55,43% C; 2,15% H; 4,97% N.
Gefunden:
55,26% C; 2,02% H; 4,93% N.

f) 4,97 g $K_2CO_3$ und 6,07 g Bromessigsäureäthylester werden zu einer Lösung von 9,3 g 7-Chlor-3-(2-difluorphenyl)-6-hydroxy-1,2-benzisoxazol in 100 ml DMF getropft. Die Mischung wird 2 Stunden bei 60°C gehalten. 200 ml Wasser und 15 ml 50%ige NaOH werden zu der Mischung gegeben, die dann 90 Minuten bei 90°C gerührt, in Wasser gegossen und dann angesäuert wird. Das Produkt wird mit Essigester extrahiert, über $Na_2SO_4$ getrocknet und eingedampft. Ergebnis: {[7-Chlor-3-(2,5-difluorphenyl)-1,2-benzisoxazol-6-yl]oxy}-essigsäure.

Analyse:

Berechnet für $C_{15}H_8ClF_2NO_4$:
53,04% C; 2,37% H; 4,12% N.
Gefunden:
53,37% C; 2,39% H; 3,01% N.

Beispiel 40

a) 3,4 g 2-Chlorresorcindimethyläther werden in 20 ml $CH_2Cl_2$ gelöst und mit 4,3 ml $TiCl_4$ versetzt. Die erhaltene Lösung wird mit 2,3 g Dichlormethylmethyläther versetzt. Nach 30 Minuten wird die Reaktionsmischung in Wasser gegossen und mit Äther extrahiert. Der Extrakt wird getrocknet und verdampft, wobei man 3-Chlor-2,4-dimethoxybenzaldehyd erhält mit einem Schmelzpunkt von 107–108°C.

Analyse:

Berechnet für $C_9H_9ClO_3$:
53,88% C; 4,52% H; 17,68% Cl.
Gefunden:
53,93% C; 4,52% H; 17,42% Cl.

b) 2,75 g 3-Chlor-2,4-dimethoxybenzaldehyd werden 30 Minuten lang in 20 ml Dichloräthan mit 1,8 g $AlCl_3$ unter Rückfluß erhitzt. Das Reaktionsgemisch wird in Wasser gegossen und mit $CH_2Cl_2$ extrahiert. Nach Verdampfung und Umkristallisation aus Isopropanol erhält man 3-Chlor-2-hydroxy-4-methoxybenzaldehyd mit einem Schmelzpunkt von 125°C.

Analyse:

Berechnet für $C_8H_7ClO_3$:
51,49% C; 3,78% H; 19,00% Cl.
Gefunden:
51,33% C; 3,78% H; 18,65% Cl.

c) 1,48 g 3-Chlor-2-hydroxy-4-methoxybenzaldehyd werden in 15 ml $H_2O$ suspendiert und mit 1,08 g Hydroxyamin-o-sulfonsäure und 0,1 g $Na_2SO_4$ versetzt. Nach 3 Stunden werden weitere 15 ml Wasser zugegeben. Nach insgesamt 4 Stunden wird das Reaktionsmischung mit einer 8%igen $NaHCO_3$-Lösung behandelt und in Äther extrahiert. Der Extrakt wird verdampft und mit Hexan verrieben, wobei sich ein Feststoff bildet. Nach Umkristallisation aus Toluol/Hexan erhält man 7-Chlor-6-methoxy-1,2-benzisoxazol mit einem Schmelzpunkt von 115–118°C.

Analyse:

Berechnet für $C_8H_6ClNO_2$:
52,33% C; 3,29% H; 7,63% N.
Gefunden:
52,35% C; 3,30% H; 7,71% N.

d) Beispiel 35e und 35f werden unter Verwendung von 7-Chlor-6-methoxy-1,2-benzisoxazol wiederholt, wobei man {[7-Chlor-1,2-benzisoxazol-6-yl]oxy}-essigsäure erhält.

Beispiel 41

a) Eine Lösung aus 1,7 g 2-Chlorresorcindimethyläther und 2,08 g o-Trifluormethylbenzoylchlorid in 50 ml 1,2-Dichloräthan wird allmählich mit 1,6 g Eisenchlorid bei einer Temperatur von 5−7°C versetzt. Das Gemisch wird auf Zimmertemperatur erhitzt, 18 Stunden stehengelassen, 30 Minuten lang unter Rückfluß erhitzt und in 5%ige Salzsäure mit Eis gegossen. Die wäßrige Phase wird mit zusätzlichem organischen Lösungsmittel extrahiert. Die vereinigten organischen Extrakte werden mit Wasser gewaschen, über $Na_2SO_4$ getrocknet und verdampft, wobei man 3-Chlor-2-hydroxy-4-methoxy-2′-trifluormethylbenzophenon mit einem Schmelzpunkt von 101−102°C erhält.

Analyse:

Berechnet für $C_{15}H_{10}ClF_3O_3$:
54,48% C; 3,05% H; 17,24% F.
Gefunden:
54,16% C; 2,91% H; 17,16% F.

b) Es wird wie in den obigen Beispielen, z. B. wie im Beispiel 35, verfahren, wobei 3-Chlor-2-hydroxy-4-methoxy-2′-trifluormethyl-benzophenon zum entsprechenden Oxim umgesetzt wird, das zyklisiert wird und aus dem dann die {[7-Chlor-3-(2-trifluorphenyl)-1,2-benzisoxazol-6-yl]oxy}-essigsäure gebildet wird.

Beispiel 42

a) 10 g 7-Chlor-3-(2-fluorphenyl)-6-methoxy-1,2-benzisoxazol aus Beispiel 36b werden in 800 ml Eisessig unter Rühren gelöst. Dann wird gasförmiges Chlor langsam eine halbe Stunde hindurchgeleitet, wobei eine Lösung entsteht, die eine kleine Menge suspendierten Ausgangsmaterials enthält. Die Reaktionsmischung wird 18 Stunden bei Zimmertemperatur gerührt und dann unter Rühren in Eiswasser gegossen, wobei 5,7-Dichlor-3-(2-fluorphenyl)-6-methoxy-1,2-benzisoxazol ausfällt. Schmelzpunkt: 121°C.

Analyse:

Berechnet für $C_{14}H_8Cl_2FNO_2$:
53,87% C; 2,59% H; 4,49% N.
Gefunden:
53,54% C; 2,59% H; 4,51% N.

b) 10 g 5,7-Dichlor-3-(2-fluorphenyl)-6-methoxy-1,2-benzisoxazol werden mit 100 g Pyridinhydrochlorid versetzt und eine halbe Stunde bei 200°C erhitzt. Die heiße Schmelze wird schnell in gerührtes Eiswasser gegossen, wobei sich ein Niederschlag bildet, der abfiltriert wird und 48 Stunden im Vakuum bei 64°C getrocknet wird. Der Feststoff wird aus Toluol umkristallisiert, wobei man 5,7-Dichlor-3-(2-fluorphenyl)-6-hydroxy-1,2-benzisoxazol mit einem Schmelzpunkt von 194 bis 196°C erhält.

Analyse:

Berechnet für $C_{13}H_6Cl_2FNO_2$:
52,44% C; 2,01% H; 4,70% N; 23,53% Cl.
Gefunden:
52,07% C; 2,08% H; 4,96% N; 23,92% Cl.

c) 1,4 g NaH werden unter Rühren in 50 ml DMF suspendiert. Zu der erhaltenen Suspension wird eine

Lösung aus 7,2 g 5,7-Dichlor-3-(2-fluorphenyl)-6-hydroxy-1,2-benzisoxazol in 50 ml DMF getropft. Die Lösung wird eine Stunde auf 45°C erhitzt. Dann werden 4,0 g Bromessigsäureäthylester in 20 ml DMF zugetropft, und das Reaktionsgemisch wird 2 Stunden bei 40°C gerührt. Die Lösung wird in 1 l Wasser gegossen, gerührt und mit Essigester extrahiert. Die organischen Phasen werden mit gesättigter NaCl-Lösung gewaschen, das Lösungsmittel wird im Vakuum entfernt, wobei man {[5,7-Dichlor-3-(2-fluorphenyl)-1,2-benzisoxazol-6-yl]oxy}-essigsäureäthylester erhält. Schmelzpunkt: 105–106°C.

Analyse:

Berechnet für $C_{17}H_{12}Cl_2FNO_2$:
53,26% C; 3,13% H; 3,65% N; 18,39% Cl.
Gefunden:
52,91% C; 3,00% H; 3,41% N; 18,09% Cl.

d) 14,0 g {[5,7-Dichlor-3-(2-fluorphenyl)-1,2-benzisoxazol-6-yl]oxy}-essigsäureäthylester werden in 700 ml einer Mischung aus 75%igem Äthanol und Wasser unter Rühren erhitzt, bis sich alle Komponenten gelöst haben. Dann gibt man 20 ml einer 50%igen Natronlauge hinzu, wobei sich ein Niederschlag bildet, der unter weiterem Rühren und Erhitzen in Lösung geht und dann 2,5 Stunden stehengelassen wird. Das Äthanol wird im Vakuum verdampft, und der Rückstand wird mit 10%iger Salzsäure angesäuert. Der niedergeschlagene Feststoff wird abfiltriert und im Vakuum getrocknet, wobei man {[5,7-Dichlor-3-(2-fluorphenyl)-1,2-benzisoxazol-6-yl]oxy}-essigsäure erhält. Schmelzpunkt: 160–170°C.

Analyse:

Berechnet für $C_{15}H_8Cl_2FNO_4$:
50,59% C; 2,26% H; 3,93% N.
Gefunden:
50,53% C; 2,32% H; 3,88% N.

Beispiel 43

a) Eine Suspension von 1,0 g NaH in 50 ml DMF wird mit einer Lösung aus 5,0 g 7-Chlor-3-(2-fluorphenyl)-6-hydroxy-1,2-benzisoxazol aus Beispiel 36c in 50 ml DMF versetzt. Die Lösung wird eine Stunde bei Zimmertemperatur gerührt, auf 5°C abgekühlt und auf einmal mit 3,5 g N,N-Dimethylthiocarbamoylchlorid versetzt. Das Reaktionsgemisch wird allmählich auf 60°C erhitzt und 2,5 Stunden gerührt. Die Lösung wird in Wasser gegossen und mit Methylenchlorid extrahiert, bis die Extrakte farblos werden. Die vereinigten organischen Extrakte werden nacheinander mit 10%iger $K_2CO_3$-Lösung und mit gesättigter Natriumchloridlösung gewaschen. Das Lösungsmittel wird im Vakuum entfernt, wobei 7-Chlor-6-(O-N,N-dimethylthiocarbamoyl)-3-(2-fluorphenyl)-1,2-benzisoxazol entsteht. Schmelzpunkt: 153–154°C.

Analyse:

Berechnet für $C_{16}H_{12}ClFN_2O_2S$:
54,8% C; 3,4% H; 7,9% N; 9,1% S.
Gefunden:
54,4% C; 3,4% H; 7,9% N; 9,1% S.

b) 4,5 g 7-Chlor-6-(O-N,N-dimethylthiocarbamyl)-3-(2-fluorphenyl)-1,2-benzisoxazol werden unter Stickstoff 45 Minuten bei 205°C erhitzt. Die beim Abkühlen erhaltene feste Substanz wird aus Essigester umkristallisiert, wobei 7-Chlor-6-(S-N,N-dimethylthiocarbamyl)-3-(2-fluorphenyl)-1,2-benzisoxazol als farblose Prismen anfällt. Schmelzpunkt: 140–142°C.

Analyse:

Berechnet für $C_{16}H_{12}ClFN_2O_2S$:
54,77% C; 3,44% H; 7,98% N; 9,14% S.
Gefunden:
54,87% C; 3,56% H; 7,86% N; 9,28% S.

c) 2,0 g 7-Chlor-6-(S-N,N-dimethylthiocarbamyl)-3-(2-fluorphenyl)-1,2-benzisoxazol werden in Methanol gelöst und mit 25 ml 15%iger wäßriger Natronlauge versetzt. Die erhaltene Lösung wird

drei Stunden unter Rückfluß erhitzt. Das Reaktionsgemisch wird in eine große Menge Wasser gegossen und mit Salzsäure angesäuert, wobei 7-Chlor-3-(2-fluorphenyl)-6-mercapto-1,2-benzisoxazol ausfällt. Schmelzpunkt: 125–129°C.

Analyse:

Berechnet für $C_{13}H_7ClFNOS$:
55,81% C; 2,50% H; 5,01% N; 11,46% S.
Gefunden:
55,94% C; 2,58% H; 5,09% N; 11,52% S.

d) 60 ml DMF werden mit 3,2 g 7-Chlor-3-(2-fluorphenyl)-6-mercapto-1,2-benzisoxazol, 3,1 g $K_2CO_3$ und 3,67 g Bromessigsäureäthylester versetzt und 2 Stunden bei einer Temperatur von 50°C unter Rühren erhitzt. Die erhaltene Lösung wird in 700 ml Wasser gegossen und mit Essigester extrahiert. Die vereinigten organischen Extrakte werden über $K_2CO_3$ getrocknet, und das Lösungsmittel wird im Vakuum entfernt, wobei {[7-Chlor-3-(2-fluorphenyl)-1,2-benzisoxazol-6-yl[]thio}-essigsäureäthylester erhalten wird.

Analyse:

Berechnet für $C_{17}H_{13}ClFNO_3S$:
55,89% C; 3,56% H; 3,83% N; 8,76% S.
Gefunden:
55,92% C; 3,70% H; 3,80% N; 8,91% S.

e) 1,7 g {[7-Chlor-3-(2-fluorphenyl)-1,2-benzisoxazol-6-yl]thio}-essigsäureäthylester werden unter Rühren und leichtem Erhitzen in 50 ml Äthanol gelöst und mit 3 ml einer 50%igen Natronlauge mit 25 ml Wasser versetzt, wobei sich ein fester Niederschlag bildet. Nach einer Stunde wird das Äthanol entfernt, und der Rückstand wird mit Salzsäure angesäuert. Der Niederschlag wird abfiltriert und getrocknet, wobei {[5,7-Dichlor-3-(2-fluorphenyl)-1,2-benzisoxazol-6-yl]thio}-essigsäure anfällt. Schmelzpunkt: 165°C.

Analyse:

Berechnet für $C_{15}H_9ClFNO_3S$:
53,41% C; 2,67% H; 4,15% N; 9,49% S.
Gefunden:
53,39% C; 2,68% H; 4,19% N; 9,44% S.

Beispiel 44

a) Eine Lösung von 47,6 g o-Fluorbenzoylchlorid in 100 ml Dichlormethan wird innerhalb von 30 Minuten portionsweise mit 40,0 $AlCl_3$ versetzt, wobei man eine dunkle Lösung erhält, zu der innerhalb von 15 Minuten eine Lösung von 52,0 g 1-Chlor-3,5-dimethylbenzol in 120 ml Dichlormethan zutropft. Die Mischung wird 4 Stunden bei Zimmertemperatur gerührt, in 1 Liter mit Eis verdünnter Salzsäurelösung gegossen und 30 Minuten gerührt. Die organische Schicht wird abgetrennt und eingedampft, wobei ein Öl zurückbleibt, das in Äther gelöst wird und dann mit Wasser, verdünnter Natronlauge, Wasser und gesättigter Kochsalzlösung gewaschen wird und über wasserfreiem Magnesiumsulfat getrocknet wird. Nach dem Filtrieren wird das Lösungsmittel verdampft, wobei eine feste Substanz zurückbleibt, die mit Silicagel gereinigt wird und mit Dichlormethan eluiert wird. Man erhält dabei 2-Chlor-4,6-dimethoxy-2'-fluorbenzophenon mit einem Schmelzpunkt von 88–92°C.

Analyse:

Berechnet für $C_{15}H_{12}ClFO_3$:
61,13% C; 4,11% H; 12,03% Cl; 6,45% F.
Gefunden:
60,95% C; 4,06% H; 11,85% Cl; 6,38% F.

b) Zu einer Lösung von 33 g 2-Chlor-4,6-dimethoxy-2'-fluorbenzophenon in 150 ml Dichloräthan werden innerhalb von 15 Minuten portionsweise 15 g $AlCl_3$ gegeben. Die Mischung wird 3 Stunden unter Rückfluß (90°C) unter Rühren erhitzt, abgekühlt, in 1 Liter mit Eis vedünnter Salzsäurelösung gegossen, 30 Minuten gerührt und mit Äther extrahiert. Die Äther/Dichloräthanlösung wird

mit Wasser und gesättigter NaCl-Lösung gewaschen, mit wasserfreiem Magnesiumsulfat getrocknet und filtriert. Dann werden die Lösungsmittel verdampft, wobei 2-Chlor-2'-fluor-6-hydroxy-4-methoxybenzophenon mit einem Schmelzpunkt von 85–90°C anfällt.

Analyse:

Berechnet für $C_{14}H_{10}ClFO_3$:
59,90% C; 3,59% H; 6,77% F.
Gefunden:
59,78% C; 3,53% H; 7,00% F.

c) 125 ml Pyridin werden mit 28,5 g 2-Chlor-2'-fluor-6-hydroxy-4-methoxybenzophenon und 14 g Hydroxylaminhydrochlorid versetzt, 3 Stunden unter Rückfluß (120°C) gerührt und abgekühlt. Das Pyridin wird dann verdampft, wobei ein halbfester gelber Stoff zurückbleibt, der in Äther gelöst wird, mit Wasser und gesättigter Kochsalzlösung gewaschen wird und über wasserfreiem Magnesiumsulfat getrocknet wird. Nach dem Filtrieren wird das Lösungsmittel verdampft, wobei ein Öl zurückbleibt, das nach Verreiben mit Petroleumäther beim Erstarren Z-2-Chlor-2'-fluor-6-hydroxy-4-methoxybenzophenoxim liefert. Schmelzpunkt: 130–140°C.

Analyse:

Berechnet für $C_{14}H_{11}ClFNO_3$:
56,86% C; 3,75% H; 4,74% N.
Gefunden:
56,74% C; 3,70% H; 4,74% N.

d) Z-2-Chlor-2'-fluor-6-hydroxy-4-methoxybenzophenoxim kann mit Acetanhydrid, wie im Beispiel 30c beschrieben, zum E-2-Chlor-2'-fluor-6-hydroxy-4-methoxybenzophenon-O-acetyloxim umgesetzt werden.

e) Eine Suspension aus 2,4 g NaH in 20 ml DMF wird mit einer Lösung aus 15 g E-2-Chlor-2'-fluor-6-hydroxy-4-methoxybenzophenon-O-acetyloxim in 50 ml DMF versetzt, 2 Stunden bei Zimmertemperatur gerührt, in 1 Liter Eiswasser gegossen und 30 Minuten gerührt, wobei sich ein Niederschlag bildet, der mit Wasser gewaschen wird und getrocknet wird, wobei man 4-Chlor-3-(2-fluorphenyl)-6-methoxy-1,2-benzisoxazol mit einem Schmelzpunkt von 113–115°C erhält.

Analyse:

Berechnet für $C_{14}H_{19}ClFNO_2$:
60,55% C; 3,27% H; 5,05% N.
Gefunden:
60,52% C; 3,33% H; 4,96% N.

f) Es wird wie in den vorstehenden Beispielen, z. B. Beispiel 20d und 20e, verfahren, wobei unter Verwendung von 4-Chlor-3-(2-fluorphenyl)-6-methoxy-1,2-benzisoxazol {[4-Chlor-3-(2-fluorphenyl)-1,2-benzisoxazol-6-yl]oxy}-essigsäure erhalten wird. Schmelzpunkt: 172–174°C.

Beispiel 45

a) 5 g o-Methoxybenzoylchlorid werden zu einer Mischung aus 3,9 g $AlCl_3$ und 4,67 g 2,3-Dichloranisol in 70 ml 1,2-Dichloräthan bei −10°C getropft. Die Mischung wird 2,5 Stunden gerührt und allmählich auf 5°C erwärmt. Die Reaktionsmischung wird dann in eine Mischung von konzentrierter Salzsäure und Eis gegossen und eine halbe Stunde gerührt, wobei die Komplexverbindung abgebaut wird. Die wäßrige Schicht wird mit zusätzlichem organischem Lösungsmittel extrahiert. Die vereinigten organischen Phasen werden neutral gewaschen, über $Na_2SO_4$ getrocknet und eingedampft, wobei ein Öl zurückbleibt, das beim Verreiben mit Hexan fest wird. Das Rohprodukt wird aus 95%igem Äthanol zu 2,3-Dichlor-2',4-dimethoxybenzophenon umkristallisiert. Schmelzpunkt 94–96°C.

Analyse:

Berechnet für $C_{15}H_{12}Cl_2O_3$:
58,00% C; 3,89% H.
Gefunden:
57,84% C; 3,81% H.

b) Eine feste Mischung aus 13 g 2,3-Dichlor-2',4-dimethoxybenzophenon und 52 g Pyridinhydrochlorid wird eine Stunde bei 200°C erhitzt. Die heiße Mischung wird dann in kräftig gerührtes Eiswasser gegossen und mit Äthylacetat extrahiert. Der Extrakt wird über $Na_2SO_4$ getrocknet und eingedampft, wobei 2,3-Dichlor-2',4-dihydroxybenzophenon anfällt. Schmelzpunkt: 197—201°C.

Analyse:

Berechnet für $C_{13}H_3Cl_2O_3$:
55,15% C; 2,80% H.
Gefunden:
55,26% C; 2,86% H.

c) Eine Suspension von 2,87 g Natriumhydrid in 150 ml DMF wird nacheinander mit 30,76 g 2,3-Dichlor-2',4-dihydroxybenzophenon in 100 ml DMF und mit 18,37 g Äthylbromacetat versetzt. Die Mischung wird ca. 1 ½ Stunden gerührt, auf Eis und Säure gegeben und mit $CHCl_3$ extrahiert. Der $CHCl_3$-Extrakt wird über $Na_2SO_4$ getrocknet und verdampft, wobei 2,3-Dichlor-4-(2-hydroxybenzoyl)-phenoxyessigsäureäthylester zurückbleibt. Schmelzpunkt: 109—110°C.

Analyse:

Berechnet für $C_{17}H_{14}Cl_2O_5$:
55,30% C; 3,82% H.
Gefunden:
55,15% C; 3,81% H.

d) Es wird wie in den vorstehenden Beispielen verfahren, wobei unter Verwendung von 2,3-Dichlor-4-(2-hydroxybenzoyl)-phenoxyessigsäureäthylester das entsprechende Oxim erhalten wird, das zyklisiert wird. Der dabei entstandene Ester wird zur [7-Chlor-3-(2-hydroxyphenyl)-1,2-benzisoxazol-6-yl]oxyessigsäure hydrolysiert.

Beispiel 46

a) Zu einer Lösung von 25 g Phenacetylchlorid in 150 ml Schwefelkohlenstoff werden zunächst unter Rühren innerhalb von 30 Minuten portionsweise 22 g $AlCl_3$ und dann eine Lösung von 2,3-Dichloranisol (28 g) in 50 ml Schwefelkohlenstoff gegeben.
Die Mischung wird drei Stunden unter Rückfluß (50°C) und Rühren erhitzt, mit 22 g $AlCl_3$ versetzt, 2 Stunden unter Rückfluß gerührt, abgekühlt, in eine kalte 15%ige Salzsäurelösung gegossen, 30 Minuten gerührt und mit Äthylacetat/Äthyläther extrahiert. Der organische Extrakt wird mit Wasser und gesättigter NaCl-Lösung gewaschen und über wasserfreiem Magnesiumsulfat getrocknet.
Es wird filtriert, und die Lösungsmittel werden verdampft, wobei man 2,3-Dichlor-4-phenacetylphenol erhält. Schmelzpunkt: 173—180°C.

Analyse:

Berechnet für $C_{14}H_{10}Cl_2O_2$:
59,81% C; 3,59% H.
Gefunden:
60,15% C; 3,65% H.

b) Gemäß den vorstehenden Beispielen wird 2,3-Dichlor-4-phenacetylphenol mit Hydroxylaminhydrochlorid in Pyridin zu 2,3-Dichlor-4-phenacetylphenoloxim umgesetzt.

c) Eine Lösung aus 6,3 g 2,3-Dichlor-4-phenacetylphenoloxim in 25 ml trockenem DMF wird zu einer Suspension aus 2,54 g NaH in 10 ml trockenem DMF gegeben.
Die Reaktionsmischung wird 2 Stunden bei 80°C gerührt, abgekühlt, mit einer Lösung von 4,2 g Äthylbromacetat in 10 ml trockenem DMF versetzt und jeweils 30 Minuten zunächst bei Zimmertemperatur und dann bei 60°C gerührt.
Die Mischung wird abgekühlt, in 500 ml Wasser gegossen, 30 Minuten gerührt und mit Essigester extrahiert. Die organische Schicht wird mit Wasser und gesättigter NaCl-Lösung gewaschen und über wasserfreiem Magnesiumsulfat getrocknet. Nach dem Filtrieren werden die Lösungsmittel verdampft, wobei ein Öl zurückbleibt, aus dem {[3-Benzyl-7-chlor-1,2-benzisoxazol-6-yl]oxy}-essigsäureäthylester hergestellt wird. Schmelzpunkt: 120—122°C.

Analyse:

Berechnet für $C_{18}H_{14}ClNO_4$:
62,52% C; 4,66% H; 4,05% N.
Gefunden:
62,35% C; 4,74% H; 3,83% N.

d) 650 ml absoluten Äthanols werden nacheinander mit 25,0 g {[(3-Benzyl-7-chlor-1,2-benzisoxazol-6-yl)oxy]}-essigsäureäthylester und mit 30 ml 50%iger Natronlauge versetzt. Die Mischung wird 1 Stunde unter Rückfluß erhitzt (80°C), mit 500 ml Wasser versetzt, der pH-Wert wird mit konzentrierter Salzsäure auf 1 eingestellt, und dann wird ein weiterer Liter Wasser zugegeben. Der sich bildende Niederschlag wird abgetrennt, mit Wasser gewaschen und in Dichlormethan gelöst. Die Dichlormethanlösung wird mit Wasser und mit gesättigter NaCl-Lösung gewaschen und über wasserfreiem Magnesiumsulfat getrocknet.
Nach dem Filtrieren wird das Lösungsmittel verdampft, wobei {[(3-Benzyl-7-chlor-1,2-benzisoxazol-6-yl)oxy]}-essigsäure zurückbleibt. Schmelzpunkt: 147–153°C.

Analyse:

Berechnet für $C_{16}H_{12}ClNO_4$:
60,48% C; 3,81% H; 4,41% N; 11,16% Cl.
Gefunden:
60,36% C; 3,96% H; 4,33% N; 10,91% Cl.

## Beispiel 47

a) Die Friedel-Crafts-Methode aus Beispiel 46 wird wiederholt, wobei man unter Verwendung von 2,3-Dichloranisol, 1-Naphthylchlorid und $AlCl_3$ (2,3-Dichlor-4-hydroxyphenyl)-(1-naphthyl)-methanon enthält.

b) Eine Lösung aus 22,48 g (2,3-Dichlor-4-hydroxyphenyl)-(1-naphthyl)-methanon in 150 ml Pyridin wird mit 9,87 g Hydroxylaminhydrochlorid versetzt. Die erhaltene Mischung wird ca. 64 Stunden unter Rückfluß erhitzt. Weitere 9,87 g Hydroxylaminhydrochlorid werden zugegeben, und das Reaktionsgemisch wird 18 Stunden unter Rückfluß erhitzt. Das Pyridin wird im Vakuum verdampft, und Rückstand wird zwischen 5%iges HCl und Essigester verteilt. Der Essigesterextrakt wird mit Wasser gewaschen, über $Na_2SO_4$ getrocknet und verdampft, wobei ein halbfester Stoff zurückbleibt, der in ca. 100 ml Essigester gelöst wird und dann durch eine Kolonne mit Holzkohle geschickt wird. Das Filtrat wird eingedampft, wobei ein Feststoff anfällt, der nach Verreiben mit Hexanäther (2,3-Dichlor-4-hydroxyphenyl)-(1-naphthyl)-methanonoxim mit einem Schmelzpunkt von 145–160°C liefert.

Analyse:

Berechnet für $C_{17}H_{11}Cl_2NO_2$:
61,46% C; 3,34% H; 4,22% N.
Gefunden:
61,53% C; 3,45% H; 4,14% N.

c) Eine Lösung aus 3 g (2,3-Dichlor-4-hydroxyphenyl)-(1-naphthyl)-methanonoxim in 25 ml DMF wird mit 0,54 g NaH unter Stickstoff versetzt. Das Reaktionsgemisch wird auf eine Innentemperatur von 100°C eine Stunde und 20 Minuten lang erhitzt und auf Zimmertemperatur abgekühlt. Dann werden 1,65 g Bromessigsäureäthylester zugetropft. Die Reaktionsmischung wird 18 Stunden gerührt, mit Wasser versetzt und mit Essigester extrahiert. Der Essigesterextrakt wird mit Wasser gewaschen, über $Na_2SO_4$ getrocknet und eingedampft, wobei {[7-Chlor-3-(1-naphthyl)-1,2-benzisoxazol-6-yl]oxy}-essigsäureäthylester mit einem Schmelzpunkt von 75–85°C zurückbleibt.

Analyse:

Berechnet für $C_{21}H_{16}ClNO_4$:
66,06% C; 4,22% H; 3,67% N.
Gefunden:
65,81% C; 4,24% H; 3,53% N.

d) Eine Suspension aus 1,85 g {[7-Chlor-3-(1-naphthyl)-1,2-benzisoxazol-6-yl]oxy}-essigsäureäthylester, 100 ml Äthanol und 2 ml 50%iger Natronlauge wird eine Stunde unter Rückfluß erhitzt.

Das heiße Gemisch wird erst mit 100 ml Wasser und dann mit so viel konzentrierter Salzsäure versetzt, daß es sauer wird. Die Mischung wird dann gerührt und abgekühlt, wobei sich ein Niederschlag bildet. Äthanol wird im Vakuum eingedampft und {[7-Chlor-3-(1-naphthyl)-1,2-benzisoxazol-6-yl]oxy}-essigsäure wird abfiltriert und im Vakuum getrocknet. Schmelzpunkt: 172—174°C.

Analyse:

Berechnet für $C_{19}H_{12}ClNO_4$:
64,50% C; 3,42% H; 3,96% N.
Gefunden:
64,51% C; 3,38% H; 3,97% N.

Beispiel 48

a) Entsprechend der Friedel-Crafts-Reaktion in den vorstehenden Beispielen wird unter Verwendung von 2,3-Dichloranisol, 3-Fluorbenzoylchlorid und $AlCl_3$ 2,3-Dichlor-4-hydroxy-3'-fluorbenzophenon erhalten.

b) Eine Lösung von 5 g 2,3-Dichlor-4-hydroxy-3'-fluorbenzophenon in 50 ml Pyridin wird mit 1,58 g Hydroxylaminhydrochlorid versetzt. Die Mischung wird 18 Stunden unter Rückfluß erhitzt. Das Pyridin wird im Vakuum verdampft, und der Rückstand wird zwischen Essigester und 5% HCl verteilt.
Die Essigester-Phase wird mit Wasser gewaschen, über $Na_2SO_4$ getrocknet und verdampft, wobei 2,3-Dichlor-4-hydroxy-3'-fluorbenzophenon in Form eines Isomerengemisches zurückbleibt. Schmelzpunkt: 178—185°C.

Analyse:

Berechnet für $C_{14}H_{10}Cl_2FNO_2$:
53,52% C; 3,21% H; 4,46% N.
Gefunden:
53,66% C; 3,14% H; 4,39% N.

c) Eine Lösung aus 3 g 2,3-Dichlor-4-hydroxy-3'-fluorbenzophenonoxim in 20 ml DMF wird mit 0,25 g NaH in einer $N_2$-Atmosphäre versetzt. Die Mischung wird 18 Stunden gerührt, dann 1 Stunde auf 100°C erhitzt und in Eiswasser gegeben, wobei 7-Chlor-3-(3-fluorphenyl)-6-methoxy-1,2-benzisoxazol anfällt. Schmelzpunkt: 149—150°C.

Analyse:

Berechnet für $C_{14}H_9ClFNO_4$:
60,55% C; 3,26% H; 5,05% N.
Gefunden:
60,54% C; 3,00% H; 4,91% N.

d) Ein festes Gemisch aus 14,47 g 7-Chlor-3-(3-fluorphenyl)-6-methoxy-1,2-benzisoxazol und 58 g Pyridinhydrochlorid wird 1 Stunde bei 190—200°C erhitzt. Die heiße Mischung wird in kräftig gerührtes Eiswasser gegossen. Dann wird 7-Chlor-6-hydroxy-3-(3-fluorphenyl)-1,2-benzisoxazol abfiltriert und sorgfältig mit Wasser gewaschen. Schmelzpunkt: 215—217°C.

Analyse:

Berechnet für $C_{13}H_7ClFNO_2$:
59,22% C; 2,68% H; 5,31% N.
Gefunden:
59,15% C; 2,66% H; 5,16% N.

e) Eine Mischung aus 1,3 g NaH in 25 ml DMF wird nacheinander mit einer Lösung aus 10,2 g 7-Chlor-6-hydroxy-3-(3-fluorphenyl)-1,2-benzisoxazol und mit einer Lösung aus 6,68 g Bromessigsäureäthylester in 25 ml DMF versetzt und 2½ Stunden gerührt. Dann gibt man 10 ml 50%iger Natronlauge, 175 ml Wasser und 30 ml DMF hinzu und rührt die Reaktionsmischung 1 Stunde bei 80—85°C. Die Mischung wird dann mit konzentrierter Salzsäure und angesäuert und mit Wasser versetzt. Dann wird {[7-Chlor-3-(3-fluorphenyl)-1,2-benzisoxazol-6-yl]oxy}-essigsäure abfiltriert. Schmelzpunkt: 205—209°C.

Analyse:

Berechnet für $C_{15}H_9ClFNO_4$:

56,00% C; 2,83% H; 4,46% N.
Gefunden:
55,96% C; 2,81% H; 4,21% N.

## Beispiel 49

a) Durch eine Lösung aus 1,0 g 4-Chlor-3-(o-fluorphenyl)-6-methoxy-1,2-benzisoxazol aus Beispiel 44e in 55 ml Eisessig wird gasförmiges Chlor geleitet. Es wird 1 Stunde bei Zimmertemperatur gerührt, dann wird die Mischung in 500 ml Wasser gegossen und 15 Minuten gerührt, wobei sich ein Niederschlag bildet, der mit Äther/Äthylacetat extrahiert wird. Die organische Schicht wird mit Wasser und mit gesättigter Kochsalzlösung gewaschen, über wasserfreiem Magnesiumsulfat getrocknet, und nach dem Filtrieren werden die Lösungsmittel verdampft, wobei 3-(o-Fluorphenyl)-6-methoxy-4,5,7-trichlor-1,2-benzisoxazol zurückbleibt. Schmelzpunkt: 120—140°C.

Analyse:

Berechnet für $C_{14}H_7Cl_3FNO_2$:
48,51% C; 2,04% H.
Gefunden:
48,15% C; 2,24% H.

b) Gemäß dem Verfahren in den Beispielen 20d und 20e wird unter Verwendung von 3-(Fluorphenyl)-6-methoxy-4,5,7-trichlor-1,2-benzisoxazol die Verbindung {[4,5,7-Trichlor-3-(2-fluorphenyl)-1,2-benzisoxazol-6-yl]oxy}-essigsäure erhalten.

## Beispiel 50

10 g 7-Chlor-6-hydroxy-3-(2-fluorphenyl)-1,2-benzisoxazol aus Beispiel 36c werden in 70 ml DMF gelöst. Dann werden 7,86 g $K_2CO_3$ eingerührt. Man gibt 3,6 ml Chloracetonitril zu und rührt die Mischung ½ Stunde bei Zimmertemperatur. Anschließend läßt man sie 2 Stunden bei einer Temperatur von 55°C. Die Mischung wird dann noch 15 Stunden bei Zimmertemperatur gerührt, mit 1,5 ml Chloracetonitril versetzt und nochmals 6 Stunden bei 50°C gerührt. Dann wird sie in eine große Menge Eiswasser gegossen, wobei {[7-Chlor-3-(2-fluorphenyl)-1,2-benzisoxazol-6-yl]oxy} acetonitril ausfällt, das dann abfiltriert wird. Schmelzpunkt: 147°C.

Analyse:

Berechnet für $C_{15}H_8ClFN_2O_2$:
59,51% C; 2,66% H; 9,25% N.
Gefunden:
59,38% C; 2,67% H; 9,36% N.

## Beispiel 51

Eine Suspension von 3,0 g Natriumhydrid in 75 ml DMF wird mit 15 g 7-Chlor-6-hydroxy-3-(2-fluorphenyl)-1,2-benzisoxazol in 75 ml DMF versetzt. Die Mischung wird 1 Stunde bei Zimmertemperatur stehengelassen, mit 16,7 ml 2-Bromisobuttersäureäthylester versetzt, 72 Stunden bei einer Temperatur von 50°C stehengelassen, in eine Eis/Salzsäuremischung gegossen und mit $CH_2Cl_2$ extrahiert. Dann wird die organische Phase nacheinander mit 5%igem $K_2CO_3$ und mit gesättigter Natriumchloridlösung gewaschen, über $MgSO_4$ getrocknet, filtriert und verdampft, wobei ein braunes Öl zurückbleibt, das bei vermindertem Druck destilliert wird, um nicht umgesetzten 2-Bromisobuttersäureäthylester zu entfernen. Der Rückstand wird mit Äther/Petroläther verrieben und filtriert. Die Mutterlauge wird verdampft, wobei ein Öl zurückbleibt, das bei 200°C und einem Druck von 1 mm Hg destilliert wird. Man erhält so 2-{[7-Chlor-3-(2-fluorphenyl)-1,2-benzisoxazol-6-yl]oxy}-2-methylpropionsäureäthylester.

Analyse:

Berechnet für $C_{19}H_{17}ClFNO_4$:
60,6% C; 4,53% H; 3,70% N.
Gefunden:
60,0% C; 4,72% H; 3,60% N.

Beispiel 52

a) Eine Mischung aus 46 g 4-Chlor-2-fluorbenzoylchlorid und 38,9 g 2,3-Dichloranisol in 150 ml 1,2-Dichloräthan wird allmählich mit 32 g $AlCl_3$ versetzt. Die Mischung wird unter heftiger Gasentwicklung auf 40°C erhitzt und dann in konzentrierte Salzsäure mit Eis gegossen. Die organische Schicht wird abgetrennt, und die wäßrige Schicht wird mit zusätzlichem organischem Lösungsmittel extrahiert. Die vereinigten organischen Extrakte werden mit Wasser gewaschen, über $Na_2SO_4$ getrocknet und verdampft. Das Rohprodukt wird mit Hexan verrieben, wobei sich 2,3-Dichlor-4-methoxy-4'-chlor-2'-fluorbenzophenon bildet. Eine analytische Probe wird aus Äthanol umkristallisiert. Schmelzpunkt: 115—116°C.

Analyse:

Berechnet für $C_{14}H_8Cl_3FO_2$:
50,41% C; 2,42% H; 5,80% F.
Gefunden:
50,11% C; 2,36% H; 6,17% F.

b) Ähnlich wie im Beispiel 21b wird 2,3-Dichlor-4-methoxy-4'-chlor-2'-fluorbenzophenon zu 2,3-Dichlor-4-methoxy-4'-chlor-2'-fluorbenzophenonoxim umgesetzt.

c) Zu einer Mischung aus 2,24 g NaH in 100 ml DMF tropft man 21,75 g 2,3-Dichlor-4-methoxy-4'-chlor-2'-fluorbenzophenonoxim in 100 ml DMF. Die Mischung wird dann 1 Stunde gerührt und in Eiswasser getrocknet, wobei man ein Isomerengemisch erhält, das an Silicagel mit 50%igem Hexan und 50%igem Toluol als Eluiermittel chromatographiert wird. Man erhält dabei 7-Chlor-3-(4-chlor-2-fluorphenyl)-6-methoxy-1,2-benzisoxazol. Schmelzpunkt: 193—194°C.

Analyse:

Berechnet für $C_{14}H_8Cl_2FNO_2$:
53,87% C; 2,58% H; 4,49% N.
Gefunden:
54,01% C; 2,56% H; 4,44% N.

d) Ein festes Gemisch aus 5,4 g 7-Chlor-3-(4-chlor-2-fluorphenyl)-6-methoxy-1,2-benzisoxazol und 22,4 g Pyridinhydrochlorid wird 2 Stunden bei 200°C erhitzt und nachfolgend in kräftig gerührtes Eiswasser gegossen, wobei man ein demethyliertes Produkt erhält.
Eine Lösung von 3,9 g dieses demethylierten Produktes in 40 ml DMF wird mit 1,9 g $K_2CO_3$ und 2,3 g Bromessigsäureäthylester versetzt. Die Reaktionsmischung wird 2 Stunden auf 60°C erhitzt und 18 Stunden stehengelassen. Dann wird sie mit 100 ml Wasser und 10 ml 50%iger Natronlauge versetzt, 90 Minuten bei 90°C erhitzt, in Wasser gegossen, angesäuert, mit Essigester extrahiert, getrocknet und verdampft, wobei {[7-Chlor-3-(4-chlor-2-fluorphenyl)-1,2-benzisoxazol-6-yl]oxy}-essigsäure anfällt. Schmelzpunkt: 226—227°C.

Analyse:

Berechnet für $C_{15}H_8Cl_2FNO_4$:
50,59% C; 2,26% H; 3,93% N.
Gefunden:
50,39% C; 2,24% H; 4,06% N.

Beispiel 53

Eine Suspension aus 9 g Lithiumaluminiumhydrid (98%ig) in 100 ml wasserfreiem Äther wird mit einer Lösung aus 10 g 2-{[7-Chlor-3-(2-fluorphenyl)-1,2-benzisoxazol-6-yl]oxy}-essigsäureäthylester aus Beispiel 1d) in 200 ml Äther, das so viel Tetrahydrofuran enthält, daß eine Lösung möglich ist, versetzt. Die Reaktionsmischung wird 2 Stunden bei Zimmertemperatur gerührt und 1 Stunde unter Rückfluß. Nachfolgend wird sie mit 0,9 ml Wasser, 0,9 ml 15%iger Natronlauge und mit 2,7 ml Wasser versetzt. Die gerührte Suspension wird filtriert, und das Filtrat wird eingeengt, wobei 2-{[7-Chlor-(2-fluorphenyl)-1,2-benzisoxazol-6-yl]oxy}-äthanol gebildet wird. Schmelzpunkt: 112°C.

Analyse:

Berechnet für $C_{15}H_{11}ClFNO_3$:
58,63% C; 3,58% H; 4,56% N.
Gefunden:
58,48% C; 3,62% H; 4,49% N.

Beispiel 54

4,5 g 2-{[7-Chlor-3-(2-fluorphenyl)-1,2-benzisoxazol-6-yl]oxy}-2-methylpropionsäureäthylester aus Beispiel 51 werden in 35 ml Methanol gelöst und mit 30 ml einer 15%igen Natronlauge versetzt. Die erhaltene Suspension wird 4 Stunden unter Rückfluß erhitzt, in Eiswasser gegossen und angesäuert, wobei ein öliger Niederschlag entsteht, der mit Äther extrahiert wird. Die Ätherextrakte werden mit 10%igem Natriumhydrogencarbonat gewaschen. Die basischen Extrakte werden dann mit konzentrierter Salzsäure angesäuert und abgekühlt, wobei 2-{[7-Chlor-3-(2-fluorphenyl)-1,2-benzisoxazol-6-yl]oxy}-2-methylpropionsäure entsteht. Schmelzpunkt: 108°C.

Analyse:

Berechnet für $C_{17}H_{13}ClFNO_4$:
58,45% C; 3,72% H; 4,01% N.
Gefunden:
58,39% C; 3,75% H; 3,96% N.

Beispiel 55

3,35 g $NaN_3$ und 2,25 g $AlCl_3$ werden eine halbe Stunde in 50 ml THF unter Rückfluß gerührt, mit einer Lösung aus 5 g {[7-Chlor-3-(2-fluorphenyl)-1,2-benzisoxazol-6-yl]oxy}-acetonitril aus Beispiel 50 in 50 ml THF versetzt und ca. 120 Stunden unter Rückfluß gerührt. Dann gibt man Wasser zu und entfernt das Lösungsmittel. Der Rückstand wird mit verdünnter Salzsäure behandelt und mit $CHCl_3$ extrahiert. Nach Extraktion der Chloroformlösung mit 15%iger Natronlauge bildet sich ein Niederschlag, der filtriert wird, in heißem Wasser gelöst wird und sauer gemacht wird, wobei man 7-Chlor-3-(2-fluorphenyl)-6-{[5-tetrazolylmethyl]-oxy}-1,2-benzisoxazol mit einem Schmelzpunkt von 198 bis 200°C erhält.

Analyse:

Berechnet für $C_{15}H_9ClFN_5O_2$:
52,17% C; 2,60% H; 20,28% N.
Gefunden:
52,02% C; 2,69% H; 20,37% N.

**Patentansprüche**

1. Verbindungen der allgemeinen Formel

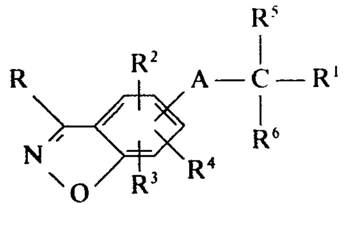

(I)

worin R Wasserstoff, Naphthyl, einen Rest der Formel

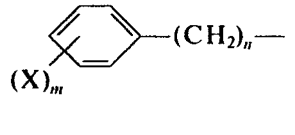

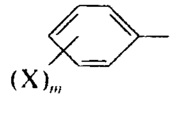

oder

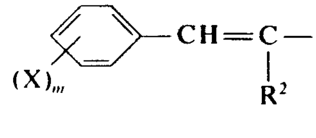

unsubstituiertes oder mit Halogen oder $C_{1-4}$-Alkyl substituiertes Thienyl, Furyl, Pyridyl oder Pyridyl-

N-oxyd bedeutet, $R^1$ für eine freie oder veresterte Carboxylgruppe mit 1 bis 8 Kohlenstoffatomen, einen Rest der Formel

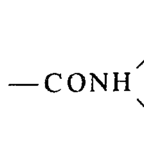 $—CH_2OH$ $—CH(OR^9)_2$ 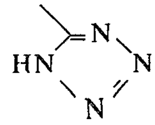 oder 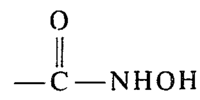

steht, $R^2$, $R^3$ und $R^4$, die gleich oder verschieden sein können, Wasserstoff, Halogen oder $C_{1-4}$-Alkyl bedeuten, X Wasserstoff, Halogen, $C_{1-4}$-Alkyl, $C_{1-4}$-Alkoxy, Hydroxy, Trifluormethyl, Nitro bedeutet, $R^5$, $R^6$, $R^7$, $R^8$ und $R^9$, die gleich oder verschieden sein können, Wasserstoff oder $C_{1-4}$-Alkyl bedeuten, A für O oder S steht und m und n, die gleich oder verschieden sein können, 1, 2 oder 3 bedeuten, sowie die physiologisch verträglichen Salze der obigen Verbindungen.

2. Eine Verbindung gemäß Anspruch 1 der Formel

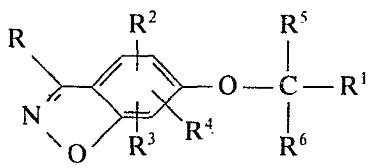

(I')

worin R, $R^1$, $R^2$, $R^3$, $R^4$, $R^5$ und $R^6$ die im Anspruch 1 angegebene Bedeutung haben.

3. Verfahren zur Herstellung einer Verbindung der Formel

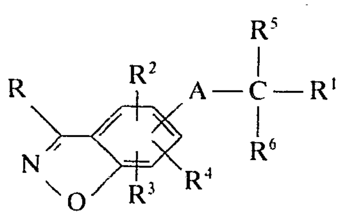

(I)

worin R, $R^1$, $R^2$, $R^3$, $R^4$, $R^5$, $R^6$, A die im Anspruch 1 angegebene Bedeutung haben, dadurch gekennzeichnet, daß man

a) eine Verbindung der Formel

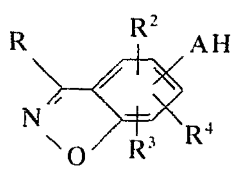

worin R, $R^2$, $R^3$, $R^4$, A die im Anspruch 1 angegebene Bedeutung haben, mit einer Verbindung der Formel

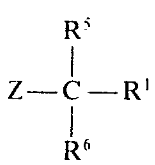

worin $R^1$, $R^5$ und $R^6$ die im Anspruch 1 angegebene Bedeutung haben, und Z Chlor oder Fluor bedeutet, in Gegenwart einer Base und eines Lösungsmittels umsetzt oder

b) eine Verbindung der Formel

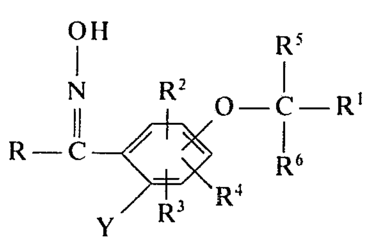

oder eine Verbindung der Formel

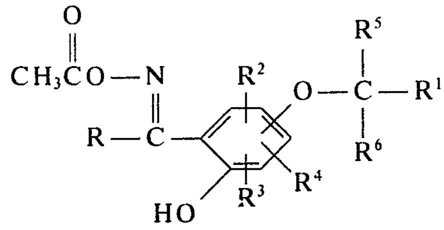

worin R, $R^1$, $R^2$, $R^3$, $R^4$ und $R^5$ die im Anspruch 1 angegebene Bedeutung haben, durch eine Behandlung mit einer Base in Gegenwart eines Lösungsmittels bei einer Temperatur im Bereich von Raumtemperatur bis zur Rückflußtemperatur des Reaktionsmediums cyclisiert oder

c) eine Verbindung der Formel

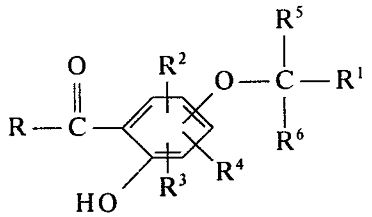

worin $R^1$, $R^2$, $R^3$, $R^4$, $R^5$ und $R^6$ die im Anspruch 1 angegebene Bedeutung haben und R für Wasserstoff steht, mit Hydroxylamin-o-sulfonsäure umsetzt,

d) gegebenenfalls eine Verbindung der Formel I, worin $R^1$ für eine veresterte Carboxylgruppe oder CN steht, durch Hydrolyse in eine entsprechende Verbindung, worin $R^1$ für COOH steht, umwandelt.

e) gegebenenfalls eine Verbindung der Formel I, worin A für Sauerstoff steht, X, $R^1$, $R^2$, $R^3$ und $R^4$ nicht niederes Alkyl bedeuten und $R^1 = CH_2OH$ oder CHO ist, durch Oxydation in eine entsprechende Verbindung, worin $R^1$ für COOH steht, umwandelt.

f) gegebenenfalls eine Verbindung der Formel I, worin $R^1 = COZ$, durch eine Behandlung mit

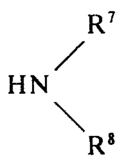

in eine entsprechende Verbindung, worin $R^1$ für

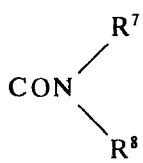

steht, umwandelt,

g) gegebenenfalls eine Verbindung der Formel I, worin $R^1 = CN$, durch Behandlung mit $HN_3$ in Dimethylformamid in eine entsprechende Verbindung mit

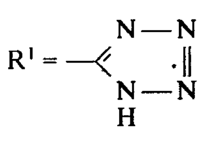

umwandelt,

h) gegebenenfalls eine Verbindung der Formel I, worin $R^1$ für

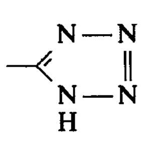

steht, durch saure oder basische Hydrolyse in die entsprechende Verbindung mit $R^1 = COOH$ überführt,

i) gegebenenfalls eine Verbindung der Formel I, worin $R^1$ für COOH steht, durch Behandlung mit einer geeigneten organischen Base oder Alkali-/Erdalkali in das entsprechende Salz überführt,

j) gegebenenfalls eine Verbindung der Formel I, worin

R = 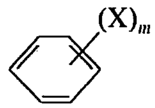

m 1 oder 2 bedeutet und X nicht für $NO_2$ steht, durch Behandlung mit Salpetersäure in Eisessig in die entsprechende Verbindung, worin R für

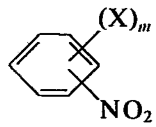

steht, überführt, und

k) gegebenenfalls eine Verbindung der Formel I, worin R für

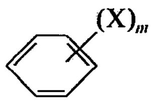 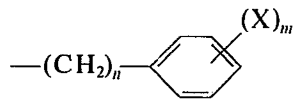 oder 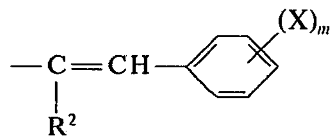

und X für Alkoxy steht, durch Dealkylisierung in eine entsprechende Verbindung, worin X für Hydroxy steht, umwandelt.

4. Verfahren nach Anspruch 3, dadurch gekennzeichnet, daß man eine Verbindung der Formel I herstellt, in welcher A Sauerstoff bedeutet.

5. Pharmazeutisches Mittel mit diuretischer Wirkung, gekennzeichnet durch eine pharmazeutisch wirksame Menge einer Verbindung gemäß Anspruch 1 in Verbindung mit einem pharmazeutisch verträglichen Träger.

6. Pharmazeutisches Mittel zur Steigerung der Harnsäureausscheidung, gekennzeichnet durch eine pharmazeutisch wirksame Menge einer Verbindung gemäß Anspruch 1 in Verbindung mit einem pharmazeutisch verträglichen Träger.

7. Pharmazeutisches Mittel zur Blutdrucksenkung, gekennzeichnet durch eine pharmazeutisch wirksame Menge einer Verbindung gemäß Anspruch 1 in Verbindung mit einem pharmazeutisch verträglichen Träger.

## Claims

1. Compounds of the general formula

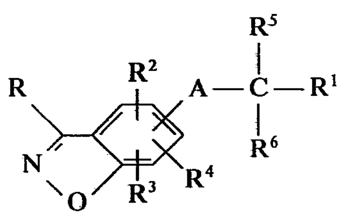 (I)

in which R is hydrogen, naphthyl, a radical of the formula

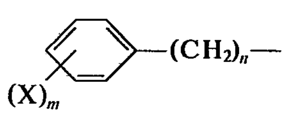 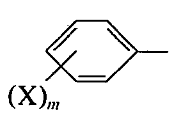 or 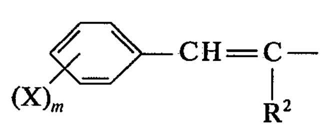

unsubstituted thienyl or thienyl substituted with halogen or $C_{1-4}$-alkyl, furyl, pyridyl or pyridyl N-oxide; $R^1$ is a free or esterified carboxyl group of from 1 to 8 carbon atoms, a radical of the formula

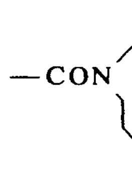 $-CH_2OH$ $-CH(OR^9)_2$ 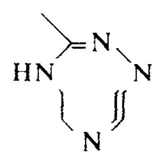 or 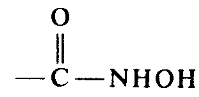

$R^2$, $R^3$ and $R^4$ are the same or different and each can be hydrogen, halogen or $C_{1-4}$-alkyl; X is hydrogen, halogen, $C_{1-4}$-alkyl, $C_{1-4}$-alkoxy, hydroxy, trifluoromethyl, nitro; $R^5$, $R^6$, $R^7$, $R^8$ and $R^9$ are the same or different and can be hydrogen or $C_{1-4}$-alkyl; A is O or S; and m and n are the same or different and each can be the integer 0, 2 or 3; or a physiologically acceptable salt thereof.

2. A compound as claimed in claim 1 depicted by the formula

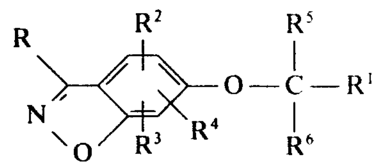

(I')

wherein R, $R^1$, $R^2$, $R^3$, $R^4$, $R^5$ and $R^6$ are as defined in claim 1.

3. A method for preparing a compound of the formula I

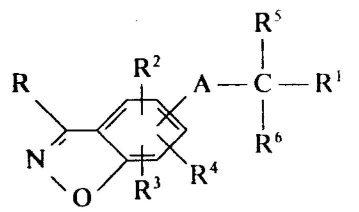

(I)

in which R, $R^1$, $R^2$, $R^3$, $R^4$, $R^5$, $R^6$ and A are as defined in claim 1, which comprises

a) reacting a compound of the formula

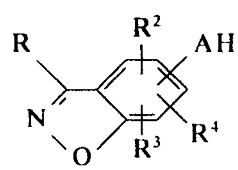

in which R, $R^2$, $R^3$, $R^4$ and A are as defined in claim 1 with a compound of the formula

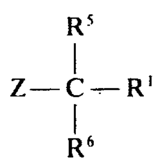

in which $R^1$, $R^5$ and $R^6$ are as defined in claim 1 and Z is chlorine, bromine or fluorine in the presence of a base and a solvent or

b) cyclizing a compound of the formula

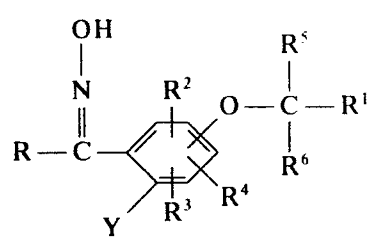

or a compound of the formula

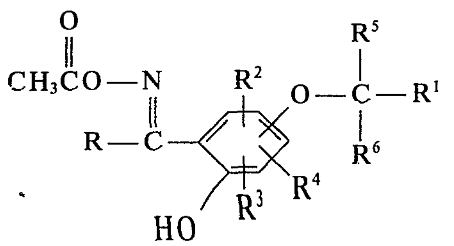

in which R, $R^1$, $R^2$, $R^3$, $R^4$ and $R^5$ are as defined in claim 1 by treatment with a base in the presence of a solvent at a temperature of from ambient to reflux of the reaction medium, or

c) reacting a compound of the formula

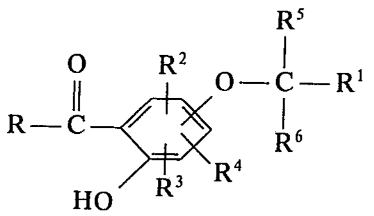

in which $R^1$, $R^2$, $R^3$, $R^4$, $R^5$ and $R^6$ are as defined in claim 1 and R is hydrogen with hydroxylamine-o-sulfonic acid,

d) optionally converting a compound of the formula I in which $R^1$ is a carboxylic acid ester or CN by hydrolysis to a corresponding compound in which $R^1$ is COOH, optionally converting a compound of the formula I in which $R^1$ is $CH(OR^1)_2$ by hydrolysis to a corresponding compound in which $R^1$ is $-CHO$,

e) optionally converting a compound of the formula I in which A represents oxygen, X, $R^2$, $R^3$ and $R^4$ are not loweralkyl and $R^1$ is $CH_2OH$ or CHO by oxidation to a corresponding compound in which $R^1$ is COOH, optionally converting a compound in which $R^1$ is COOH, optionally converting a compound of the formula I in which $R^1$ is COOH to a corresponding compound in which $R^1$ is COZ,

f) optionally converting a compound of the formula I in which $R^1$ is COZ by treatment with

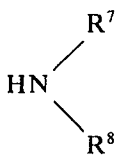

to the corresponding compound in which $R^1$ is

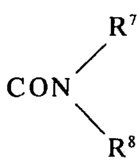

g) optionally converting a compound of the formula I in which $R^1$ is CN by treatment with $HN_3$ in dimethylformamide to the corresponding compound in which $R^1$ is

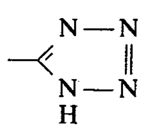

h) optionally converting a compound of the formula I in which $R^1$ is

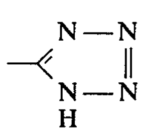

via acid or basic hydrolysis to the corresponding compound in which $R^1$ is COOH,

i) optionally converting a compound of the formula I in which $R^1$ is COOH to the corresponding

salt via treatment with a appropriate organic or alkali/alkaline earth base,

j) optionally converting a compound of the formula I in which R is

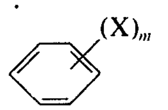

wherein m is 1 or 2 and X is not $NO_2$ via treatment with nitric acid in glacial acetic acid to a corresponding compound in which R is

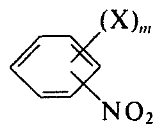

and

m) optionally converting a compound of the formula I in which R is

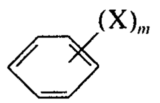

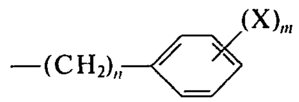

or

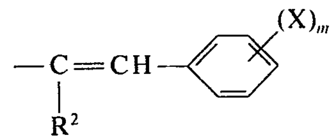

and X is alkoxy by dealkylating to a corresponding compound in which X is hydroxy.

4. A method as claimed in claim 3 which comprises preparing a compound of the formula I in which A is oxygen.

5. A pharmaceutical composition useful for producing diuresis which comprises a pharmaceutically effective amount of a compound defined in claim 1 and a pharmaceutically acceptable carrier therefor.

6. A pharmaceutical composition useful for increasing uric acid excretion which comprises a pharmaceutically effective amount of a compound defined in claim 1 and a pharmaceutically acceptable carrier therefor.

7. A pharmaceutical composition useful for depressing blood pressure which comprises a pharmaceutically effective amount of a compound defined in claim 1 and a pharmaceutically acceptable carrier therefor.

**Revendications**

1. Composés de formule générale

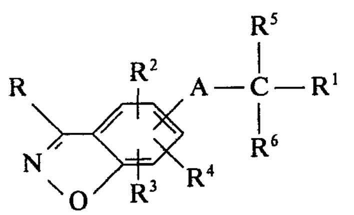

(I)

dans laquelle R représente l'hydrogène, un groupe naphthyle, un groupe de formule

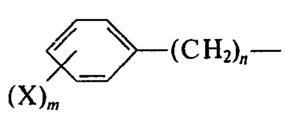

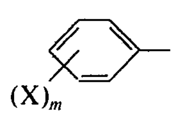

ou

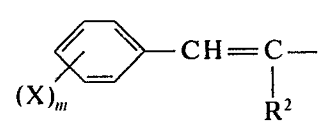

un groupe thiényle, furyle, pyridyle ou pyridyl-N-oxyde non-substitué ou substitué par un halogène ou un groupe alkyle en $C_{1-4}$; $R^1$ représente un groupe carboxyle libre ou estérifié ayant de 1 à 8 atomes de carbone, un groupe de formule

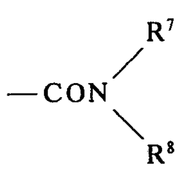

$-CH_2OH$ $-CH(OR^9)_2$

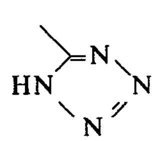

ou

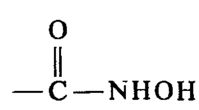

$R^2$, $R^3$ et $R^4$ qui peuvent être identiques ou différents, représentent l'hydrogène, un halogène ou

un groupe alkyle en $C_{1-4}$; X représente l'hydrogène, un halogène, un groupe alkyle en $C_{1-4}$, alcoxy en $C_{1-4}$, hydroxy, trifluorméthyle, nitro; $R^5$, $R^6$, $R^7$, $R^8$ et $R^9$ qui peuvent être identiques ou différents représentent l'hydrogène ou un groupe alkyle en $C_{1-4}$; A est O ou S, et m et n qui peuvent être identiques ou différents, sont égaux à 1, 2 ou 3, ainsi que les sels physiologiquement acceptables des composés ci-dessus.

2. Composé selon la revendication 1, de formule

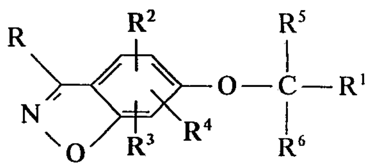

(I')

où R, $R^1$, $R^2$, $R^3$, $R^4$, $R^5$ et $R^6$ ont les significations indiquées dans la revendication 1.

3. Procédé pour la préparation d'un composé de formule

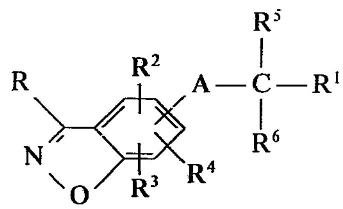

(I)

dans laquelle R, $R^1$, $R^2$, $R^3$, $R^4$, $R^5$, $R^6$ et A ont les significations indiquées dans la revendication 1, caractérisé en ce que:

a) on fait réagir un composé de formule

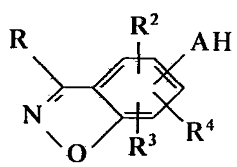

dans laquelle R, $R^2$, $R^3$, $R^4$ et A ont les significations indiquées dans la revendication 1, avec un composé de formule

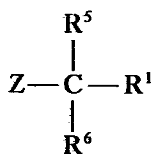

dans laquelle $R^1$, $R^5$ et $R^6$ ont les significations indiquées dans la revendication 1, et Z représente le chlore ou le flour, en présence d'une base et d'un solvant, ou

b) on cyclise un composé de formule

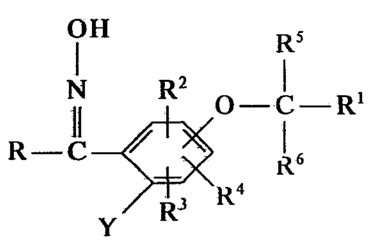

ou un composé de formule

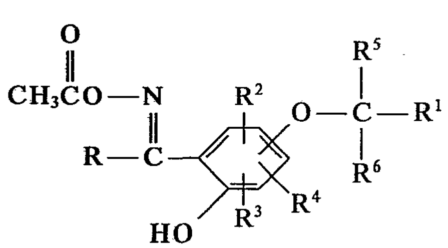

dans lesquelles R, $R^1$, $R^2$, $R^3$, $R^4$ et $R^5$ ont les significations indiquées dans la revendication 1, par traitement avec une base en présence d'un solvant à une température allant de la température de reflux du milieu de réaction, ou

c) on fait réagir un composé de formule

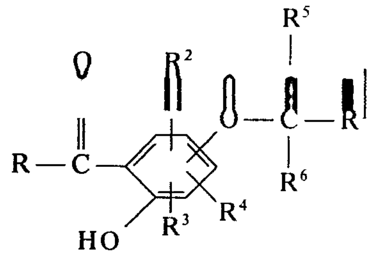

dans laquelle $R^1$, $R^2$, $R^3$, $R^4$, $R^5$ et $R^6$ ont les significations indiquées dans la revendication 1 et R représente l'hydrogène, avec l'acide hydroxylamine-o-sulfonique.

d) par hydrolyse, on convertit éventuellement un composé de formule I où $R^1$ est un groupe carboxyle estérifié ou CN, en un composé correspondant où $R^1$ est COOH,

e) par oxydation, on convertit éventuellement un composé de formule I où A est l'oxygène, X, $R^2$, $R^3$ et $R^4$ ne représentent pas un groupe alkyle inférieur et $R^1 = CH_2OH$ ou CHO, en un composé correspondant où $R^1$ est COOH,

f) par un traitement avec

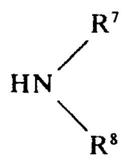

on convertit éventuellement un composé de formule I où $R^1 = COZ$, en un composé correspondant où $R^1$ est

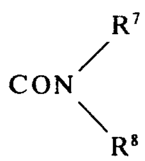

g) par un traitement avec $HN_3$ dans le diméthylformamide, on convertit éventuellement un composé de formule I où $R^1 = CN$, en un composé correspondant où

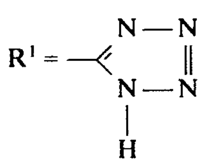

h) par une hydrolyse acide ou basique, on convertit éventuellement un composé de formule I ou $R^1$ est

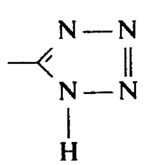

en le composé correspondant où $R^1 = COOH$,

i) par un traitement avec une base organique appropriée ou une base alcaline ou alcalino-terreuse, on convertit éventuellement un composé de formule I où $R^1$ est COOH, en le sel correspondant,

j) par un traitement avec l'acide nitrique dans l'acide acétique glacial, on convertit éventuellement un composé de formule I où $R^1$ est

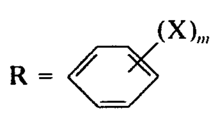

m représente 1 ou 2 et X n'est pas $NO_2$, en le composé correspondant où R représente

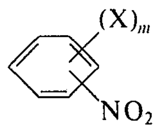

k) par désalkylation, on convertit éventuellement un composé de formule I où R représente

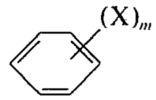

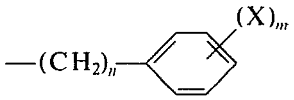

ou

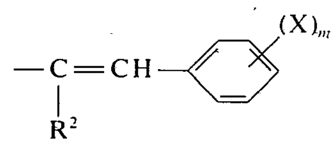

et X est un groupe alcoxy, en un composé correspondant où X représente un groupe hydroxy.

4. Procédé selon la revendication 3, caractérise en ce qu'on prépare un composé de formule I dans lequel A représente l'oxygène.

5. Composition pharmaceutique avec une action diurétique, caractérisée en ce qu'elle contient une quantité pharmaceutiquement efficace d'un composé selon la revendication 1, associée avec un véhicule pharmaceutiquement acceptable.

6. Composition pharmaceutique pour augmenter l'excrétion d'acide urique, caractérisée en ce qu'elle contient une quantité pharmaceutiquement efficace d'un composé selon la revendication 1 en association avec un véhicule pharmaceutiquement acceptable.

7. Composition pharmaceutique pour diminuer la pression sanguine, caractérisée en ce qu'elle contient une quantité pharmaceutiquement efficace d'un composé selon la revendication 1, en association avec un véhicule pharmaceutiquement acceptable.